# EUROPÄISCHE PATENTSCHRIFT

(11) **EP 0 817 769 B1**
(45) Veröffentlichungstag und Bekanntmachung des Hinweises auf die Patenterteilung: **26.05.1999**
(21) Anmeldenummer: 96909153.7
(22) Anmeldetag: 29.03.1996
(51) Int. Cl.: C07C 271/22, C07D 303/16, A61K 31/325, A61K 31/335, C07D 401/12, C07D 409/12, C07D 407/12, C07D 333/24, C07D 213/55

(54) **BORNEOLESTER, VERFAHREN ZU IHRER HERSTELLUNG UND IHRE PHARMAZEUTISCHE VERWENDUNG**
BORNEOL ESTERS, PROCESS FOR THEIR PREPARATION AND THEIR PHARMACEUTICAL USE
ESTERS DE BORNEOL, LEUR PROCEDE DE PREPARATION ET LEUR UTILISATION PHARMACEUTIQUE

(30) Priorität: 29.03.1995 DE 19513040
(43) Veröffentlichungstag der Anmeldung: 14.01.1998
(73) Patentinhaber: SCHERING AKTIENGESELLSCHAFT, 13353 Berlin (DE)
(72) Erfinder: KLAR, Ulrich, D-13503 Berlin (DE); GRAF, Hermann, D-13465 Berlin (DE); NEEF, Günter, D-10771 Berlin (DE); BLECHERT, Siegfried, D-13503 Berlin (DE)
(86) Internationale Anmeldenummer: EP9601404
(87) Internationale Veröffentlichungsnummer: WO9632376

(56) Entgegenhaltungen:
- EP-A- 0 253 739

## Beschreibung

Die Erfindung betrifft neue pharmakologisch wirksame Verbindungen, welche die Fähigkeit haben, die Tubulin-Polymerisation bzw. Tubulin-Depolymerisation zu beeinflussen.

Eine Reihe natürlicher Mitosegifte werden als Antitumormittel eingesetzt bzw. befinden sich in der klinischen Prüfung. Es existieren verschiedene Klassen dieser Mitosegifte, die ihre zytotoxische Wirkung entweder durch Inhibition der Polymerisation von Mikrotubuli im Spindelapparat entfalten (z. B. Vinka-Alkaloide, Colchicin) oder dies durch eine GTP-unabhängige Steigerung der Polymerisation des Tubulins und Verhinderung der Depolymerisation von Mikrotubuli erreichen (z. B. Taxol, Taxotere). Mitosegifte haben aufgrund bisher unverstandener physikochemischer Eigenschaften und durch die Besonderheiten neoplastischer Zellen eine gewisse Selektivität für Tumorzellen, es besteht jedoch auch eine nicht unerhebliche Zytotoxizität gegenüber nicht transformierten Zellen.
Vinka-Alkaloide besitzen bis heute große Bedeutung in der kombinierten Chemotherapie myeloischer Tumore. Taxane haben in jüngster Zeit bedeutende Anwendungsgebiete erschlossen, die durch bisher verfügbare Zytostatika nicht zugänglich waren, z.B. Ovarialkarzinome, maligne Melanome. Die Nebenwirkungen der Taxane sind allerdings denen anderer Zytostatika vergleichbar (z.B. Haarausfall, sensorische Neuropathie). Multi-Drug-resistente Tumorzellen, die das P-Glycoprotein überexprimieren sind gegenüber Taxanen resistent. Die begrenzte Verfügbarkeit des Naturstoffs Taxol behindert zudem eine breitere klinische Testung.
Es wurden deshalb Naturstoffe und synthetische Pharmaka getestet, die ein von den bisherigen Mitosegiften abweichendes Wirkspektrum besitzen. Eine *in vitro* Versuchsanordnung ermöglicht die Suche nach Substanzen, die die GTP-abhängige Polymerisation von Tubulin nicht beeinflussen, die Depolymerisation der gebildeten Mikrotubuli jedoch verhindern. Substanzen mit einem solchen Wirkprofil sollten die vielseitigen Funktionen von Mikrotubuli in extranukleären Zellkompartimenten weniger stark beeinflussen, als die Dynamik des Spindelapparats während der Mitose (Meta/Anaphase). Folgerichtig sollten solche Verbindungen geringere Nebenwirkungen *in vivo* zeigen als Taxane oder Vinka-Alkaloide.
Tubulin ist ein essentieller Bestandteil der mitotischen Spindel. Es dient unter anderem zur Aufrechterhaltung der Zellform, zum Transport von Organellen innerhalb der Zelle und zur Beeinflussung der Zellbeweglichkeit.

Taxane stellten bislang die einzige bekannte Strukturklasse dar, die in der Lage ist, die Polymerisation von Tubulin (überwiegend in der G2-Phase) zu beschleunigen sowie die gebildeten Mikrotubuli-Polymere zu stabilisieren. Dieser Mechanismus unterscheidet sich deutlich von denjenigen die andere, ebenfalls die phasenspezifische Zellteilung beeinflussende Strukturklassen aufweisen. So hemmen beispielsweise Substanzen aus der Gruppe der Vinka-Alkaloide (z.B. Vincristine und Vinblastine) aber auch Colchicin die Polymerisation der Tubulindimeren in der M-Phase.

Es wurde nun gefunden, daß vergleichsweise einfach herzustellende Verbindungen der Formel I in der Lage sind, die Depolymerisation von Mikrotubuli zu hemmen, ohne GTP-unabhängig die Bildung von Mikrotubuli zu steigern. Darüber hinaus wurden Verbindungen mit einem völlig neuen Wirkprofil identifiziert, die in der Lage sind, die Depolymerisation von Mikrotubuli zu beschleunigen. Auf Grund dieser Eigenschaften stellen die Verbindungen der Formel I wertvolle Pharmaka dar, die prinzipiell in der Lage sind, die schwer zu synthetisierenden und in ausreichenden Mengen noch nicht verfügbaren Taxane wie z.B. Taxol und Taxotere® in der Therapie maligner Tumore zu ergänzen bzw. zu ersetzen (EP-A 253739).

Die neuen Borneolester sind gekennzeichnet durch die allgemeine Formel I 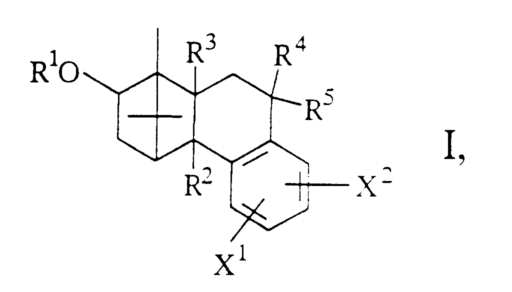 worin
- R¹: C(O)-CH(OR⁶)-CH(NR^{7a}R^{7b})-R⁸, C(O)-CH(OR^{6a})-CH[NH(C(O)-CH(OR^{6b})-CH(NR^{7a}R^{7b})-R^{8a})]-R^{8b},
- R²: Wasserstoff, -OH, C₁-C₁₀-Alkyl, C₁-C₁₀-Alkoxy, -OC(O)R^{9a}, -OSO₂R^{9a}, NHR^{9a}, NR^{9a}R^{9b},
- R³: Wasserstoff, -OH, C₁-C₁₀-Alkoxy, -OC(O)R^{9b}, -OSO₂R^{9b}, oder
- R², R³: gemeinsam ein Sauerstoffatom.
- R⁴: Wasserstoff, C₁-C₁₀-Alkyl, -(CH₂)ₙ-OR^{11a},
- R⁵: Wasserstoff, C₁-C₁₀-Alkyl, -(CH₂)ₚ-OR^{11b}, oder
- R⁴, R⁵: gemeinsam ein Sauerstoffatom, eine =CHR¹⁰-Gruppe,
- R^{6a}, R^{6b}: gleich oder verschieden sind und R⁶ bedeuten.
- R⁴, R⁵: gemeinsam ein Sauerstoffatom eine =CHR¹⁰-Gruppe, eine -CH₂-CH₂-Gruppe, eine CH₂-O-Gruppe,
- R^{6a}, R^{6b}: gleich oder verschieden sind und R⁶ bedeuten,
- R⁶: Wasserstoff, C₁-C₁₀-Alkyl, Aryl, C₁-C₂₀-Acyl, C₇-C₂₀-Aralkyl, -SO₂R^{9c}, R¹⁵ bedeuten,
- R^{7a}, R^{7b}: gleich oder verschieden sind und R⁷ bedeuten,
- R⁷: Wasserstoff, A, -C(O)R¹², -C(O)OR¹², -C(O)SR¹², -C(O)NHR^{9d}, -C(O)NR^{9d}R^{9e}, -SO₂R¹², C₁-C₁₀-Alkyl,
- R^{8a}, R^{8b}: gleich oder verschieden sind und R⁸ bedeuten,
- R⁸: 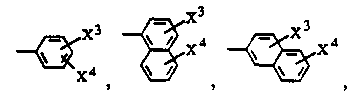 durch X³ substituiertes Heteroaryl, C₇-C₁₆-Aralkyl, C₁-C₁₀-Alkyl,
- R^{9a-g}, R¹²: gleich oder verschieden sind und C₁-C₂₀-Alkyl, C₄-C₈-Cycloalkyl, Aryl, C₇-C₂₀-Aralkyl bedeuten,
- R¹⁰: Wasserstoff, C₁-C₁₀-Alkyl, -(CH₂)ₛ-OR¹³,
- R^{11a,b} R¹³: gleich oder verschieden sind und Wasserstoff, C₁-C₁₀-Alkyl, Aryl, C₁-C₂₀-Acyl, C₇-C₂₀-Aralkyl, -SO₂R^{9c} bedeuten,
- R^{14a,b}: gleich oder verschieden sind und Wasserstoff, A, C₁-C₁₀-Alkyl, Aryl, C₇-C₁₆-Aralkyl bedeuten,
- R¹⁵: (Yⁱ₁-Yⁱ₂-Yⁱ₃-...-Yⁱᵣ)-H,
- A: B-[O-(CH₂)ₜ-C(O)]_{0 oder 1}-, Farnesyl-P(O)(OR^{9d})-O-(CH₂)ₜ-C(O)-,
- B: Hemmstoffe der Proteinkinasen oder Hemmstoffe der Farnesylproteintransferase wie z.B. Farnesyl, 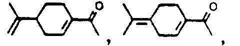
- T: eine Bindung, Zⁱ oder eine Gruppe 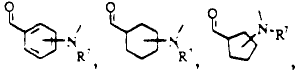
- X¹...X⁴: gleich oder verschieden sind und X bedeuten, wobei X³ und X⁴ im Falle von R⁸ und R^{8b} aber nicht gleichzeitig in der Bedeutung von Wasserstoff verwendet werden können,
- X: Wasserstoff, Halogen, -NO₂, -N₃, -CN, -NR^{14a}R^{14b}, -NHSO₂R^{9g}, C₁-C₁₀-Alkyl, C₁-C₂₀-Acyl, C₁-C₂₀-Acyloxy, OR⁶, -OP(O)R^{9g}(OR¹²) -CO₂R¹⁴, -O-A sein kann
- Yⁱ_{1...r}: gleich oder verschieden sind und -[C(O)-Wⁱ-NH]- = Zⁱ bedeuten, wobei Zⁱ als HO-Zⁱ-H eine, an ihrem terminalen Ende angeknüpfte α-, β- oder γ-Aminosäure "i" darstellt,
- n: 0 bis 8
- p: 1 bis 8
- r: 1 bis 5
- s: 1 bis 8
- t: 1 bis 6,
und freie Hydroxylgruppen in R¹, R², R³, R⁴, R⁵, R¹⁰, Z und X durch Veretherung oder Veresterung weiter funktionell abgewandelt sein können und freie Aminogruppen in R¹, R¹⁵ oder X bzw. freie Säuregruppen in X in deren Salze mit physiologisch vertraglichen Säuren bzw. Basen überführt sein können, sowie deren α-, β- oder γ-Cyclodextrinclathrate, sowie die mit Liposomen verkapselten Verbindungen der allgemeinen Formel I bedeuten.

Die Erfindung betrifft die Diastereomeren und/oder Enantiomeren dieser Borneolderivate und auch deren Gemische.
Als Alkylgruppen R², R⁴, R⁵, R⁶, R⁷, R⁸, R⁹, R¹⁰, R¹¹, R¹² R¹³, R¹⁴ und X sind gerad- oder verzweigtkettige Alkylgruppen mit 1-20 Kohlenstoffatomen zu betrachten, wie beispielsweise Methyl, Ethyl, Propyl, Isopropyl, Butyl, Isobutyl, tert.-Butyl, Pentyl, Isopentyl, Neopentyl, Heptyl, Hexyl, Decyl.
Die Alkylgruppen R², R⁴, R⁵, R⁶, R⁷, R⁸, R⁹, R¹⁰, R¹¹, R¹² R¹³, R¹⁴ und X können substituiert sein durch 1-3 Halogenatome, Hydroxygruppen, C₁-C₄-Alkoxygruppen, C₆-C₁₂-Arylgruppen, die durch 1-3 Halogenatome substituiert sein können, Di-(C₁-C₄)-Alkylamine und Tri-(C₁-C₄)-Alkylammonium.
Als Cycloalkylgruppen R⁹, R¹² kommen substituierte und unsubsutuierte Reste mit 4 bis 8 Kohlenstoffatomen in Betracht.
Als Arylrest R⁶, R⁹, R¹¹, R¹², R¹³ und R¹⁴ kommen substituierte und unsubstituierte carbocyclische oder heterocyclische (R⁸) Reste wie z.B. Phenyl, Naphtyl, Furyl, Thienyl, Pyridyl, Pyrazolyl, Pyrimidinyl, Oxazolyl, Pyridazinyl, Pyrazinyl, Chinolyl, die mehrfach substituiert sein können durch die in X definierten Gruppen, in Frage.
Die in R², R³ R⁶, R¹¹, R¹³ und X der allgemeinen Formel I enthaltenen Alkoxy-, Acyl- sowie Acyloxygruppen sollen jeweils 1 bis 20 Kohlenstoffatome enthalten, wobei Methoxy-, Ethoxy- Propoxy- Isopropoxy-, t-Butyloxy-, Formyl, Acetyl, Propionyl-. und Isopropionylgruppen bevorzugt sind.
Die Aralkylgruppen in R⁶, R⁸, R⁹, R¹¹, R¹², R¹³ und R¹⁴ können im Ring bis 14 C-Atome enthalten, bevorzugt 6 bis 10 und in der Alkylkette 1 bis 6, bevorzugt 1 bis 4 Atome. Bevorzugte Aralkylreste sind z.B. Benzyl, Phenylethyl, Naphtylmethyl bzw. Naphtylethyl. Die Ringe können mehrfach substituiert sein durch die in X definierten Gruppen.
Die Aminosäure HO-Z-H kann eine natürliche oder unnäturliche, d.h. bezüglich ihrer Konfiguration oder Substitution modifizierte α-, β- oder γ-Aminosäure darstellen. Bevorzugt werden z.B. Glycin, Alanin, Valin. Leucin, Isoleucin, Prolin.
Freie Aminogruppen in R¹, R¹⁵, X oder HO-Z-H können durch Alkylierung und/oder Acylierung funktionell abgewandelt oder mit den, dem Fachmann geläufigen Schutzgruppen wie z.B. tert.-Butyloxycarbonyl-, Benzyloxycarbonyl- Allyloxycarbonyl, 9-Fluorenylmethoxycarbonyl versehen sein.

Tertiäre Aminogruppen in R¹, R², R¹⁵ und X können funktionell abgewandelt sein, beispielsweise durch Oxidation zu den entsprechenden N-Oxiden, durch Salzbildung mit Alkylhalogeniden oder mit physiologisch verträglichen Säuren.
Freie Hydroxylgruppen in R¹, R², R³, R⁴, R⁵, R¹⁰, Z und X können funktionell abgewandelt sein, beispielsweise durch Veretherung oder Veresterung. Neben freien Hydroxylgruppen -OH sind solche Derivate bevorzugt, die in der Lage sind, die Wasserlöslichkeit der Verbindungen zu verbessern. Beispielsweise seien hier genannt die Veresterung mit Aminosäuren und deren Derivate, Pyridiniumsalze mit physiologisch verträglichen Säuren wie z.B. 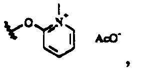 Phosphate bzw. deren Salze mit physiologisch verträglichen Basen und deren Ester wie z.B. -O-P(O)(OH)₂, -O-P(O)(OR^{9f})₂, -O-P(O)(OR^{9f})(OH), Sulfate bzw. deren Salze mit physiologisch verträglichen Basen und deren Ester wie z.B. -O-SO₃H, -O-SO₃R^{9f}, Ester und Ether mit wasserlöslichen Polymeren wie z.B. Polyethylenglykol (PEG), die über geeignete Gruppen mit den freien Hydroxylfunktionen verknüpft werden wie z.B. -O-C(O)-CH₂-O-PEG, -O-C(O)-CH₂-CH₂-C(O)-NH-PEG, -O-C(O)-CH₂-NH-C(O)-O-PEG.
Ebenfalls bevorzugt sind Ether, Ester und Amide mit Verbindungen, die ihrerseits in der Lage sind, tumorinhibierende Wirkung zu entfalten. Die bevorzugte Anknüpfungsposition derartiger Verbindungen stellen die Reste R⁷ und R¹⁴ dar. Beispielsweise seien genannt Verbindungen, welche die intrazelluläre Isoprenylierung von Proteinen hemmen wie z.B. Perillsäure, Dihydroperillsäure, Farnesyl etc.
Als Ether- und Acylreste kommen die dem Fachmann bekannten Reste in Betracht.
Bevorzugt sind leicht abspaltbare Etherreste, wie beispielsweise der Tetrahydropyranyl-, Tetrahydrofuranyl-, tert.-Butyldimethylsilyl-, tert.-Butyldiphenylsilyl-, Tribenzylsilylrest.
Als Acylreste kommen z.B. Acetyl, Propionyl, Butyryl, Benzoyl, durch z.B. mit Amino- und/oder Hydroxygruppen substituiertes Alkanoyl in Frage.
Halogen in den Definitionen für X bedeutet Fluor, Chlor, Brom und Iod.
Zur Salzbildung mit den freien Säuren bzw. Basen sind anorganische und organische Basen bzw. Säuren geeignet, wie sie dem Fachmann zur Bildung physiologisch verträglicher Salze bekannt sind Beispielsweise seien genannt: Alkalihydroxide, wie Natrium- oder Kaliumhydroxid, Erdalkalihydroxide, wie Calciumhydroxid, Ammoniak, Amine, wie Ethanolamin, Diethanolamin, Triethanolamin, N-Methylglucamin, Morpholin, Tris-(hydroxymethyl)-methylamin bzw. Essigsäure, Zitronensäure, Weinsäure, Salzsäure u.s.w.
Die Erfindung betrifft ferner Verfahren zur Herstellung von Borneolderivaten der Formel I, welches dadurch gekennzeichnet ist, daß man einen Alkohol der allgemeinen Formel II 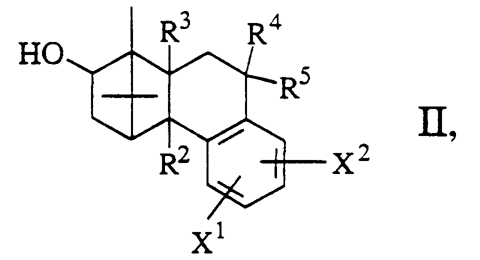 in dem R², R³, R⁴, R⁵, X¹ und X² die oben genannten Bedeutungen haben und in R², R³, R⁴, R⁵, X¹ oder X² enthaltene Hydroxylgruppen gegebenfalls geschützt sind, mit einer Verbindung der allgemeinen Formel IIIa, IIIb oder IIIc, 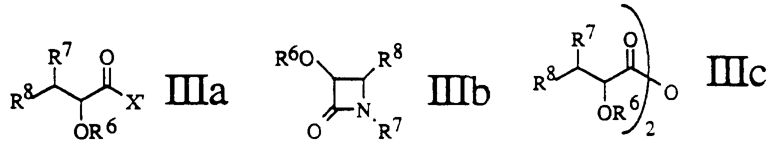 in denen R⁶, R⁷ und R⁸ jeweils die oben genannten Bedeutungen haben und X' Hydroxyl, OR⁹, Halogen und NR^{15a}R^{15b} sein kann, zu Verbindungen der allgemeinen Formel I, in denen freie Hydroxylgruppen durch Veretherung oder Veresterung weiter funktionell abgewandelt werden können, umsetzt.
Die Überführung der Verbindungen der Formel II in eine Verbindung der Formel I kann in unterschiedlicher Reihenfolge vorgenommen werden:
1) Veresterung der Alkoholfunktion (R¹ = Wasserstoff) → Modifikation von R⁴ und/oder R⁵ → gegenenfalls Epoxidöffnung, falls R² und R³ gemeinsam ein Sauerstoffatom darstellen, gegebenfalls mit anschließender Modifikation von R² und R³.
2) Veresterung der Alkoholfunktion (R¹ = Wasserstoff) → gegenenfalls Epoxidöffnung, falls R² und R³ gemeinsam ein Sauerstoffatom darstellen, gegebenfalls mit anschließender Modifikation von R², R³, R⁴ und/oder R⁵.
3) Schutz der Alkoholfunktion (R¹ = Wasserstoff) → gegenenfalls Epoxidöffnung, falls R² und R³ gemeinsam ein Sauerstoffatom darstellen, gegebenfalls mit anschließender Modifikation von R² und R³ → Modifikation von R⁴ und/oder R⁵ → Freisetzung und anschließende Veresterung der Alkoholfunktion (R¹ = Wasserstoff).
4) Schutz der Alkoholfunktion (R¹ = Wasserstoff) → Modifikation von R⁴ und/oder R⁵ → Freisetzung und anschließende Veresterung der Alkoholfunction (R¹ = Wasserstoff) → gegenenfalls Epoxidöffnung, falls R² und R³ gemeinsam ein Sauerstoffatom darstellen, gegebenfalls mit anschließender Modifikation von R² und R³.
Zur Veresterung der Alkoholfunktion (R¹ = Wasserstoff) wird mit einer Base wie z.B. Metallhydriden (z.B. Natriumhydrid), Alkalialkoholaten (z.B. Natriummethanolat, Kalium-tert.-butanolat), Alkalihexamethyldisilazan (z.B. Natriumhexamethyldisilazan), 1,5-Diazabicyclo[4.3.0]non-5-en (DBN), 1,8-Diazabicyclo[5.4.0]undec-7-en (DBU), Triethylamin, 4-(Dimethylamino)pyridin (DMAP), 1,4-Diazabicyclo[2.22]octan (DABCO) deprotoniert und mit Carbonsäurederivaten der allgemeinen Formel III in einem inerten Solvens wie zB. Dichlormethan, Diethylether, Tetrahydrofuran bei -70°C bis +50°C umgesetzt. Bevorzugt wird die Umsetzung mit Natriumhexamethyldisilazan als Base, einem cyclischen Säureamid als Carbonsäurederivat, Tetrahydrofuran als Solvens bei Temperaturen von -40°C bis +25°C.
Falls R⁴ und R⁵ gemeinsam eine =CHR¹⁰-Gruppe darstellen, kann die Funktionalisierung der olefinischen Doppelbindung nach den, dem Fachmann bekannten Methoden erfolgen. Beispielsweise kann Wasserstoff angelagert werden z.B. durch kat. Hydrierung, es können Hydroxylgruppen eingeführt werden durch Wasseranlagerung (Hydroborierung, Oxymercurierung) oder durch 1.2-Bishydroxylierung z.B. mit Osmiumtetroxid oder Kaliumpermanganat. Die Einführung einer Carbonylgruppe (R⁴, R⁵ stellen gemeinsam ein Sauerstoffatom dar) gelingt nach Spaltung der Doppelbindung z.B. durch Ozonolyse oder durch oxidative Spaltung eines 1.2-Diols. Eine derartig erzeugte Carbonylgruppe kann beispielsweise reduziert, alkyliert oder als Carbonylkomponente in einer Wittig-Reaktion zum Aufbau modifizierter =CHR¹⁰-Gruppen verwendet werden.
Falls R² und R³ gemeinsam ein Sauerstoffatom darstellen, kann das Epoxid durch Nucleophile wie beispielsweise Wasser, Carbonsäurederivate (Carbonsäuren, Carbonsäurehalogenide, Carbonsäureanhydride), Sulfonsäurederivate (Sulfonsäuren, Sulfonsäurehalogenide, Sulfonsäureanhydride), Amine, in Gegenwart von mineralischen oder organischen Säuren wie beispielsweise Chlorwasserstoff, para-Toluolsulfonsäure oder Lewissäuren wie beispielsweise Bortrifluoridetherat, Titantetraisopropoxid, Cerammoniumnitrat entweder in inerten oder als Nucleophil fungierenden Lösungsmittel bei -70°C bis +50°C umgesetzt werden.
Freie Hydroxylgruppen in I können nach den, dem Fachmann bekannten Methoden beispielsweise durch Veretherung oder Veresterung weiter funktionell abgewandelt werden. Beispielsweise lassen sich freie Hydroxylgruppen in Pyridiniumsalze mit physiologisch verträglichen Säuren, in Phosphate bzw. deren Salze mit physiologisch verträglichen Basen oder in deren Ester, in Sulfate bzw. deren Salze mit physiologisch verträglichen Basen oder in deren Ester oder in Ester und Ether mit wasserlöslichen Polymeren überführen. Ebenfalls lassen sich Ether und Ester von Verbindungen, die ihrerseits in der Lage sind, tumorinhibierende Wirkung zu entfalten, herstellen.

Biologische Wirkungen und Anwendungsbereiche der neuen Borneolderivate:

Die neuen Verbindungen der Formel I sind wertvolle Pharmaka. Sie interagieren mit Tubulin, indem sie gebildete Mikrotubuli stabilisieren und sind somit in der Lage, die Zellteilung phasenspezifisch zu beeinflussen. Dies betrifft vor allem schnell wachsende, neoplastische Zellen, deren Wachstum durch interzelluläre Regelmechanismen weitgehend unbeeinflußt ist. Wirkstoffe dieser Art sind prinzipiell geeignet zur Behandlung von Erkrankungen, bei denen die Beeinflussung der Zellteilung therapeutisch indiziert sein kann.
Exemplarisch genannt seien hier die Behandlung maligner Tumoren, der Malaria, die Therapie von Erkrankungen welche durch gram-negative Bakterien verursacht sind, sowie die Behandlung von Erkrankungen des zentralen und peripheren Nervensystems, die auf exzitotoxischen Mechanismen beruhen wie z.B. die Therapie akuter neurodegenerativer Erscheinungen, wie sie beispielsweise durch Schlaganfall oder traumatische Hirnverletzungen entstehen, die Therapie chronischer neurodegenerativer Erscheinungen einschließlich des Morbus Alzheimer sowie die Therapie der amyothrophen Lateralsklerose.
Als Anwendungsbereich für maligne Tumoren seien beispielweise genannt die Therapie von Ovarial-, Magen-, Colon-, Adeno-, Brust-, Lungen-, Kopf- und Nacken-Karzinomen, dem malignen Melanom, der akuten lymphozytären und myelocytären Leukämie.
Die erfindungsgemäßen Verbindungen können generell alleine oder zur Erzielung additiver oder synergistischer Wirkungen in Kombination mit weiteren in den jeweiligen Therapiegebieten anwendbaren Prinzipien und Substanzklassen verwendet werden.
Am Beispiel der Tumortherapie seien genannt die Kombination mit
○ Platinkomplexen wie z.B. Cisplatin, Carboplatin,
○ interkalierenden Substanzen z.B. aus der Klasse der Anthracycline wie z.B. Doxorubicin oder aus der Klasse der Antrapyrazole wie z.B. CI-941,
○ mit Tubulin interagierenden Substanzen z.B. aus der Klasse der Vinka-Alkaloide wie z.B. Vincristin, Vinblastin oder aus der Klasse der Taxane wie z.B. Taxol, Taxotere oder aus der Klasse der Makrolide wie z.B. Rhizoxin oder andere Verbindungen wie z.B. Colchicin, Combretastatin A-4,
○ DNA Topoisomeraseinhibitoren wie z.B. Camptothecin, Etoposid, Topotecan, Teniposid,
○ Folat- oder Pyrimidin-Antimetaboliten wie z.B. Lometrexol, Gemcitubin,
○ DNA alkylierenden Verbindungen wie z.B. Adozelesin, Dystamycin A,
○ Inhibitoren von Wachstumsfaktoren (z.B. von PDGF, EGF, TGFβ, EGF) wie z.B. Somatostatin, Suramin, Bombesin-Antagonisten,
○ Inhibitoren der Protein Tyrosin Kinase oder der Protein Kinasen A oder C wie z.B. Erbstatin, Genistein, Staurosporin, Ilmofosin, 8-Cl-cAMP,
○ Antihormonen aus der Klasse der Antigestagene wie z.B. Mifepriston, Onapriston oder aus der Klasse der Antiöstrogene wie z.B. Tamoxifen oder aus der Klasse der Antiandrogene wie z.B. Cyproteronacetat,
○ Metastasen inhibierenden Verbindungen z.B. aus der Klasse der Eicosanoide wie z.B. PGI₂, PGE₁, 6-Oxo-PGE₁ sowie deren stabiler Derivate (z.B. Iloprost, Cicaprost, Beraprost),
○ Inhihitoren des transmembranären Ca²⁺-Influx wie z.B. Verapamil, Galopamil, Flunarizin, Diltiazem, Nifedipin, Nimodipin,
○ Neuroleptika wie z.B. Chlorpromazin, Trifuoperazin, Thioridazin, Perphenazin,
○ Lokalanästhetika wie z.B. Carbanilat-Ca7, Cinchocain, Carbanilat-Ca3, Articain, Carbanilat, Lidocain.
○ die Angiogenese inhibierenden Substanzen wie z.B. anti-VEGF-Antikörpern, Endostatin B, Interferon α, AGM 1470,
○ Inhibitoren der Zellproliferation bei Psoriasis, Kaposisarcom, Neuroblastom.

Die Erfindung betrifft auch Arzneimittel auf Basis der pharmazeutisch verträglichen, d.h. in den verwendeten Dosen nicht toxischen Verbindungen der allgemeinen Formel I, gegebenenfalls zusammen mit den üblichen Hilfs- und Trägerstoffen.
Die erfindungsgemäßen Verbindungen können nach an sich bekannten Methoden der Galenik zu pharmazeutischen Präparaten für die enterale, percutane, parenterale oder lokale Applikation verarbeitet werden. Sie können in Form von Tabletten, Dragees, Gelkapseln, Granulaten, Suppositorien, Implantaten, injizierbaren sterilen wäßrigen oder öligen Lösungen, Suspensionen oder Emulsionen. Salben, Cremes und Gelen verabreicht werden.
Der oder die Wirkstoffe können dabei mit den in der Galenik üblichen Hilfsstoffen wie z.B. Gummiarabikum, Talk, Starke, Mannit, Methylcellulose, Laktose, Tensiden wie Tweens oder Myrj, Magnesiumstearat, wäßrigen oder nicht wäßrigen Trägern, Paraffinderivaten, Netz-, Dispergier-, Emulgier-, Konservierungsmitteln und Aromastoffen zur Geschmackskorrektur (z.B. etherischen Ölen) gemischt werden.
Die Erfindung betrifft somit auch pharmazeutische Zusammensetzungen, die als Wirkstoff zumindest eine erfindungsgemäße Verbindung enthalten. Eine Dosiseinheit enthält etwa 0`1-100 mg Wirkstoff(e). Die Dosierung der erfindungsgemäßen Verbindungen liegt beim Menschen bei etwa 0,1-1000 mg pro Tag.

Die nachfolgenden Ausführungsbeispiele dienen zur weiteren Erläuterung des erfindungsgemäßen Verfahrens.

### BEISPIEL 1

### [1R-[1α,2β(2R*,3S*),4α,4aβ,10aβ]]-3-(Ethoxycarbonyl)amino-2-hydroxy-3-)4-methoxyphenyl)-propansäure-9-methylen-1,2,3,4,4a,9,10,10a-octahydro-1,12,12-trimethyl-4a,10a-epoxy-1,4-methanophenanthren-2-ylester (A) und [1S-[1α,2β(2R*,3S*),4α,4aβ,10aβ]]-3-(Ethoxycarbonyl)amino-2-hydroxy-3-(4-methoxyphenyl)-propansäure-9-methylen-1,2,3,4,4a,9,10,10a-octahydro-1,12,12-trimethyl-4a,10a-epoxy-1,4-methanophenanthren-2-ylester (B)

18 mg (26 µmol) der nach Beispiel 1a dargestellten Verbindungen löst man unter einer Atmosphäre aus trockenem Argon in 0,3 ml wasserfreiem Tetrahydrofuran, versetzt mit 39 µl einer 1,1M Lösung von Tetrabutylammoniumfluorid in Tetrahydrofuran und rührt 0,5 Stunden bei 23°C. Man engt ein und reinigt den Rückstand durch Chromatographie an einer analytischen Dünnschichtplatte. Als Laufmittel dient ein Gemisch aus n-Hexan und Ethylacetat, als Elutionsmittel ein Gemisch aus Diethylether in 2-Propanol. Isoliert werden 3 mg (5,5 µmol, 21%) der Titelverbindung A sowie 4 mg (7,3 µmol, 28%) der Titelverbindung B, jeweils als farbloses Öl.
¹H-NMR (CDCl₃) von A: δ = 0,87 (3H), 0,93 (3H), 1,09 (3H), 1,23 (3H), 1,76 (1H), 2,31 (1H), 2,71 (1H), 2,79 (1H), 3,10 (1H), 3,14 (1H), 3,76 (3H), 4,12 (2H), 4,51 (1H), 4,93 (1H), 5,05 (1H), 5,20 (1H), 5,52 (1H), 6,33 (1H), 6,81 (2H), 7,28-7,43 (4H), 7,51 (1H), 7,56 (1H) ppm.
¹H-NMR (CDCl₃) von B: δ = 0,85 (3H), 0,95 (3H), 1,08 (3H), 1,21 (3H), 1,82 (1H), 2,39 (1H), 2,70 (1H), 2,72 (1H), 3,17 (1H, 3,20 (1H), 3,80 (3H), 4,10 (2H), 4,54 (1H), 5,03 (1H), 5,10 (1H), 5,26 (1H), 5,50 (1H), 5,65 (1H), 6,88 (2H), 7,22-7,39 (4H), 7,45 (1H), 7,51 (1H) ppm.

### BEISPIEL 1a

### [1R-[1α,2β(2R*,3S*),4α,4aβ,10aβ]]-3-(Ethoxycarbonyl)amino-2-(triisopropylsilyloxy)-3-(4-methoxyphenyl)-propansäure-9-methylen-1,2,3,4,4a,9,10,10a-octahydro-1,12,12-trimethyl-4a,10a-epoxy-1,4-methanophenanthren-2-ylester und [1S-[1α,2β(2R*,3S*),4α,4aβ,10aβ]]-3-(Ethoxycarbonyl)amino-2-(triisopropylsilyloxy)-3-(4-methoxyphenyl)-propansäure-9-methylen-1,2,3,4,4a,9,10,10a-octahydro-1,12,12-trimethyl-4a,10a-epoxy-1,4-methanophenanthren-2-ylester

30 mg (106 µmol) eines ca. 6:4-Gemisches aus [1R-(1α,2β,4α,4aβ,10aβ)]-9-methylen-1, 2,3,4,4a,9,10,10a-octahydro-1,12,12-trimethyl-4a,10a-epoxy-1,4-methanophenanthren-2-ol und [1S-(1α,2β,4α,4aβ,10aβ)]-9-methylen-1,2,3,4,4a,9,10,10a-octahydro-1,12,12-trimethyl-4a,10a-epoxy-1,4-methanophenanthren-2-ol, das man in Analogie zu dem in J. Am. Chem Soc. 1992, auf Seite 5879ff beschriebenen Verfahren hergestellt hat, sowie 70 mg (3R,4S)-1-(Ethoxycarbonyl)-3-triisopropylsilyloxy-4-(4-methoxyphenyl)-2-azetidinon, das man in Analogie zu dem in Tetrahedron 1992 auf Seite 6985ff beschriebenen Verfahren hergestellt hat, löst man unter einer Atmosphäre aus trockenem Argon in 1,1 ml wasserfreiem Tetrahydrofuran, versetzt bei 0°C mit 0,14 ml einer 1M Lösung von Natriumhexamethyldisilazan in Tetrahydrofuran, läßt auf 23°C erwärmen und rührt 2 Stunden nach. Man gießt in gesättigte Ammoniumchloridlösung, extrahiert mehrfach mit Ethylacetat, wäscht die vereinigten organischen Extrakte mit Wasser und gesättigter Natriumchloridlösung und trocknet über Natriumsulfat. Den nach Filtration und Lösungsmittelabzug erhaltenen Rückstand trennt man durch Chromatographie an einer analytischen Dünnschichtplatte. Als Laufmittel dient ein Gemisch aus n-Hexan und Ethylacetat, als Elutionsmittel ein Gemisch aus Diethylether in 2-Propanol. Isoliert werden 25 mg (36 µmol, 34%) der Titelverbindungen, die ohne Trennung weiter umgesetzt werden, als farbloses Öl.

### BEISPIEL 2

### [1R-[1α,2β(2R*,3S*),4α,4aβ,10aβ]]-3-(Ethoxycarbonyl)amino-2-hydroxy-3-(4-fluorphenyl)-propansäure-9-methylen-1,2,3,4,4a,9,10,10a-octahydro-1,12,12-trimethyl-4a,10a-epoxy-1,4-methanophenanthren-2-ylester (A) und [1S-[1α,2β(2R*,3S*),4α,4aβ,10aβ]]-3-(Ethoxycarbonyl)amino-2-hydroxy-3-(4-fluorphenyl)-propansäure-9-methylen-1,2,3,4,4a,9,10,10a-octahydro-1,12,12-trimethyl-4a,10a-epoxy-1,4-methanophenanthren-2-ylester (B)

In Analogie zu Beispiel 1 werden 10 mg (14 µmol) der nach Beispiel 2a dargestellten Verbindungen umgesetzt. Nach Aufarbeitung und Reinigung isoliert man 2,4 mg (4,5 µmol, 32%) der Titelverbindung A sowie 2,2 mg (4,1 µmol, 29%) der Titelverbindung B, jeweils als farbloses Öl.
¹H-NMR (CDCl₃) von A: δ = 0,86 (3H), 0,93 (3H), 1.08 (3H), 1,21 (3H), 1,69 (1H), 2,31 (1H), 2,72 (1H), 2,79 (1H), 3,11 (1H), 3,16 (1H), 4,10 (2H), 4,51 (1H), 4,92 (1H), 5,06 (1H), 5,24 (1H), 5,53 (1H), 6,39 (1H), 6,97 (2H), 7,32 (2H), 7,40-7,60 (4H) ppm.
¹H-NMR (CDCl₃) von B: δ = 0,85 (3H), 0,97 (3H), 1,07 (3H), 1,22 (3H), 1,81 (1H), 2,38 (1H), 2,70 (1H), 2,72 (1H), 3,17 (1H), 3,22 (1H), 4,10 (2H), 4,53 (1H), 5,04 (1H), 5,09 (1H), 5,28 (1H), 5,49 (1H), 5,68 (1H), 7,03 (2H), 7,23-7,43 (4H), 7,45 (1H), 7,51 (1H) ppm.

### BEISPIEL 2a

### [1R-[1α,2β(2R*,3S*),4α,4aβ,10aβ]]-3-(Ethoxycarbonyl)amino-2-(triisopropylsilyloxy)-3-(4-fluorphenyl)-propansäure-9-methylen-1,2,3,4,4a,9,10,10a-octahydro-1,12,12-trimethyl-4a,10a-epoxy-1,4-methanophenanthren-2-ylester und [1S-[1α,2β(2R*,3S*),4α,4aβ,10aβ]]-3-(Ethoxycarbonyl)amino-2-(triisopropylsilyloxy)-3-(4-fluorphenyl)-propansäure-9-methylen-1,2,3,4,4a,9,10,10a-octahydro-1,12,12-trimethyl-4a,10a-epoxy-1,4-methanophenanthren-2-ylester

In Analogie zu Beispiel 1a werden 20 mg (70 µmol) eines ca. 7:3-Gemisches aus [1R-(1α,2β,4α,4aβ,10aβ)]-9-methylen-1,2,3,4,4a,9,10,10a-octahydro-1,12,12-trimethyl-4a,10a-epoxy-1,4-methanophenanthren-2-ol und [1S-(1α,2β,4α,4aβ,10aβ)]-9-methylen-1,2,3,4,4a,9,10,10a-octahydro-1,12,12-trimethyl-4a,10a-epoxy-1,4-methanophenanthren-2-ol unter Verwendung von (3R,4S)-1-(Ethoxycarbonyl)-3-triisopropylsilyloxy-4-(4-fluorphenyl)-2-azetidinon, das man in Analogie zu dem in Tetrahedron 1992 auf Seite 6985ff beschriebenen Verfahren hergestellt hat, umgesetzt. Nach Aufarbeitung und Reinigung isoliert man 31 mg (45 µmol, 64%) der Titelverbindungen als farbloses Öl.

### BEISPIEL 3

### [1R-[1α,2β(2R*,3S*),4α,4aβ,10aβ]]-3-(Ethoxycarbonyl)amino-2-hydroxy-3-(4-hydroxyphenyl)-propansäure-9-methylen-1,2,3,4,4a,9,10,10a-octahydro-1,12,12-trimethyl-4a,10a-epoxy-1,4-methanophenanthren-2-ylester (A) und [1S-[1α,2β(2R*,3S*),4α,4aβ,10aβ]]-3-(Ethoxycarbonyl)amino-2-hydroxy-3-(4-hydroxyphenyl)-propansäure-9-methylen-1,2,3,4,4a,9,10,10a-octahydro-1,12,12-trimethyl-4a,10a-epoxy-1,4-methanophenanthren-2-ylester (B)

In Analogie zu Beispiel 1 werden 16 mg (20 µmol) der nach Beispiel 3a dargestellten Verbindungen umgesetzt. Nach Aufarbeitung und Reinigung isoliert man 2,6 mg (4,9 µmol, 24%) der Titelverbindung A sowie 2,9 mg (5,4 µmol, 27%) der Titelverbindung B, jeweils als farbloses Öl.
¹H-NMR (CDCl₃) von A: δ = 0,85 (3H), 0,93 (3H), 1,07 (3H), 1,21 (3H), 1,73 (1H), 2,30 (1H), 2,71 (1H), 2,78 (1H), 3,13 (2H), 4,10 (2H), 4,49 (1H), 4,92 (1H), 5,04 (1H), 5,18 (1H), 5,52 (1H), 6,33 (1H), 6,69 (2H), 8,22-7,39 (4H), 7,50 (1H), 7,55 (1H) ppm.
¹H-NMR (CDCl₃) von B: δ = 0,84 (3H), 0,95 (3H), 1.07 (3H), 1,22 (3H), 1,81 (1H), 2,38 (1H), 2,70 (1H), 2,72 (1H), 3,18 (1H), 3,21 (1H) 4,09 (2H), 4,51 (1H), 5,03 (1H), 5,10 (1H), 5,22 (1H), 5,40 (1H), 5,50 (1H), 5,67 (1H), 6,72 (2H), 7,16-7,33 (4H), 7,46 (1H), 7,52 (1H) ppm.

### BEISPIEL 3a

### [1R-[1α,2β(2R*3S*),4α,4aβ,10aβ]]-3-(Ethoxycarbonyl)amino-2-(triisopropylsilyloxy)-3-(4-hydroxyphenyl)-propansäure-9-methylen-1,2,3,4,4a,9,10,10a-octahydro-1,12,12-trimethyl-4a,10a-epoxy-1,4-methanophenanthren-2-ylester und [1S-[1α,2β(2R*,3S*),4α,4aβ,10aβ]]-3-(Ethoxycarbonyl)amino-2-(triisopropylsilyloxy)-3-(4-hydroxyphenyl)-propansäure-9-methylen-1,2,3,4,4a,9,10,10a-octahydro-1,12,12-trimethyl-4a,10a-epoxy-1,4-methanophenanthren-2-ylester

In Analogie zu Beispiel 1a werden 121 mg (428 µmol) eines ca. 7:3-Gemisches aus [1R-(1α,2β,4α,4aβ,10aβ)]-9-methylen-1,2,3,4,4a,9,10,10a-octahydro-1,12,12-trimethyl-4a,10a-epoxy-1,4-methanophenanthren-2-ol und [1S-(1α,2β,4α,4aβ,10aβ)]-9-methylen-1,2,3,4,4a,9,10,10a-octahydro-1,12,12-trimethyl-4a,10a-epoxy-1,4- methanophenanthren-2-ol unter Verwendung von (3R,4S)-1-(Ethoxycarbonyl)-3-triisopropylsilyloxy-4-[4-(tert.-butyldimethylsilyloxy)phenyl]-2-azetidinon, das man in Analogie zu dem in Tetrahedron 1992 auf Seite 6985ff beschriebenen Verfahren hergestellt hat, umgesetzt. Nach Aufarbeitung und Reinigung isoliert man 180 mg (224 µmol, 52%) der Titelverbindungen als farbloses Öl.

### BEISPIEL 4

### [1R-[1α,2β(2R*,3S*),4α,4aβ,10aβ]]-3-(Ethoxycarbonyl)amino-2-hydroxy-3-(3-methylphenyl)-propansäure-9-methylen-1,2,3,4,4a,9,10,10a-octahydro-1,12,12-trimethyl-4a,10a-epoxy-1,4-methanophenanthren-2-ylester

In Analogie zu Beispiel 1 werden 14 mg (20 µmol) der nach Beispiel 4a dargestellten Verbindung umgesetzt. Nach Aufarbeitung und Reinigung isoliert man 7 mg (13 µmol, 66%) der Titelverbindung als farbloses Öl.
¹H-NMR (CDCl₃): δ = 0,86 (3H), 0,93 (3H), 1,08 (3H), 1,23 (3H), 1,78 (1H), 2,27 (3H), 2,32 (1H), 2,72 (1H), 2,79 (1H), 3,08 (1H), 3,14 (1H), 4,11 (2H), 4,52 (1H), 4,93 (1H), 5,06 (1H), 5,21 (1H), 5,53 (1H), 6,36 (1H), 7,04 (1H), 7,16 (1H), 7,21-7,36 (4H), 7,49 (1H), 7,55 (1H) ppm.

### BEISPIEL 4a

### [1R-[1α,2β(2R*,3S*),4α,4aβ,10aβ]]-3-(Ethoxycarbonyl)amino-2-(triisopropylsilyloxy)-3-(3-methylphenyl)-propansäure-9-methylen-1,2,3,4,4a,9,10,10a-octahydro-1,12,12-trimethyl-4a,10a-epoxy-1,4-methanophenanthren-2-ylester

In Analogie zu Beispiel 1a werden 30 mg (106 µmol) [1R-(1α,2β,4α,4aβ,10aβ)]-9 methylen-1,2,3,4,4a,9,10,10a-octahydro-1,12,12-trimethyl-4a,10a-epoxy-1,4-methanophenanthren-2-ol unter Verwendung von (3R,4S)-1-(Ethoxycarbonyl)-3-triisopropylsilyloxy-4-(3-methylphenyl)-2-azetidinon, das man in Analogie zu dem in Tetrahedron 1992 auf Seite 6985ff beschriebenen Verfahren hergestellt hat, umgesetzt. Nach Aufarbeitung und Reinigung isoliert man 17 mg (25 µmol, 23%) der Titelverbindung als farbloses Öl.
¹H-NMR (CDCl₃): δ = 0,82 (3H), 0,91 (3H), 0,86-1,19 (24H), 1,23 (3H), 1,60 (1H), 2,23 (1H), 2,27 (3H), 2,64 (1H), 2,74 (1H), 3,10 (1H), 4,11 (2H), 4,63 (1H), 4,89 (1H), 5,03 (1H), 5,20 (1H), 5,51 (1H), 6,35 (1H), 7,01 (1H), 7,13 (1H), 7,19-7,35 (4H), 7,45 (1H), 7,53 (1H) ppm.

### BEISPIEL 5

### [1R-[1α,2β(2R*,3S*),4α,4aβ,10aβ]]-3-(Ethoxycarbonyl)amino-2-hydroxy-3-(2-naphtyl)-propansäure-9-methylen-1,2,3,4,4a,9,10,10a-octahydro-1,12,12-trimethyl-4a,10a-epoxy-1,4-methanophenanthren-2-ylester

In Analogie zu Beispiel 1 werden 8 mg (11 µmol) der nach Beispiel 5a dargestellten Verbindung umgesetzt. Nach Aufarbeitung und Reinigung isoliert man 3 mg (5,3 µmol 48%) der Titelverbindung als farbloses Öl.
¹H-NMR (CDCl₃): δ = 0,86 (3H), 0,91 (3H), 1,07 (3H), 1,23 (3H), 1,74 (1H), 2,23 (1H), 2,71 (1H), 2,80 (1H), 3,13 (1H), 3,18 (1H), 4,12 (2H), 4,61 (1H), 4,91 (1H), 5,07 (1H), 5,43 (1H), 5,56 (1H), 6,53 (1H), 7,34 (2H), 7,42 (2H), 7,50-7,65 (3H), 7,65-7,87 (3H), 7,97 (1H) ppm.

### BEISPIEL 5a

### [1R-[1α,2β(2R*,3S*),4α,4aβ,10aβ]]-3-(Ethoxycarbonyl)amino-2-(triisopropylsilyloxy)-3-(2-naphtyl)-propansäure-9-methylen-1,2,3,4,4a,9,10,10a-octahydro-1,12,12-trimethyl-4a,10a-epoxy-1,4-methanophenanthren-2-ylester

In Analogie zu Beispiel 1a werden 5 mg (18 µmol) [1R-(1α,2β,4α,4aβ,10aβ)]-9-methylen-1,2,3,4,4a,9,10,10a-octahydro-1,12,12-trimethyl-4a,10a-epoxy-1,4-methanophenanthren-2-ol unter Verwendung von (3R,4S)-1-(Ethoxycarbonyl)-3-triisopropylsilyloxy-4-(2-naphtyl)-2-azetidinon, das man in Analogie zu dem in Tetrahedron 1992 auf Seite 6985ff beschriebenen Verfahren hergestellt hat, umgesetzt. Nach Aufarbeitung und Reinigung isoliert man 8 mg (11 µmol, 61%) der Titelverbindung als farbloses Öl.
¹H-NMR (CDCl₃): δ = 0,71-1,35 (34H), 1,51 (1H), 2,11 (1H), 2,61 (1H), 2,75 (1H), 3,12 (1H), 4,14 (1H), 4,73 (1H), 4,83 (1H), 5,04 (1H), 5,40 (1H), 5,53 (1H), 6,55 (1H), 7,22-7,86 (10H), 7,93 (1H) ppm.

### BEISPIEL 6

### [1R-[1α,2β(2R*,3S*),4α,4aβ,10aβ]]-3-(Ethoxycarbonyl)amino-2-hydroxy-3-(4-chlorphenyl)-propansäure-9-methylen-1,2,3,4,4a,9,10,10a-octahydro-1,12,12-trimethyl-4a,10a-epoxy-1,4-methanophenanthren-2-ylester

In Analogie zu Beispiel 1 werden 14 mg (20 µmol) der nach Beispiel 6a dargestellten Verbindung umgesetzt. Nach Aufarbeitung und Reinigung isoliert man 8,7 mg (16 µmol, 79%) der Titelverbindung als farbloses Öl.
¹H-NMR (CDCl₃): δ = 0,84 (3H), 0,92 (3H), 1,06 (3H), 1,21 (3H), 1,68 (1H), 2,30 (1H), 2,71 (1H), 2,79 (1H), 3,12 (1H), 3,16 (1H), 4,10 (2H), 4,51 (1H), 4,92 (1H), 5,05 (1H), 5,23 (1H), 5,53 (1H), 6,40 (1H), 7,20-7,38 (4H), 7,42 (2H), 7,50 (1H), 7,56 (1H) ppm.

### BEISPIEL 6a

### [1R-[1α,2β(2R*,3S*),4α,4aβ,10aβ]]-3-(Ethoxycarbonyl)amino-2-(triisopropylsilyloxy)-3-(4-chlorphenyl)-propansäure-9-methylen-1,2,3,4,4a,9,10,10a-octahydro-1,12,12-trimethyl-4a,10a-epoxy-1,4-methanophenanthren-2-ylester

In Analogie zu Beispiel 1a werden 10 mg (35 µmol) [1R-(1α,2β,4α,4aβ,10aβ)]-9-methylen-1,2,3,4,4a,9,10,10a-octahydro-1,12,12-trimethyl-4a,10a-epoxy-1,4-methanophenanthren-2-ol unter Verwendung von (3R,4S)-1-(Ethoxycarbonyl)-3-triisopropyl silyloxy-4-(4-chlorphenyl)-2-azetidinon, das man in Analogie zu dem in Tetrahedron 1992 auf Seite 6985ff beschriebenen Verfahren hergestellt hat, umgesetzt. Nach Aufarbeitung und Reinigung isoliert man 14 mg (20 µmol, 56%) der Titelverbindung als farbloses Öl.
¹H-NMR (CDCl₃): δ = 0,82 (3H), 0,90 (3H), 0,86-1,32 (27H), 1,45 (1H), 2,20 (1H), 2,63 (1H), 2,75 (1H), 3,11 (1H), 4,11 (2H), 4,64 (1H), 4,87 (1H), 5,03 (1H), 5,21 (1H), 5,51 (1H), 6,45 (1H), 7,18-7,60 (8H) ppm.

### BEISPIEL 7

### [1R-[1α,2β(2R*,3S*),4α,4αβ,10aβ]]-3-(Ethoxycarbonyl)amino-2-hydroxy-3-(4-trifluormethylphenyl)-propansäure-9-methylen-1,2,3,4,4a,9,10,10a-octahydro-1,12,12-trimethyl-4a,10a-epoxy-1,4-methanophenanthren-2-ylester

In Analogie zu Beispiel 1 werden 13 mg (18 µmol) der nach Beispiel 7a dargestellten Verbindung umgesetzt. Nach Aufarbeitung und Reinigung isoliert man 8,2 mg (14 µmol, 78%) der Titelverbindung als farbloses Öl.
¹H-NMR (CDCl₃): δ = 0,85 (3H), 0,93 (3H), 1,07 (3H), 1,22 (3H), 1,68 (1H), 2,31 (1H), 2,73 (1H), 2,79 (1H), 3,13(1H), 3,20 (1H), 4,12 (2H), 4,56 (1H), 4,92 (1H), 5,06 (1H), 5,32 (1H), 5,53 (1H), 6,44 (1H), 7,32 (2H), 7,43-7,68 (6H) ppm.

### BEISPIEL 7a

### [1R-[1α,2β(2R*,3S*),4α,4aβ,10aβ]]-3-(Ethoxycarbonyl)amino-2-(triisopropylsilyloxy)-3-(4-trifluormethylphenyl)-propansaure-9-methylen-1,2,3,4,4a,9,10,10a-octahydro-1,12,12-trimethyl-4a,10a-epoxy-1,4-methanophenanthren-2-ylester

In Analogie zu Beispiel 1a werden 7 mg (25 µmol) [1R-(1α,2β,4α,4aβ,10aβ)]-9-methylen-1,2,3,4,4a,9,10,10a-octahydro-1,12,12-trimethyl-4a,10a-epoxy-1,4-methanophenanthren-2-ol unter Verwendung von (3R,4S)-1-(Ethoxycarbonyl)-3-triisopropylsilyloxy-4-(4-trifluormethylphenyl)-2-azetidinon, das man in Analogie zu dem in Tetrahedron 1992 auf Seite 6985ff beschriebenen Verfahren hergestellt hat, umgesetzt. Nach Aufarbeitung und Reinigung isoliert man 9 mg (12 µmol, 49%) der Titelverbindung als farbloses Öl.
¹H-NMR (CDCl₃): δ = 0,82 (3H), 0,89 (3H), 0,85-1,30 (30H), 1,42 (1H), 2,19 (1H), 2,64 (1H), 2,76 (1H), 3,10 (1H), 4,12 (2H), 4,68 (1H), 4,86 (1H), 5,03 (1H), 5,29 (1H), 5,52 (1H), 6,48 (1H), 7,30 (2H), 7,41-7,68 (6H) ppm.

### BEISPIEL 8

### [1R-[1α,2β(2R*,3S*),4α,4aβ,10aβ]]-3-(Ethoxycarbonyl)amino-2-hydroxy-3-(4-benzyloxyphenyl)-propansäure-9-methylen-1,2,3,4,4a,9,10,10a-octahydro-1,12,12-trimethyl-4a,10a-epoxy-1,4-methanophenanthren-2-ylester

In Analogie zu Beispiel 1 werden 6 mg (7,7 µmol) der nach Beispiel 8a dargestellten Verbindung umgesetzt. Nach Aufarbeitung und Reinigung isoliert man 3,7 mg (5,9 µmol, 77%) der Titelverbindung als farbloses Öl.
¹H-NMR (CDCl₃): δ = 0,84 (3H), 0,92 (3H), 1,06 (3H), 1,22 (3H), 1,74 (1H), 2,30 (1H), 2,71 (1H), 2,78 (1H), 3,11 (1H), 3,15 (1H), 4,10 (2H), 4,52 (1H), 4,92 (1H), 5,01 (2H), 5,06 (1H), 5,21 (1H), 5,53 (1H), 6,34 (1H), 6,89 (2H), 7,27-7,44 (9H), 7,49 (1H), 7,55 (1H) ppm.

### BEISPIEL 8a

### [1R-[1α,2β(2R*,3S*),4α,4aβ,10aβ]]-3-(Ethoxycarbonyl)amino-2-(triisopropylsilyloxy)-3-(4-benzyloxyphenyl)-propansäure-9-methylen-1,2,3,4,4a,9,10,10a-octahydro-1,12,12-trimethyl-4a,10a-epoxy-1,4-methanophenanthren-2-ylester

In Analogie zu Beispiel 1a werden 11 mg (40 µmol) [IR-(1α,2β,4α,4aβ,10aβ)]-9-methylen-1,2,3,4,4a,9,10,10a-octahydro-1,12,12-trimethyl-4a,10a-epoxy-1,4-methanophenanthren-2-ol unter Verwendung von (3R,4S)-1-(Ethoxycarbonyl)-3-triisopropylsilyloxy-4-(4-benzyloxyphenyl)-2-azetidinon, das man in Analogie zu dem in Tetrahedron 1992 auf Seite 6985ff beschriebenen Verfahren hergestellt hat, umgesetzt. Nach Aufarbeitung und Reinigung isoliert man 29 mg (37 µmol, 93%) der Titelverbindung als farbloses Öl.
¹H-NMR (CDCl₃): δ = 0,82 (3H), 0,85-1,29 (30H), 1,50 (1H), 2,18 (1H), 2,62 (1H), 2,74 (1H), 3,11 (1H), 4,11 (2H), 4,63 (1H), 4,88 (1H), 5,02 (3H), 5,19 (1H), 5,51 (1H), 6,40 (1H), 6,85 (2H), 7,22-7,49 (10H), 7,53 (1H) ppm.

### BEISPIEL 9

### [1R-[1α,2β(2R*,3S*),4α,4aβ,9β,10aβ]]-3-(Ethoxycarbonyl)amino-2-hydroxy-3-(4-hydroxyphenyl)-propansäure-9-methyl-1,2,3,4,4a,9,10,10a-octahydro-1,12,12-trimethyl-4a,10a-epoxy-1,4-methanophenanthren-2-ylester

In Analogie zu Beispiel 1 werden 30 mg (37 µmol) der nach Beispiel 9a dargestellten Verbindung umgesetzt. Nach Aufarbeitung und Reinigung isoliert man 11 mg (21 µmol, 56%) der Titelverbindung als farbloses Öl.
¹H-NMR (CDCl₃): δ = 0,78 (3H), 0,89 (3H), 1,01 (3H), 1,17 (3H), 1,40 (3H), 1,72 (1H), 2,08 (1H), 2,20 (1H), 2,29 (1H), 2,68 (1H), 3,06 (1H), 3,21 (1H), 4,06 (2H), 4,50 (1H), 4,92 (1H), 5,18 (1H), 5,51 (1H), 6,29 (1H), 6,68 (2H), 7,11-7,39 (5H), 7,48 (1H) ppm.

### BEISPIEL 9a

### [1R-[1α,2β(2R*,3S*),4α,4aβ,9β,10aβ]]-3-(Ethoxyarbonyl)amino-2-(triisopropylsilyloxy)-3-(4-hydroxyphenyl)-propansäure-9-methyl-1,2,3,4,4a,9,10,10a-octahydro-1,12,12-trimethyl-4a,10a-epoxy-1,4-methanophenanthren-2-ylester

120 mg (149 µmol) der nach Beispiel 3a dargestellten Verbindung A löst man in 6 ml Ethanol, versetzt mit 12 mg Palladium auf Kohle (10%ig) und hydriert bei 1 at unter einer Atmosphäre aus Wasserstoff Nach Aufnahme der theoretisch berechneten Menge wird vom Katalysator abfiltriert, das Filtrat eingeengt und der erhaltene Rückstand durch Chromatographie an ca. 40 ml feinem Kieselgel gereinigt. Als Laufmittel dient ein Gemisch aus n-Hexan und Ethylacetat Isoliert werden 89 mg (110 µmol, 74%) der Titelverbindung als farbloses Öl.
¹H-NMR (CDCl₃): δ = 0,14 (6H), 0,73 (3H), 0,79-1,29 (40H), 1,40 (3H), 1,44 (1H), 2,03 (1H), 2,17 (1H), 2,58 (1H), 3,04 (1H), 4,10 (2H), 4,62 (1H), 4,87 (1H), 5,21 (1H), 6,23 (1H), 6,72 (2H), 7,10-7.37 (5H), 7,43 (1H) ppm.

### BEISPIEL 10

### [1R-[1α,2β(2R*,3S*),4α,4aβ,9β,10aβ]]-3-(Ethoxycarbonyl)amino-2-hydroxy-3-(3-methylphenyl)-propansäure-9-methyl-1,2,3,4,4a,9,10,10a-octahydro-1,12,12-trimethyl-4a,10a-epoxy-1,4-methanophenanthren-2-ylester

In Analogie zu Beispiel 9a werden 6 mg (11 µmol) der nach Beispiel 4 dargestellten Verbindung umgesetzt. Nach Aufarbeitung und Reinigung isoliert man 3 mg (5.6 µmol, 51%) der Titelverbindung als farbloses Öl.
¹H-NMR (CDCl₃): δ = 0,79 (3H), 0,90 (3H), 1,03 (3H), 1,20 (3H), 1,40 (3H), 1,55 (1H), 1,73 (1H), 2,08 (1H), 2,21 (1H), 2,27 (3H), 2,68 (1H), 3.09 (1H), 3,12 (1H), 4,08 (2H), 4,51 (1H), 4,91 (1H), 5,23 (1H), 6,27 (1H), 7,05 (1H), 7,13-7,40 (6H), 7,49 (1H)

### BEISPIEL 11

### [1R-[1α,2β(2R*3S*),4α,4aβ,9β,10β]]-3-(Ethoxycarbonyl)amino-2-hydroxy)-3-(4-chlorphenyl)-propansaure-9-methyl-1,2,3,4,4a,9,10,10a-octahydro-1,12,12-trimethyl-4a,10a-epoxy-1,4-methanophenanthren-2-ylester

In Analogie zu Beispiel 1 werden 2,8 mg (3,9 µmol) der nach Beispiel 11a dargestellten Verbindung umgesetzt. Nach Aufarbeitung und Reinigung isoliert man 2,0 mg (3,6 µmol, 93%) der Titelverhindung als farbloses Öl.
¹H-NMR (CDCl₃): δ = 0,78 (3H), 0,90 (3H), 1,02 (3H), 1,19 (3H), 1,40 (3H), 1,67 (1H), 2,09 (1H), 2,20 (1H), 2,29 (1H), 2,68 (1H), 3,07 (1H), 3,19 (1H), 4,09 (2H), 4,52 (1H), 4,91 (1H), 5,23 (1H), 6,30 (1H), 7,12-7,33 (5H), 7,40-7,55 (3H) ppm.

### BEISPIEL 11a

### [1R-[1α,2β(2R*,3S*),4α4aβ,9β,10aβ]]-3-(Ethoxycarbonyl)amino-2-(triisopropylsilyloxy)-3-(4-chlorphenyl)-propansäure-9-methyl-1,2,3,4,4a,10.10a-octahydro-1,12,12-trimethyl-4a,10a-epoxy-1,4-methanophenanthren-2-ylester

In Analogie zu Beispiel 9a werden 8 mg (11 µmol) der nach Beispiel 6a dargestellten Verbindung umgesetzt Nach Aufarbeitung und Reinigung isoliert man 2,8 mg (3,9 µmol, 36%) der Titelverbindung als farbloses Öl.
¹H-NMR (CDCl₃): δ = 0,74 (3H), 0,79-1,31 (30H), 1,40 (3H), 1,50 (1H), 2,06 (1H), 2,15 (1H), 2,19 (1H), 2,60 (1H), 3,05 (1H), 4,09 (2H), 4,63 (1H), 4,85 (1H), 5,23 (1H), 6,34 (1H), 7,10-7,50 (8H) ppm.

### BEISPIEL 12

### [1R-[1α,2β(2R*,3S*),4α,4aβ,9β,10aβ]]-3-(Ethoxycarbonyl)amino-2-hydroxy-3-(4-trifluormethylphenyl)-propansäure-9-methyl-1,2,3,4,4a,9,10,10a-octahydro-1,12,12-trimethyl-4a,10a-epoxy-1,4-methanophenanthren-2-ylester

In Analogie zu Bespiel 1 werden 4,5 mg (6 µmol) der nach Beispiel 12a dargestellten Verbindung umgesetzt. Nach Aufarbeitung und Reinigung isoliert man 3,2 mg (5,4 µmol, 91%) der Titelverbindung als farbloses Öl.
¹H-NMR (CDCl₃): δ = 0,78 (3H), 0,89 (3H), 1,02 (3H), 1,19 (3H), 1,40 (3H), 1,65 (1H), 2,10 (1H), 2,21 (1H), 2,29 (1H), 2,68 (1H), 3,07 (1H), 3,22 (1H), 4,10 (2H), 4,54 (1H), 4,91 (1H), 5,32 (1H), 6,35 (1H), 7,12-7,34 (3H), 7,44-7,69 (5H) ppm.

### BEISPIEL 12a

### [1R-[1α,2β(2R*,3S*),4α,4aβ,9β,10aβ]]-3-(Ethoxycarbonyl)amino-2-(triisopropylsilyloxy)-3-(4-trifluormethylphenyl)-propansäure-9-methyl-1,2,3,4,4a,9,10,10a-octahydro-1,12,12-trimethyl-4a,10a-epoxy-1,4-methanophenanthren-2-ylester

In Analogie zu Beispiel 9a werden 9 mg (12 µmol) der nach Beispiel 7a dargestellten Verbindung umgesetzt. Nach Aufarbeitung und Reinigung isoliert man 4,5 mg (6,0 µmol, 50%) der Titelverbindung als farbloses Öl.
¹H-NMR (CDCl₃): δ = 0,73 (3H), 0,82-1,30 (31H), 1,41 (4H), 2,06 (1H), 2,18 (1H), 2,60 (1H), 3,06 (1H), 4,10 (2H), 4,67 (1H), 4,85 (1H), 5,31 (1H), 6,37 (1H), 7,10-7,32 (3H), 7,49-7,67 (5H) ppm.

### BEISPIEL 13

### [1R-[1α,2β(2R*,3S*),4α,4aβ,9β,10aβ]]-3-(Ethoxycarbonyl)amino-2-hydroxy-3-(4-benzyloxyphenyl)-propansäure-9-methyl-1,2,3,4,4a,9,10,10a-octahydro-1,12,12-trimethyl-4a,10a-epoxy-1,4-methanophenanthren-2-ylester

In Analogie zu Beispiel 1 werden 7 mg (9 µmol) der nach Beispiel 13a dargestellten Verbindung umgesetzt. Nach Aufarbeinung und Reinigung isoliert man 4,5 mg (7,2 µmol, 80%) der Titelverbindung als farbloses Öl.
¹H-NMR (CDCl₃): δ = 0,79 (3H), 0,89 (3H), 1,02 (3H), 1,20 (3H), 1,40 (3H), 1,71 (1H), 2,08 (1H), 2,21 (1H), 2,28 (1H), 2,67 (1H), 3,07 (1H), 3,16 (1H), 4,09 (2H), 4,51 (1H), 4,90 (1H), 5,02 (2H), 5,21 (1H), 6,26 (1H), 6,90 (2H), 7,12-7,55 (11H) ppm.

### BEISPIEL 13a

### [1R-[1α,2β(2R*,3S*),4α,4aβ,9β,10aβ]]-3-(Ethoxycarbonyl)amino-2-(triispronylsilyloxy)-3-(4-benzyloxyphenyl)-propansäure-9-methyl-1,2,3,4,4a,10,10a-octahydro-1,12,12-trimethyl-4a,10a-epoxy-1,4-methanophenanthren-2-ylester

In Analogie zu Beispiel 9a werden 23 mg (30 µmol) der nach Beispiel 8a dargestellten Verbindung umgesetzt. Nach Aufarbeitung und Reinigung isoliert man 7 mg (9,0 µmol, 30%) der Titelverbindung als farbloses Öl.
¹H-NMR (CDCl₃): δ = 0,73 (3H), 0,87 (3H), 0,90-1,30 (28H), 1,41 (3H), 1,50 (1H), 2,04 (1H), 2,18 (1H), 2,58 (1H), 3,05 (1H), 4,09 (2H), 4,62 (1H), 4,85 (1H), 5,02 (2H), 5,21 (1H), 6,28 (1H), 6,87 (2H), 7,08-7,52 (11H) ppm.

### BEISPIEL 14

### [1R-[1α,2β[2R*,3S*(4S*)1,4α,4aβ,9β,10aβ]]-3-(Ethoxycarbonyl)amino-2-triisopropylsilyloxy-3-[4-(4-(1-methylethylen)cyclohex-1-en-carbonyloxy)phenyl]-propansäure-9-methyl-1,2,3,4,4a,9,10,10a-octahydro-1,12,12-trimethyl-4a,10a-epoxy-1,4-methanophenanthren-2-ylester

10 mg (19 µmol) der nach Beispiel 9 dargestellten Verbindung löst man in 0.3 ml wasserfreiem Dichlormethan, versetzt bei 0°C unter einer Atmosphäre aus trocknem Argon mit 2,6 µl Triethylamin, mit einem Moläquivalent einer Lösung von L-(-)-Perillsäurechlorid in Dichlormethan und läβt 3 Stunden rühren. Man gießt in eine gesättigte Natriumhydrogencarbonatlösung, extrahiert mit Dichlormethan und trocknet die organische Phase über Natriumsulfat Den nach Filtration und Lösungsmittelabzug erhaltenen Rückstand reinigt man durch Chromatographie an einer analytischen Dünnschichtplatte. Als Laufmittel dient ein Gemisch aus n-Hexan und Ethylacetat, als Elutionsmittel ein Gemisch aus Diethylether in 2-Propanol. Isoliert werden 3 mg (4,4 µmol, 23%) der Titelverbindung als farbloses Öl.
¹H-NMR (CDCl₃): δ = 0,78 (3H), 0,89 (3H), 1,02 (3H), 1,19 (3H), 1,40 (3H), 1,53 (1H), 1,71 (1H), 1,77 (3H), 1,94 (1H), 2,08 (1H), 2,13-2,49 (6H), 2,57 (1H), 2,67 (1H), 3,07 (1H), 3,22 (1H), 4,10 (2H), 4,52 (1H), 4,77 (2H), 4,91 (1H), 5,28 (1H), 6,26 (1H), 7,03 (2H), 7,12-7,31 (4H), 7,41-7,59 (3H) ppm.

### BEISPIEL 15

### [1R-[1α,2β[2R*,3S*(4S*)],4α,4aβ,9β,10aβ]]-3-(Ethoxycarbonyl)amino-2-hydroxy-3-[4-(diethoxyphosphinyloxy)phenyl]-propansäure-9-methyl-1,2,3,4,4a,9,10,10a-octahydro-1,12,12-trimethyl-4α,10a-epoxy-1,4-methanophenanthren-2-ylester

In Analogie zu Beispiel 1 werden 1,6 mg (1,9 µmol) der nach Beispiel 15a dargestellten Verbindung umgesetzt. Nach Aufarbeitung und Reinigung isoliert man 0,9 mg (1,3 µmol, 71%) der Titelverbindung als farbloses Öl.
¹H-NMR (CDCl₃): δ = 0,78 (3H), 0,89 (3H), 1,01 (3H), 1,19 (3H), 1,32 (6H), 1,40 (3H), 1,69 (1H), 2,08 (1H), 2,20 (1H), 2,27 (1H), 2,67 (1H), 3,07 (1H), 3,15 (1H), 4,09 (2H), 4,18 (4H), 4,51 (1H), 4,91 (1H), 5,25 (1H), 6,26 (1H), 7,10-7,32 (5H), 7,43-7,53 (3H) ppm.

### BEISPIEL 15a

### [IR-(1α,2β[2R*,3S*(4S*)],4α,4aβ,9β,10aβ]]-3-(Ethoxycarbonyl)amino-2-(triisopropylsilyloxy)-3-[4-(diethoxyphosphinyloxy)phenyl]-propansäre-9-methyl-1,2,3,4,4a,9,10,10a-octahydro-1,12,12-trimethyl-4a,10a-epoxy-1,4-methanophenanthren-2-ylester

Die Losung von 4,4 mg (6,4 µmol) der nach Beispiel 9a dargestellten Verbindung in 0,2 ml wassefreiem Dimethylforamid versetzt man bei 0°C unter einer Atmosphäre aus trockenem Argon mit 0,3 mg einer 55%igen Natriumhydrid-Dispersion und und nach 30 Minuten mit 1,1 µl Phosphorsäurediethylesterchlorid. Man rührt 5 Stunden bei 23°C, gibt nochmals gleiche Mengen Natriumhydrid und Phosphorsäurediethylesterchlorid zu und rührt eine weitere Stunde. Man gießt in gesättigt Natriumchloridlösung, extrahiert mit Diethylether und trocknet über Magnesiumsulfat. Den nach Filtration und Lösungsmittelabzug erhaltenen Rückstand trennt man durch Chromatographie an einer analytischen Dünnschichtplatte. Als Laufmittel dient ein Gemisch aus n-Hexan und Ethylacetat, als Elutionsmittel ein Gemisch aus Diethylether in 2-Propanol. Isoliert werden 1,6 mg (1,9 µmol, 30%) der Titelverbindung als farbloses Öl.
¹H-NMR (CDCl₃): δ = 0,73 (3H), 0,78-1,37 (37H), 1,40 (3H), 1,51 (1H), 2,05 (1H), 2,18 (1H), 2,59 (1H), 3,04 (1H), 4,09 (2H), 4,17 (4H), 4,63 (1H), 4,85 (1H), 5,25 (1H), 6,30 (1H), 7,06-7,30 (5H), 7,38-7,52 (3H) ppm.

### BEISPIEL 16

### [1R-[1α,2β,(2R*,3S*),4α,4aβ,9β,10aβ]]-3-(Ethoxycarbonyl)amino-2-hydroxy-3-(4-fluorphenyl)-propansäure-9-methyl-1,2,3,4,4a,9,10,10a-octahydro-1,12,12-trimethyl-4a,10a-epoxy-1,4-methanophenanthren-2-ylester (A) und [1S-[1α,2β(2R*,3S*),4α,4aβ,10aβ]]-3-(Ethoxycarbonyl)amino-2-hydroxy-3-(4-fluorphenyl)-propansäure-9-methylen-1,2,3,4,4a,9,10,10a-octahydro-1,12,12-trimethyl-4a,10a-epoxy-1,4-methanophenanthren-2-ylester (B)

In Analogie zu Beispiel 1 werden 13 mg (18,7 µmol) der nach Beispiel 15a dargestellten Verbindung umgesetzt. Nach Aufarbeitung und Reinigung isoliert man 4,5 mg (8,4 µmol, 45%) der Titelverbindung A sowie 3,0 mg (5,6 µmol, 30%) der Titelverbindung B, jeweils als farbloses Öl.
¹H-NMR (CDCl₃) von A: δ = 0,77 (3H), 0.89 (3H), 1,02 (3H), 1,20 (3H), 1,40 (3H), 1,66 (1H), 2,08 (1H), 2,21 (1H), 2,28 (1H), 2,68 (1H), 3,07 (1H), 3,18 (1H), 4,09 (2H), 4,51 (1H), 4,90 (1H), 5,25 (1H), 6,29 (1H), 6,98 (2H), 7,13-7,33 (3H), 7,41-7,56 (3H)
¹H-NMR (CDCl₃) von B: δ = 0,78 (3H), 0,91 (3H), 0,95 (3H), 1,20 (3H), 1,38 (3H), 1,82 (1H), 2,02 (1H), 2,23 (1H), 2,33 (1H), 2,68 (1H), 3,05 (1H), 3,23 (1H), 4,09 (2H), 4,51 (1H), 5,15 (1H), 5,30 (1H), 5,88 (1H), 7,03 (2H), 7,12-7,31 (3H), 7,36·7,50 (3H)

### BEISPIEL 16a

### [1R-[1α,2β(2R*,3S*),4α,4aβ,9β,10aβ]]-3-(Ethoxycarbonyl)amino-2-(triisopropylsilyloxy)-3-(4-fluorphenyl)-propansäure-9-methyl-1,2,3,4,4a,9,10,10a-octahydro1,12,12-trimethyl-4a,10a-epoxy-1,4-methanophenanthren-2-ylester und [1S-[1α,2β(2R*,3S*),4α,4aβ,10aβ]]-3-(Ethoxycarbonyl)amino-2-(triisopropylsilyloxy)-3-(4-fluorphenyl)-propansäure-9-methylen-1,2,3,4,4a,9,10,10a-octahydro-1,12,12-trimethyl-4a,10a-epoxy-1,4-methanophenanthren-2-ylester

In Analogie zu Beispiel 9a werden 21 mg (30 µmol) der nach Beispiel 2a dargestellten Verbindungen umgesetzt. Nach Aufarbeitung und Reinigung isoliert man 13 mg (18,7 µmol, 62%) der Titelverbindungen, die ohne Trennung weiter umgesetzt werden, als farbloses Öl.

### BEISPIEL 17

### [1R-[1α,2β(2R*,3S*),4α,4aβ,9β,10aβ]]-3-(Ethoxycarbonyl)amino-2-hydroxy-3-(4-methoxyphenyl)-propansäure-9-methyl-1,2,3,4,4a,9,10,10a-octahydro-1,12,12-trimethyl-4a,10a-epoxy-1,4-methanophenanthren-2-ylester

In Analogie zu Beispiel 1 werden 43 mg (61 µmol) der nach Beispiel 17a dargestellten Verbindung umgesetzt. Nach Aufarbeitung und Reinigung isoliert man 25 mg (45 µmol, 75%) der Titelverbindung als farbloses Öl.
¹H-NMR (CDCl₃): δ = 0,78 (3H), 0,88 (3H), 1,02 (3H), 1,18 (3H), 1,40 (3H), 1,73 (1H), 2,08 (1H), 2,22 (1H), 2,29 (1H), 2,67 (1H), 3,07 (1H), 3,15 (1H), 3,75 (3H), 4,09 (2H), 4,50 (1H), 4,91 (1H), 5,21 (1H), 6,26 (1H), 6,82 (2H), 7,14-7,23 (3H), 7,42 (2H), 7,50 (1H) ppm.

### BEISPIEL 17a

### [1R-[1α,2β(2R*,3S*),4α,4aβ,9β,10aβ]]-3-(Ethoxycarbonyl)amino-2-(triisopropylsilyloxy)-3-(4-methoxyphenyl)-propansäure-9-methyl-1,2,3,4,4a,9,10,10a-octahydro-1,12,12-trimethyl-4a,10a-epoxy-1,4-methanophenanthren-2-ylester

In Analogie zu Beispiel 9a werden 66 mg (94 µmol) der nach Beispiel 1a dargestellten Verbindung umgesetzt. Nach Aufarbeitung und Reinigung isoliert man 43 mg (61 µmol, 65%) der Titelverbindung als farbloses Öl.
¹H-NMR (CDCl₃): δ = 0,75 (3H), 0,86 (3H), 0,90-1,25 (27H), 1,41 (3H), 1,51 (1H), 2,05 (1H), 2,18 (2H), 2,58 (1H), 3,04 (1H), 3,75 (3H), 4,09 (2H), 4,62 (1H), 4,86 (1H), 5,21 (1H), 6,28 (1H), 6,80 (2H), 7,11-7,50 (6H) ppm.

### BEISPIEL 18

### [1R-[1α,2β(2R*,3S*),4α,4aβ,10aβ]]-3-(Ethoxycarbonyl)amino-2-hydroxy-3-(3-thienyl)-propansäure-9-methylen-1,2,3,4,4a,9,10,10a-octahydro-1,12,12-trimethyl-4a,10a-epoxy-1,4-methanophenanthren-2-ylester

In Analogie zu Beispiel 1 werden 20 mg (29 µmol) der nach Beispiel 18a dargestellten Verbindung umgesetzt. Nach Aufarbeitung und Reinigung isoliert man 8,5 mg (16 µmol, 56%) der Titelverbindung als farbloses Öl.
¹H-NMR (CDCl₃): δ = 0,83 (3H), 0,91 (3H), 1,08 (3H), 1,26 (3H), 1,53 (1H), 2,26 (1H), 2,68 (1H), 2,78 (1H), 3,15 (1H), 3,24 (1H), 4,15 (2H), 4,50 (1H), 4,89 (1H), 5,05 (1H), 5,34 (1H), 5,53 (1H), 6,40 (1H), 7,21 (2H), 7,28-7,40 (3H), 7,50 (1H), 7,56 (1H) ppm.

### BEISPIEL18a

### [1R-[1α,2β(2R*,3S*),4α,4aβ,10aβ]]-3-(Ethoxycarbonyl)amino-2-(triisopropylsilyloxy)-3-(3-thienyl)-propansäure-9-methylen-1,2,3,4,4a,9,10,10a-octahydro-1,12,12-trimethyl-4a,10a-epoxy-1,4-methanophenanthren-2-ylester

In Analogie zu Beispiel 1a werden 30 mg (106 µmol) [1R-(1α,2β,4α,4aβ,10aβ)]-9-methylen-1,2,3,4,4a,9,10,10a-octahydro-1,12,12-trimethyl-4a,10a-epoxy-1,4-methanophenanthren-2-ol unter Verwendung von (3R,4S)-1-(Ethoxycarbonyl)-3-triisopropylsilyloxy-4-(3-thienyl)-2-azetidinon, das man in Analogie zu dem in Tetrahedron 1992 auf Seite 6985ff beschriebenen Verfahren hergestellt hat, umgesetzt. Nach Aufarbeitung und Reinigung isoliert man 66 mg (97 µmol, 92%) der Titelverbindung als farbloses Öl.
¹H-NMR (CDCl₃): δ = 0,78 (3H), 0,85 (3H), 0,96-1,30 (28H), 2,11 (1H), 2,58 (1H), 2,74 (1H), 3,12 (1H), 4,17 (2H), 4,62 (1H), 4,82 (1H), 5,03 (1H), 5,28 (1H), 5,53 (1H), 6,66 (1H), 7,18 (1H), 7,22-7,36 (4H), 7,44 (1H), 7,55 (1H) ppm.

### BEISPIEL 19

### [1R-[1α,2β(2R*,3S*),4α,4aβ,9β,10aβ]]-3-(Ethoxycarbonyl)amino-2-hydroxy-3-(3-thienyl)-propansäure-9-methyl-1,2,3,4,4a,9,10,10a-octahydro-1,12,12-trimethyl-4a,10a-epoxy-1,4-metanophenanthren-2-ylester

In Analogie zu Beispiel 1 werden 15 mg (22 µmol) der nach Beispiel 19a dargestellten Verbindung umgesetzt. Nach Aufarbeitung und Reinigung isoliert man 8,9 mg (17 µmol, 77%) da Titelverbindung als farbloses Öl.
¹H-NMR (CDCl₃): δ = 0,77 (3H), 0,88 (3H), 1,02 (3H), 1,23 (3H), 1,41 (3H), 1,50 (1H), 2,08 (1H), 2,23 (2H), 2,64 (1H), 3,08 (1H), 3,28 (1H), 4,13 (2H), 4,50 (1H), 4,86 (1H), 5,35 (1H), 6,28 (1H), 7,13-7,33 (5H), 7,42 (1H), 7,49 (1H) ppm.

### BEISPIEL 19a

### [1R-[1α,2β(2R*,3S*),4α,4aβ,9β,10aβ]]-3-(Ethoxycarbonyl)amino-2-(triisopropylsilyloxy)-3-(3-thienyl)-propansäure-9-methyl-1,2,3,4,4a,9,10,10a-octahydro-1,12,12-trimethyl-4a,10a-epoxy-1,4-methanophenanthren-2-ylester

In Analogie zu Beispiel 9a werden 40 mg (59 µmol) der nach Beispiel 18a dargestellten Verbindung umgesetzt. Nach Aufarbeitung und Reinigung isoliert man 15 mg (22 µmol, 37%) der Titelverbindung als farbloses Öl.
¹H-NMR (CDCl₃): δ = 0,72 (3H), 0,79-1,30 (31H), 1,42 (3H), 2,05 (2H), 2,21 (1H), 2,53 (1H), 3,05 (1H), 4,15 (2H), 4,62 (1H), 4,82 (1H), 5,30 (1H), 6,48 (1H), 7,10-7,31 (5H), 7,34 (1H), 7,45 (1H) ppm.

### BEISPIEL 20

### [1R-[1α,2β[2R*,3S*(4R*)],4α,4aβ,9β,10aβ]]-3-(Ethoxycarbonyl)amino-2-hydroxy-3-[4-(4-(1-methylethylen)cyclohex-1-en-carbonyloxy)phenyl]-propansäure-9-methyl-1,2,3,4,4a,9,10,10a-octahydro-1,12,12-trimethyl-4a,10a-epoxy-1,4-methanophenanthren-2-ylester

In Analogie zu Beispiel 1 werden 4,2 mg (5,0 µmol) der nach Beispiel 20a dargestellten Verbindung A umgesetzt. Nach Aufarbeitung und Reinigung isoliert man 1,9 mg (2,8 µmol, 56%) der Titelverbindung als farbloses Öl.
¹H-NMR (CDCl₃): δ = 0,78 (3H), 0,89 (3H), 1,03 (3H), 1,20 (3H), 1,40 (3H), 1,71 (1H), 1,78 (3H), 1,95 (1H), 2,09 (1H), 2,14-2,48 (7H), 2,58 (1H), 2,68 (1H), 3,08 (1H), 3,22 (1H), 4,10 (2H), 4,53 (1H), 4,78 (1H), 4,92 (1H), 5,29 (1H), 6,28 (1H), 7,05 (2H), 7,13-7,3,5 (5H), 7,41-7,60 (3H) ppm.

### BEISPIEL 20a

### [1R-[1α,2β[2R*,3S*(4R*)],4α,4aβ,9β,10aβ]]-3-(Ethoxycarbonyl)amino-2-triisopropyhilyloxy-3-[4-(4-(1-methylethylen)cyclohex-1-en-carbonyloxy)phenyl]-propansäure-9-methyl-1,2,3,4,4a,9,10,10a-octahydro-1,12,12-trimethyl-4a,10a-epoxy-1,4-methanophenanthren-2-ylester (A) und [1R-[1α,2β[2R*,3S*],4α,4aβ, 9β,10aβ]]-3-(Ethoxycarbonyl)amino-2-triisopropylsilyloxy-3-[4-(4-(1-methyl ethyliden)cyclohex-1-en-carbonyloxy)phenyl]-propansäure-9-methyl-1,2,3,4,4a,9, 10,10a-octahydro-1,12,12-trimethyl-4a,10a-epoxy-1,4-methanophenanthren-2-ylester (B)

In Analogie zu Beispiel 14 werden 10 mg (14,5 µmol) der nach Beispiel 20b dargestellten Verbindung unter Verwendung eines Gemisches aus R-(+)-Perillsäurechlorid und 4-(1-methylethyliden)cyclohex-1-en-carbonsäurechlorid umgesetzt Nach Aufarbeitung und Reinigung isoliert man 4,2 mg (5,0 µmol, 33%) der Titelverbindung A sowie 3,9 mg (4,6 µmol, 31%) der Titelverbindung B jeweils als farbloses Öl.

### BEISPIEL 20b

### [1R-[1α,2β,(2R*,3S*),4α,4aβ,9β,10aβ]]-3-(Ethoxycarbonyl)amino-2-triisopropylsilyloxy-3-(4-hydroxyphenyl)-propansäure-9-methyl-1,2,3,4,4a,9,10,10a-octahydro-1,12,12-trimethyl-4a,10a-epoxy-1,4-methanophenanthren-2-ylester

In Analogie zu Beispiel 9a werden 520 mg (750 µmol) der nach Beispiel 20c dargestellten Verbindung hydriert. Nach Aufarbeitung und Reinigung isoliert man 260 mg (376 µmol, 50%) der Titelverbindung als farbloses Öl.
¹H-NMR (CDCl₃): δ = 0,74 (3H), 0,81-1,31 (30H), 1,40 (3H), 1,52 (1H), 1,99-2,30 (3H), 2,59 (1H), 3,04 (1H), 4,09 (2H), 4,62 (1H), 4,87 (1H), 5,14 (1H), 5,19 (1H), 6,28 (1H), 6,67 (2H), 7,09-7,40 (5H), 7,45 (1H) ppm.

### BEISPIEL 20c

### [1R-[1α,2β(2R*,3S*),4α,4aβ,10aβ]]-3-(Ethoxycarbonyl)amino-2-triisopropylsilyloxy-3-(4-hydroxyphenyl)-propansäure-9-methylen-1,2,3,4,4a,9,10,10a-octahydro-1,12,12-trimethyl-4a,10a-epoxy-1,4-methanophenanthren-2-ylester

In Analogie zu Beispiel 1a werden 480 mg (1,7 mmol) [1R-(1α,2β,4α,4aβ,10aβ)]-9-methylen-1,2,3,4,4a,9,10,10a-octahydro-1,12,12-trimethyl-4a,10a-epoxy-1,4-methanophenanthren-2-ol unter Verwendung von (3R,4S)-1-(Ethoxycarbonyl)-3-hydroxy-4-[4-(tert-butyldimethylsilyloxy)phenyl]-2-azetidinon, das man in Analogie zu dem in Tetrahedron 1992 auf Seite 6985ff beschriebenen Verfahren hergestellt hat, umgesetzt. Nach Aufarbeitung und Reinigung isoliert man 531 mg (0,77 mmol, 45%) der Titelverbindung als farbloses Öl.
¹H-NMR (CDCl₃): δ = 0,82 (3H), 0,85-1,40 (30H), 1,54 (1H), 2,20 (1H), 2,63 (1H), 2,73 (1H), 3,10 (1H), 4,09 (2H), 4,63 (1H), 4,90 (1H), 5,02 (1H), 5,14 (1H), 5,49 (1H), 5,76 (1H), 6,37 (1H), 6,57 (2H), 7,16-7,35 (4H), 7,46 (1H), 7,52 (1H) ppm.

### BEISPIEL 21

### [1R-[1α,2β[2R*,3S*],4α,4aβ,9β,10aβ]]-3-(Ethoxycarbonyl)amino-2-hydroxy-3-[4-(4-(1-methylethyliden)cyclohex-1-en-carbonyloxy)phenyl]-propansäure-9-methyl-1,2,3,4,4a,9,10,10a-octahydro-1,12,12-trimethyl-4a,10a-epoxy-1,4-methanophenanthren-2-ylester

In Analogie zu Beispiel 1 werden 3,9 mg (4,6 µmol) der nach Beispiel 21a dargestellten Verbindung B umgesetzt. Nach Aufarbeitung und Reinigung isoliert man 1,9 mg (2,8 µmol, 59%) der Titelverbindung als farbloses Öl.
¹H-NMR (CDCl₃): δ = 0,78 (3H), 0,90 (3H), 1,03 (3H), 1,20 (3H), 1,40 (3H), 1,60 (3H), 1,63 (3H), 1,71 (1H), 1,82 (1H), 2,00-2,40 (6H), 2,52 (1H), 2,65 (1H), 2,68 (1H), 3,08 (1H), 3,20 (1H), 4,11 (2H), 4,53 (1H), 4,92 (1H), 5,28 (1H), 6,26 (1H), 7,04 (2H), 7,12-7,33 (4H), 7,43-7,59 (3H) ppm.

### BEISPIEL 22

### [1R-[1α,2β(2R*,3S*),4α,4aβ,9β,10aβ]]-3-(Ethoxycarbonyl)amino-2-hydroxy-3-(3-fluorphenyl)-propansäure-9-methyl-1,2,3,4,4a,9,10,10a-octahydro-1,12,12-trimethyl-4a,10a-epoxy-1,4-methanophenanthren-2-ylester

In Analogie zu Beispiel 1 werden 7,8 mg (11 µmol) der nach Beispiel 22a dargestellten Verbindung umgesetzt. Nach Aufarbeitung und Reinigung isoliert man 4,4 mg (8,2 µmol, 74%) der Titelverbindung als farbloses Öl.
¹H-NMR (CDCl₃): δ = 0,79 (3H), 0,90 (3H), 1,02 (3H), 1,19 (3H), 1,40 (3H), 1,71 (1H), 2,08 (1H), 2,20 (1H), 2,29 (1H), 2,68 (1H), 3,07 (1H), 3,20 (1H), 4,09 (2H), 4,52 (1H), 4,94 (1H), 5,28 (1H), 6,29 (1H), 6,94 (1H), 7,12-7,35 (6H), 7,49 (1H) ppm.

### BEISPIEL 22a

### [1R-[1α,2β(2R*,3S*),4α,4aβ,9β,10aβ]])-3-(Ethoxycarbonyl)amino-2-triisopropylsilyloxy-3-(3-fluorphenyl)-propansäure-9-methyl-1,2,3,4,4a,9,10,10a-octahydro-1,12,12-trimethyl-4a,10a-epoxy-1,4-methanophenanthren-2-ylester

In Analogie zu Beispiel 9a werden 14 mg (20 µmol) der nach Beispiel 25a dargestellten Verbindung hydriert. Nach Aufarbeitung und Reinigung isoliert man 7,8 mg (11,2 µmol, 56%) der Titelverbindung als farbloses Öl.

### BEISPIEL 23

### [1R-[1α,2β(2R*,3S*),4α,4aβ,9β,10aβ]]-3-(Ethoxycarbonyl)amino-2-hydroxy-3-(3-chlorphenyl)-propansäure-9-methyl-1,2,3,4,4a,9,10,10a-octahydro-1,12,12-trimethyl-4a,10a-epoxy-1,4-methanophenanthren-2-ylester

In Analogie zu Beispiel 1 werden 24 mg (34 µmol) der nach Beispiel 23a dargestellten Verbindung umgesetzt. Nach Aufarbeitung und Reinigung isoliert man 12,2 mg (22 µmol, 65%) der Titelverbindung als farbloses Öl.
¹H-NMR (CDCl₃): δ = 0,78 (3H), 0,90 (3H), 1,03 (3H), 1,20 (3H), 1,41(3H), 1,71 (2H), 2,09 (1H), 2,20 (1H), 2,29 (1H), 2,68 (1H), 3,07 (1H), 3,20 (1H), 4,09 (2H), 4,52 (1H), 4,92 (1H), 5,25 (1H), 6,32 (1H), 7,12-7,31 (5H), 7,38 (1H), 7,49 (1H), 7,57 (1H) ppm.

### BEISPIEL 23a

### [1R-[1α,2β(2R*,3S*),4α,4aβ,9β,10aβ]]-3-(Ethoxycarbonyl)amino-2-triisopropylsilyloxy-3-(3-chlorphenyl)-propansäure-9-methyl-1,2,3,4,4a,9,10,10a-octahydro-1,12,12-trimethyl-4a,10a-epoxy-1,4-methanophenanthren-2-ylester

In Analogie zu Beispiel 9a werden 38 mg (54 µmol) der nach Beispiel 26a dargestellten Verbindung hydriert Nach Aufarbeitung und Reinigung isoliert man 24 mg (34 µmol, 63%) der Titelverbindung als farbloses Öl.

### BEISPIEL 24

### [1R-[1α,2β(2R*,3S*),4α,4aβ,9β,10aβ]]-3-(Ethoxycarbonyl)amino-2-hydroxy-3-(4-methylphenyl)-propansäure-9-methyl-1,2,3,4,4a,9,10,10a-octahydro-1,12,12-trimethyl-4a,10a-epoxy-1,4-methanophenanthren-2-ylester

In Analogie zu Beispiel 1 werden 4 mg (5,8 µmol) der nach Beispiel 24a dargestellten Verbindung umgesetzt. Nach Aufarbeitung und Reinigung isoliert man 1,7 mg (3,2 µmol, 55%) der Titelverbindung als farbloses Öl.
¹H-NMR (CDCl₃): δ = 0,79 (3H), 0,89 (3H), 1,02 (3H), 1,19 (3H), 1,40 (3H), 1,78 (1H), 2,09 (1H), 2,22 (1H), 2,31 (4H), 2,68 (1H), 3,06 (1H), 3,12 (1H), 4,08 (2H), 4,52 (1H), 4,91 (1H), 5,22 (1H), 6,26 (1H), 7,05-7,32 (5H), 7,38 (2H), 7,51 (1H) ppm.

### BEISPIEL 24a

### [1R-[1α,2β(2R*3S*),4α,4aβ,9β,10aβ]]-3-(Ethoxycarbonyl)amino-2-triisopropylsilyloxy-3-(4-methylphenyl)-propansäure-9-methyl-1,2,3,4,4a,9,10,10a-octahydro-1,12,12-trimethyl-4a,10a-epoxy-1,4-methanophenanthren-2-ylester

In Analogie zu Beispiel 9a werden 42 mg (61 µmol) der nach Beispiel 27a dargestellten Verbindung hydriert. Nach Aufarbeitung und Reinigung isoliert man 4 mg (5,8 µmol, 10%) der Titelverbindung als farbloses Öl.

### BEISPIEL 25

### [1R-[1α,2β(2R*,3S*),4α,4aβ,10aβ]]-3-(Ethoxycarbonyl)amino-2-hydroxy-3-(3-fluorphenyl)-propansäure-9-methylen-1,2,3,4,4a,9,10,10a-octahydro-1,12,12-trimethyl-4a,10a-epoxy-1,4-methanophenanthren-2-ylester

In Analogie zu Beispiel 1 werden 7 mg (10 µmol) der nach Beispiel 25a dargestellten Verbindung umgesetzt. Nach Aufarbeitung und Reinigung isoliert man 3 mg (5,6 µmol, 56%) der Titelverbindung als farbloses Öl.
¹H-NMR (CDCl₃): δ = 0,85 (3H), 0,93 (3H), 1,07 (3H), 1,22 (3H), 1,72 (1H), 2,31 (1H), 2,72 (1H), 2,79 (1H), 3,12 (1H), 3,16 (1H), 4,11 (2H), 4,52 (1H), 4,94 (1H), 5,06 (1H), 5,27 (1H), 5,52 (1H), 6,39 (1H), 6,92 (1H), 7,15-7,40 (5H), 7,50 (1H), 7,56 (1H) ppm.

### BEISPIEL 25a

### [1R-[1α,2β(2R*,3S*),4α,4aβ,10aβ]]-3-(Ethoxycarbonyl)amino-2-triisopropylsilyl-oxy-3-(3-fluorphenyl)-propansäure-9-methylen-1,2,3,4,4a,9,10,10a-octahydro-1,12,12-trimethyl-4a,10a-epoxy-1,4-methanophenanthren-2-ylester

In Analogie zu Beispiel 1a werden 30 mg (106 µmol) [1R-(1α,2β,4α,4aβ,10aβ)]-9-methylen-1,2,3,4,4a,9,10,10a-octahydro-1,12,12-trimethyl-4a,10a-epoxy-1,4-methanophenanthren-2-ol unter Verwendung von (3R,4S)-1-(Ethoxycarbonyl)-3-triisopropylsilyl-oxy-4-(3-fluorphenyl)-2-azetidinon, das man in Analogie zu dem in Tetrahedron 1992 auf Seite 6985ff beschriebenen Verfahren hergestellt hat, umgesetzt. Nach Aufarbeitung und Reinigung isoliert man 49 mg (73 µmol, 69%) der Titelverbindung als farbloses Öl.

### BEISPIEL 26

### [1R-[1α,2β(2R*,3S*),4α,4aβ,10aβ]]-3-(Ethoxycarbonyl)amino-2-hydroxy-3-(3-chlorphenyl)-propansäure-9-methylen-1,2,3,4,4a,9,10,10a-octahydro-1,12,12-trimethyl-4a,10a-epoxy-1,4-methanophenanthren-2-ylester

In Analogie zu Beispiel 1 werden 20 mg (28 µmol) der nach Beispiel 26a dargestellten Verbindung umgesetzt. Nach Aufarbeitung und Reinigung isoliert man 12,4 mg (22 µmol, 80%) der Titelverbindung als farbloses Öl.
¹H-NMR (CDCl₃): δ = 0,87 (3H), 0,94 (3H), 1,09 (3H), 1,23 (3H), 1,74 (1H), 2,33 (1H), 2,73 (1H), 2,81 (1H), 3,13 (1H), 3,18 (1H), 4,12 (2H), 4,52 (1H), 4,93 (1H), 5,07 (1H), 5,26 (1H), 5,55 (1H), 6,44 (1H), 7,15-7,42 (5H), 7,45-7,64 (3H) ppm.

### BEISPIEL 26a

### [1R-[1α,2β(2R*,3S*),4α,4aβ,10aβ]]-3-(Ethoxycarbonyl)amino-2-triisopropylsilyloxy-3-(3-chlorphenyl)-propansäure-9-methylen-1,2,3,4,4a,9,10,10a-octahydro-1,12,12-trimethyl-4a,10a-epoxy-1,4-methanophenanthren-2-ylester

In Analogie zu Beispiel 1a werden 38 mg (54 µmol) [1R-(1α,2β,4α,4aβ,10aβ)]-9-methylen-1,2,3,4,4a,9,10,10a-octahydro-1,12,12-trimethyl-4a,10a-epoxy-1,4-methanophenanthren-2-ol unter Verwendung von (3R,4S)-1-(Ethoxycarbonyl)-3-triisopropylsilyloxy-4-(3-chlorphenyl)-2-azetidinon, das man in Analogie zu dem in Tetrahedron 1992 auf Seite 6985ff beschriebenen Verfahren hergestellt hat, umgesetzt. Nach Aufarbeitung und Reinigung isoliert man 24 mg (34 µmol, 63%) der Titelverbindung als farbloses Öl.

### BEISPIEL 27

### [1R-[1α,2β(2R*,3S*),4a,4aβ,10aβ]]-3-(Ethoxycarbonyl)amino-2-hydroxy-3-(4-methylphenyl)-propansäure-9-methylen-1,2,3,4,4a,9,10,10a-octahydro-1,12,12-trimethyl-4a,10a-epoxy-1,4-methanophenanthren-2-ylester

In Analogie zu Beispiel 1 werden 20 mg (29 µmol) der nach Beispiel 27a dargestellten Verbindung umgesetzt. Nach Aufarbeitung und Reinigung isoliert man 9 mg (17 µmol, 58%) der Titelverbindung als farbloses Öl.
¹H-NMR (CDCl₃): δ = 0,84 (3H), 0,92 (3H), 1,07 (3H), 1,21 (3H), 1,79 (1H), 2,27 (3H), 2,30 (1H), 2,71 (1H), 2,78 (1H), 3,10 (1H), 3,12 (1H), 4,11 (2H), 4,51 (1H), 4,93 (1H), 5,05 (1H), 5,21 (1H), 5,52 (1H), 6,33 (1H), 7,08 (2H), 7,22-7,41 (4H), 7,45-7,60 (2H) ppm.

### BEISPIEL 27a

### [1R-[1α,2β(2R*,3S*),4α,4aβ,10aβ]]-3-(Ethoxycarbonyl)amino-2-triisopropylsilyloxy-3-(4-methylphenyl)-propansäure-9-methylen-1,2,3,4,4a,9,10,10a-octahydro-1,12,12-trimethyl-4a,10a-epoxy-1,4-methanophenanthren-2-ylester

In Analogie zu Beispiel 1a werden 30 mg (106 µmol) [1R-(1α,2β,4α,4aβ,10aβ)]-9-methylen-1,2,3,4,4a,9,10,10a-octahydro-1,12,12-trimethyl-4a,10a-epoxy-1,4-methanophenanthren-2-ol unter Verwendung von (3R,4S)-1-(Ethoxycarbonyl)-3-triisopropylsilyloxy-4-(4-methylphenyl)-2-azetidinon, das man in Analogie zu dem in Tetrahedron 1992 auf Seite 6985ff beschriebenen Verfahren hergestellt hat, umgesetzt. Nach Aufarbeitung und Reinigung isoliert man 63 mg (92 µmol, 86%) der Titelverbindung als farbloses Öl.

### BEISPIEL 28

### [1R-[1α,2β(2R*,3S*),4α,4aβ,10aβ]]-3-(Ethoxycarbonyl)amino-2-hydroxy-3-(2-thienyl)-propansäure-9-methylen-1,2,3,4,4a,9,10,10a-octahydro-1,12,12-trimethyl-4a,10a-epoxy-1,4-methanophenanthren-2-ylester

In Analogie zu Beispiel 1 werden 5 mg (7,4 µmol) der nach Beispiel 28a dargestellten Verbindung umgesetzt. Nach Aufarbeitung und Reinigung isoliert man 2,7 mg (5,2 µmol, 69%) der Titelverbindung als farbloses Öl.
¹H-NMR (CDCl₃): δ = 0,83 (3H), 0,92 (3H), 1,08 (3H), 1,23 (3H), 1,58 (1H), 2,25 (1H), 2,68 (1H), 2,77 (1H), 3,12 (1H), 3,41 (1H), 4,12 (2H), 4,52 (1H), 4,94 (1H), 5,04 (1H), 5,49 (1H), 5,51 (1H), 6,49 (1H), 6,90 (1H), 7,12 (1H), 7,19 (1H), 7,21-7,37 (2H), 7,48 (1H), 7,55 (1H) ppm.

### BEISPIEL 28a

### [1R-[1α,2β(2R*,3S*),4α,4aβ,10aβ]]-3-(Ethoxycarbonyl)amino-2-triisopropylsilyloxy-3-(2-thienyl)-propansäure-9-methylen-1,2,3,4,4a,9,10,10a-octahydro-1,12,12-trimethyl-4a,10a-epoxy-1,4-methanophenanthren-2-ylester

In Analogie zu Beispiel 1a werden 7,4 mg (26 µmol) [1R-(1α,2β.4α,4aβ,10aβ)]-9-methylen-1,2,3,4,4a,9,10,10a-octahydro-1,12,12-trimethyl-4a,10a-epoxy-1,4-methanophenanthren-2-ol unter Verwendung von (3R,4S)-1-(Ethoxycarbonyl)-3-triisopropylsilyloxy-4-(2-thienyl)-2-azetidinon, das man in Analogie zu dem in Tetrahedron 1992 auf Seite 6985ff beschriebenen Verfahren hergestellt hat, umgesetzt. Nach Aufarbeitung und Reinigung isoliert man 16 mg (24 µmol, 90%) der Titelverbindung als farbloses Öl.

### BEISPIEL 29

### [1R-[1α,2β(2R*,3S*),4α,4aβ,9β,10aβ]]-3-(Ethoxycarbonyl)amino-2-hydroxy-3-(2-thienyl)-propansäure-9-methyl-1,2,3,4,4a,9,10,10a-octahydro-1,12,12-trimethyl-4a,10a-epoxy-1,4-methanophenanthren-2-ylester

In Analogie zu Beispiel 1 werden 5,5 mg (8,1 µmol) der nach Beispiel 29a dargestellten Verbindung umgesetzt. Nach Aufarbeitung und Reinigung isoliert man 3,4 mg (6,5 µmol, 81%) der Titelverbindung als farbloses Öl.
¹H-NMR (CDCl₃): δ = 0,77 (3H), 0,89 (3H), 1,01 (3H), 1,20 (3H), 1,41 (3H), 1,56 (1H), 2,07 (1H), 2,20 (1H), 2,22 (1H), 2,63 (1H), 3,07 (1H), 3,44 (1H), 4,12 (2H), 4,52 (1H), 4,94 (1H), 5,50 (1H), 6,40 (1H), 6,92 (1H), 7,09-7,34 (5H), 7,48 (1H) ppm.

### BEISPIEL 29a

### [1R-[1α,2β(2R*,3S*),4α,4aβ,9β,10aβ]]-3-(Ethoxycarbonyl)amino-2-triisopropylsilyloxy-3-(2-thienyl)-propansäure-9-methyl-1,2,3,4,4a,9,10,10a-octahydro-1,12,12-trimethyl-4a,10a-epoxy-1,4-methanophenanthren-2-ylester

In Analogie zu Beispiel 9a werden 80 mg (118 µmol) der nach Beispiel 28a dargestellten Verbindung hydriert. Nach Aufarbeitung und Reinigung isoliert man 63 mg (92 µmol, 78%) der Titelverbindung als farbloses Öl.

### BEISPIEL 30

### [1R-[1α,2β(2R*,3S*),4α,4aβ,10aβ]]-3-(Ethoxycarbonyl)amino-2-hydroxy-3-(3-pyridyl)-propansäure-9-methylen-1,2,3,4,4a,9,10,10a-octahydro-1,12,12-trimethyl-4a,10a-epoxy-1,4-methanophenanthren-2-ylester

In Analogie zu Beispiel 1 werden 9 mg (13 µmol) der nach Beispiel 30a dargestellten Verbindung umgesetzt. Nach Aufarbeitung und Reinigung isoliert man 6,1 mg (12 µmol, 88%) der Titelverbindung als farbloses Öl.
¹H-NMR (CDCl₃): δ = 0,85 (3H), 0,93 (3H), 1,08 (3H), 1,22 (3H), 1,68 (1H), 2,32 (1H), 2,72 (1H), 2,80 (1H), 3,13 (1H), 3,30 (1H), 4,12 (2H), 4,55 (1H), 4,92 (1H), 5,05 (1H), 5,31 (1H), 5,54 (1H), 6,50 (1H), 7,13-7,38 (3H), 7,51 (1H), 7,56 (1H), 7,83 (1H), 8,50 (1H), 8,72 (1H) ppm.

### BEISPIEL 30a

### [1R-[1α,2β(2R*,3S*),4α,4aβ,10aβ]]-3-(Ethoxycarbonyl)amino-2-triisopropylsilyloxy-3-(3-pyridyl)-propansäure-9-methylen-1,2,3,4,4a,9,10,10a-octahydro-1,12,12-trimethyl-4a,10a-epoxy-1,4-methanophenanthren-2-ylester

In Analogie zu Beispiel 1a werden 17 mg (60 µmol) [1R-(1α,2β,4α,4aβ,10aβ)]-9-methylen-1,2,3,4,4a,9,10,10a-octahydro-1,12,12-trimethyl-4a,10a-epoxy-1,4-methanophenanthren-2-ol unter Verwendung von (3R,4S)-1-(Ethoxycarbonyl)-3-triisopropylsilyloxy-4-(3-pyridyl)-2-azetidinon, das man in Analogie zu dem in Tetrahedron 1992 auf Seite 6985ff beschriebenen Verfahren hergestellt hat, umgesetzt. Nach Aufarbeitung und Reinigung isoliert man 28 mg (41 µmol, 68%) der Titelverbindung als farbloses Öl.

### BEISPIEL 31

### [1R-[1α,2β(2R*,3S*),4α,4aβ,9β,10aβ]]-3-(Ethoxycarbonyl)amino-2-hydroxy-3-(3-pyridyl)-propansäure-9-methyl-1,2,3,4,4a,9,10,10a-octahydro-1,12,12-trimethyl-4a,10a-epoxy-1,4-methanophenanthren-2-ylester (A) und [1R-[1α,2β(2R*,3S*),4α, 4aβ,9α,10aβ]]-3-(Ethoxycarbonyl)amino-2-hydroxy-3-(3-pyridyl)-propansäure-9-methyl-1,2,3,4,4a,9,10,10a-octahydro-1,12,12-trimethyl-4a,10a-epoxy-1,4-methanophenanthren-2-ylester (B)

In Analogie zu Beispiel 1 werden 9 mg (13 µmol) der nach Beispiel 31a dargestellten Verbindung umgesetzt. Nach Aufarbeitung und Reinigung isoliert man 3,5 mg (6,7 µmol, 51%) als 6:4-Gemisch der Titelverbindungen A und B als farbloses Öl.
¹H-NMR (CDCl₃): δ = 0,78 (1,2H), 0,79 (1,8H), 0,90 (3H), 1,03 (1,8H), 1,08 (1,2H), 1,21 (3H), 1,43 (3H), 1,68 (1H), 2,10 (0,6H), 2,12 (1H), 2,31 (1H), 2,48 (0,4H), 2,7 (0,6H), 2,76 (0,4H), 3,09 (1H), 3,27 (1H), 4,12 (2H), 4,56 (1H), 4,84 (0,4H), 4,92 (0,6H), 5,32 (1H), 6,37 (0,6H), 6,95 (0,4H), 7,12-7,40 (4H), 7,50 (1H), 7,86 (1H), 8,51 (1H), 8,76 (1H) ppm.

### BEISPIEL 31a

### [1R-[1α,2β(2R*,3S*),4α,4aβ,9ξ,10aβ]]-3-(Ethoxycarbonyl)amino-2-triisopropylsilyloxy-3-(3-pyridyl)-propansäure-9-methyl-1,2,3,4,4a,9,10,10a-octahydro-1,12,12-trimethyl-4a,10a-epoxy-1,4-methanophenanthren-2-ylester

In Analogie zu Beispiel 9a werden 19 mg (28 µmol) der nach Beispiel 30a dargestellten Verbindung hydriert. Nach Aufarbeitung und Reinigung isoliert man 9 mg (13 µmol, 47%) der Titelverbindung als farbloses Öl.

### BEISPIEL 32

### [1R-[1α,2β(2R*,3S*),4α,4aβ,10aβ]]-3-(Ethoxycarbonyl)amino-2-hydroxy-3-(4-pyridyl)-propansäure-9-methylen-1,2,3,4,4a,9,10,10a-octahydro-1,12,12-trimethyl-4a,10a-epoxy-1,4-methanophenanthren-2-ylester

In Analogie zu Beispiel 1 werden 31 mg (46 µmol) der nach Beispiel 32a dargestellten Verbindung umgesetzt. Nach Aufarbeitung und Reinigung isoliert man 20 mg (39 µmol, 83%) der Titelverbindung als farbloses Öl.
¹H-NMR (CDCl₃): δ = 0,84 (3H), 0,92 (3H), 1,08 (3H), 1,23 (3H), 1,58 (1H), 1,81 (1H), 2,29 (1H), 2,71 (1H), 2,80 (1H), 3,13 (1H), 4,12 (2H), 4,56 (1H), 4,93 (1H), 5,07 (1H), 5,29 (1H), 5,53 (1H), 6,43 (1H), 7,22-7,62 (6H), 8,50 (2H) ppm.

### BEISPIEL 32a

### [1R-[1α,2β(2R*,3S*),4α,4aβ,10aβ]]-3-(Ethoxycarbonyl)amino-2-triisopropylsilyloxy-3-(4-pyridyl)-propansäure-9-methylen-1,2,3,4,4a,9,10,10a-octahydro-1,12,12-trimethyl-4a,10a-epoxy-1,4-methanophenanthren-2-ylester

In Analogie zu Beispiel 1a werden 17 mg (60 µmol) [IR-(1α,2β,4α,4aβ,10aβ)]-9-methylen-1,2,3,4,4a,9,10,10a-octahydro-1,12,12-trimethyl-4a,10a-epoxy-1,4-methanophenanthren-2-ol unter Verwendung von (3R,4S)-1-(Ethoxycarbonyl)-3-triisopropylsilyloxy-4-(4-pyridyl)-2-azetidinon, das man in Analogie zu dem in Tetrahedron 1992 auf Seite 6985ff beschriebenen Verfahren hergestellt hat, umgesetzt. Nach Aufarbeitung und Reinigung isoliert man 31 mg (46 µmol, 76%) der Titelverbindung als farbloses Öl.

### BEISPIEL 33

### [1R-[1α,2β(2R*,3S*),4α,4aβ,9β,10aβ]]-3-(Ethoxycarbonyl)amino-2-hydroxy-3-(4-pyridyl)-propansäure-9-methyl-1,2,3,4,4a,9,10,10a-octahydro-1,12,12-trimethyl-4a,10a-epoxy-1,4-methanophenanthren-2-ylester

In Analogie zu Beispiel 1 werden 30 mg (44 µmol) der nach Beispiel 33a dargestellten Verbindung umgesetzt Nach Aufarbeitung und Reinigung isoliert man 19,6 mg (38 µmol, 85%) der Titelverbindung als farbloses Öl.
¹H-NMR (CDCl₃): δ = 0,78 (3H), 0,90 (3H), 1,02 (3H), 1,19 (3H), 1,41 (3H), 1,54 (1H), 2,02-2,5, (3H), 2,67 (1H), 3,08 (1H), 3.50 (breit, 1H), 4,10 (2H), 4,54 (1H), 4,92 (1H), 5,30 (1H), 6,32 (1H), 7,11-7,57 (6H), 8,52 (2H) ppm.

### BEISPIEL 33a

### [1R-[1α,2β(2R*,3S*),4α,4aβ,9β,10aβ]]-3-(Ethoxycarbonyl)amino-2-triisopropylsilyloxy-3-(4-pyridyl)-propansäure-9-methyl-1,2,3,4,4a,9,10,10a-octahydro-1,12,12-trimethyl-4a,10a-epoxy-1,4-methanophenanthren-2-ylester

In Analogie zu Beispiel 1a werden 20 mg (70 µmol) der nach Beispiel 33b dargestellten Verbindung A unter Verwendung von (3R,4S)-1-(Ethoxycarbonyl)-3-triisopropylsilyloxy-4-(4-pyridyl)-2-azetidinon, das man in Analogie zu dem in Tetrahedron 1992 auf Seite 6985ff beschriebenen Verfahren hergestellt hat, umgesetzt. Nach Aufarbeitung und Reinigung isoliert man 30 mg (44 µmol, 63%) der Titelverhindung als farbloses Öl.

### BEISPIEL 33b

### [1R-(1α,2β,4α,4aβ,9β,10aβ)]-9-methyl-1,2,3,4,4a,9,10,10a-octahydro-1,12,12-trimethyl-4a,10a-epoxy-1,4-methanophenanthren-2-ol (A) und [1R-(1α,2β,4α,4aβ, 9α,10aβ)]-9-methyl-1,2,3,4,4a,9,10,10a-octahydro-1,12,12-trimethyl-4a,10a-epoxy-1,4-methanophenanthren-2-ol (B):

### Variante A:

Die Lösung von 622 mg (0,94 mmol) [1R-[1α,2β(2R*,3S*),4α,4aβ,9β,10aβ]]-3-(Methoxycarbonyl)amino-2-(triisopropylsilyloxy)benzenpropansäure-9-methyl-1,2,3,4, 4a,9,10, 10a-octahydro-1,12,12-trimethyl-4a,10a-epoxy-1,4-methanophenanthren-2-ylester, den man in Analogie zu dem in DE .... (EM 51113) beschrieben Verfahren hergestellt hat, in 9 ml Tetrahydrofuran versetzt man bei 23°C mit 1,4 ml einer 1M Lösung von Tetrabutylammoniumfluorid in Tetrahydrofuran, 2,4 ml einer 5%igen wässrigen Lösung von Lithiumhydroxid und rührt 1,5 Stunden. Man gießt in eine gesättigte Natriumchloridlösung, extrahiert mehrfach mit Dichlormethan, wäscht die vereinigten organischen Extrakte mit gesättigter Natriumchloridlösung und trocknet über Natriumsulfat. Den nach Filtration und Lösungsmittelabzug erhaltenen Rückstand reinigt man durch Chromatographie an ca. 70 ml feinem Kieselgel. Als Laufmittel dient ein Gemisch aus n-Hexan und Ethylacetat. Isoliert werden 230 mg (0,81 mmol, 86%) der Titelverbindung A als farbloser Feststoff.

### Variante B:

Die Lösung von 500 mg (1,77 mmol) [1R-(1α,2β,4α,4aβ,10aβ)]-9-methylen-1,2,3,4,4a,9,10,10a-octahydro-1,12,12-trimethyl-4a,10-epoxy-1,4-methanophenanthren-2-ol, das man in Analogie zu dem in J. Am. Chem. Soc. 1992, auf Seite 5879ff beschriebenen Verfahren hergestellt hat, in 8 ml wasserfreiem Tetrahydrofuran versetzt man portionsweise mit insgesamt 100 mg Tris-triphenylphosphin-rhodium(I)-chlorid und hydriert unter einer Atmosphäre aus Wasserstoff unter kräftigem Schütteln. Nach erfolgter Wasserstoffaufnahme engt man ein und chromatographiert über ca. 250 ml feines Kieselgel mit einem Gemisch aus n-Hexan und Ethylacetat. Isoliert werden 226 mg (795 µmol, 45%) der Titelverbindung A, 31 mg (109 µmol, 6%) der Titelverbindung B jeweils als farbloser Feststoff sowie 205 mg (721 µmol, 41%) einer Mischfraktion aus A und B.

### BEISPIEL 34

### [1R-[1α,2β(2R*,3S*),4α,4aβ,10aβ]]-3-(Ethoxycarbonyl)amino-2-hydroxy-3-(3-furanyl)-propansäure-9-methylen-1,2,3,4,4a,9,10,10a-octahydro-1,12,12-trimethyl-4a,10a-epoxy-1,4-methanophenanthren-2-ylester

In Analogie zu Beispiel 1 werden 37 mg (56 µmol) der nach Beispiel 34a dargestellten Verbindung umgesetzt. Nach Aufarbeitung und Reinigung isoliert man 19 mg (37 µmol, 67%) der Titelverbindung als farbloses Öl.
¹H-NMR (CDCl₃): δ = 0,83 (3H), 0,92 (3H), 1,08 (3H), 1,24 (3H), 1,51 (1H), 2,28 (1H), 2,68 (1H), 2,77 (1H), 3,14 (1H), 3,28 (1H), 4,16 (2H), 4,45 (1H), 4,88 (1H), 5,03 (1H), 5,21 (1H), 5,52 (1H), 6,29 (1H), 6,52 (1H), 7,22-7,37 (3H), 7,47 (2H), 7,54 (1H) ppm.

### BEISPEL 34a

### [1R-[1α,2β(2R*,3S*),4α,4aβ,10aβ]]-3-(Ethoxycarbonyl)amino-2-triisopropyisilyloxy-3-(3-furanyl)-propansäure-9-methylen-1,2,3,4,4a,9,10,10a-octahydro-1,12,12-trimethyl-4a,10a-epoxy-1,4-methanophenanthren-2-ylester

In Analogie zu Beispiel 1a werden 17 mg (60 µmol) [1R-(1α,2β,4α,4aβ,10aβ)]-9-methylen-1,2,3,4,4a,9,10,10a-octahydro-1,12,12-trimethyl-4a,10a-epoxy-1,4-methanophenanthren-2-ol unter Verwendung von (3R,4S)-1-(Ethoxycarbonyl)-3-triisopropylsilyloxy-4-(3-furanyl)-2-azetidinon, das man in Analogie zu dem in Tetrahedron 1992 auf Seite 6985ff beschriebenen Verfahren hergestellt hat, umgesetzt. Nach Aufarbeitung und Reinigung isoliert man 37 mg (56 µmol, 93%) der Titelverbindung als farbloses Öl.

### BEISPIEL 35

### [1R-[1α,2β(2R*,3S*),4α,4aβ,9β,10aβ]]-3-(Ethoxycarbonyl)amino-2-hydroxy-3-(3-furanyl)-propansäure-9-methyl-1,2,3,4,4a,9,10,10a-octahydro-1,12,12-trimethyl-4a,10a-epoxy-1,4-methanophenanthren-2-ylester

In Analogie zu Beispiel 9a werden 15 mg (30 µmol) der nach Beispiel 34 dargestellten Verbindung hydriert. Nach Aufarbeitung und Reinigung isoliert man 9 mg (18 µmol, 58%) der Titelverbindung als farbloses Öl.
¹H-NMR (CDCl₃): δ = 0,77 (3H), 0,89 (3H), 1,03 (3H), 1,23 (3H), 1,39 (3H), 1,50 (1H), 2,08 (1H), 2,27 (2H), 2,64 (1H), 3,08 (1H), 3,30 (1H), 4,17 (2H), 4,46 (1H), 4,85 (1H), 5,21 (1H), 6,17 (1H), 6,57 (1H), 7,11-7,35 (4H), 7,48 (1H), 7,52 (1H) ppm.

### BEISPIEL 36

### [1R-[1α,2β(2R*,3S*),4α,4aβ,10aβ]]-3-[(1-Oxobutyl)amino]-2-hydroxy-3-(3-thienyl)-propansäure-9-methylen-1,2,3,4,4a,9,10,10a-octahydro-1,12,12-trimethyl-4a,10a-epoxy-1,4-methanophenanthren-2-ylester

In Analogie zu Beispiel 1 werden 34 mg (50 µmol) der nach Beispiel 36a dargestellten Verbindung umgesetzt. Nach Aufarbeitung und Reinigung isoliert man 20 mg (38 µmol, 77%) der Titelverbindung als farbloses Öl.
¹H-NMR (CDCl₃): δ = 0,79 (3H), 0,84 (3H), 0,91 (3H), 1,07 (3H), 1,44 (1H), 1,59 (2H), 2,19 (2H), 2,25 (1H), 2,71 (1H), 2,84 (1H), 3,08 (1H), 3,26 (1H), 4,43 (1H), 4,98 (1H), 5,02 (1H), 5,53 (1H), 5,67 (1H), 7,15 (1H), 7,23 (2H), 7,36 (2H), 7,477,64 (3H) ppm.

### BEISPIEL 36a

### [1R-[1α,2β(2R*,3S*),4α,4aβ,10aβ]]-3-[(1-Oxobutyl)amino]-2-triispropylsilyloxy-3-(3-thienyl)-propansäure-9-methylen-1,2,3,4,4a,9,10,10a-octahydro-1,12,12-trimethyl-4a,10a-epoxy-1,4-methanophenanthren-2-ylester

In Analogie zu Beispiel 1a werden 17 mg (60 µmol) [1R-(1α,2β,4α,4aβ,10aβ)]-9-methylen-1,2,3,4,4a,9,10,10a-octahydro-1,12,12-trimethy-4a,10a-epoxy-1,4-methanophenanthren-2-ol unter Verwendung von (3R,4S)-1-(1-Oxobutyl)-3-triisopropylsilyloxy-4-(3-thienyl)-2-azetidinon, das man in Analogie zu dem in Tetrahedron 1992 auf Seite 6985ff beschriebenen Verfahren hergestellt hat, umgesetzt. Nach Aufarbeitung und Reinigung isoliert man 34 mg (50 µmol, 84%) der Titelverbindung als farbloses Öl.

### BEISPIEL 37

### [1R-[1α,2β(2R*,3S*),4α,4aβ,10aβ]]-3-[(3-Phenyl-1-oxopropyl)amino]-2-hydroxy-3-(3-thienyl)-propansäure-9-methylen-1,2,3,4,4a,9,10,10a-octahydro-1,12,12-trimethyl-4a,10a-epoxy-1,4-methanophenanthren-2-ylester

In Analogie zu Beispiel 1 werden 32 mg (43 µmol) der nach Beispiel 37a dargestellten Verbindung umgesetzt. Nach Aufarbeitung und Reinigung isoliert man 22 mg (38 µmol, 88%) der Titelverbindung als farbloses Öl.
¹H-NMR (CDCl₃): δ = 0,84 (3H), 0,89 (3H), 1,04 (3H), 1,24 (1H), 2,15 (1H), 2,44 (1H), 2,60 (1H), 2,68 (1H), 2,78 (1H), 2,83 (2H), 3,02 (1H), 3,22 (1H), 4,37 (1H), 4,83 (1H), 4,94 (1H), 5,30 (1H), 5,59 (1H), 6,59 (1H), 6,84 (2H), 6,93 (1H), 6,98 (2H), 7,11 (2H), 7,38 (2H), 7,47-7,62 (3H) ppm.

### BEISPIEL 37a

### [1R-[1α,2β(2R*,3S*),4α,4aβ,10aβ]]-3-[(3-Phenyl-1-oxopropyl)amino]-2-triisopropylsilyloxy-3-(3-thienyl)-propansäure-9-methylen-1,2,3,4,4a,9,10,10a-octahydro-1,12,12-trimethyl-4a,10a-epoxy-1,4-methanophenanthren-2-ylester

In Analogie zu Beispiel 1a werden 17 mg (60 µmol) [1R-(1α,2β,4α,4aβ,10aβ)]-9-methylen-1,2,3,4,4a,9,10,10a-octahydro-1,12,12-trimethyl-4a,10a-epoxy-1,4-methanophenanthren-2-ol unter Verwendung von (3R,4S)-1-(3-Pheny-1-oxopropyl)-3-triisopropylsilyloxy-4-(3-thienyl)-2-azetidinon, das man in Analogie zu dem in Tetrahedron 1992 auf Seite 6985ff beschriebenen Verfahren hergestellt hat, umgesetzt. Nach Aufarbeitung und Reinigung isoliert man 32 mg (43 µmol, 72%) der Titelverbindung als farbloses Öl.

### BEISPIEL 38

### [1R-[1α,2β(2R*,3S*),4α,4aβ,9β,10aβ]]-3-[(3-Phenyl-1-oxopropyl)amino]-2-hydroxy-3-(3-thienyl)-propansäure-9-methyl-1,2,3,4,4a,9,10,10a-octahydrodro-1,12,12-trimethyl-4a,10a-epoxy-1,4-methanophenanthren-2-ylester

In Analogie zu Beispiel 9a werden 17 mg (29 µmol) der nach Beispiel 37 dargestellten Verbindung hydriert Nach Aufarbeitung und Reinigung isoliert man 1,6 mg (3 µmol, 9%) der Titelverbindung als farbloses Öl.
¹H-NMR (CDCl₃): δ = 0,78 (3H), 0,89 (3H), 0,98 (3H), 1,29 (3H), 1,53 (1H), 2,07 (2H), 2,21 (1H), 2,44-2,72 (3H), 2,91 (2H), 3,08 (1H), 3,36 (1H), 4,43 (1H), 5,06 (1H), 5,52 (1H), 6,89 (1H), 6,93-7,06 (3H), 7,10-7,37 (8H), 7,53 (1H) ppm.

### BEISPIEL 39

### [1R-[1α,2β(2R*,3S*),4α,4aβ,9β,10aβ]]-3-[(1-Oxobutyl)amino]-2-hydroxy-3-(3-thienyl)-propansäure-9-methyl-1,2,3,4,4a,9,10,10a-octahydro-1,12,12-trimethyl-4a,10a-epoxy-1,4-methanophenanthren-2-ylester

In Analogie zu Beispiel 9a werden 17 mg (33 µmol) der nach Beispiel 36 dargestellten Verbindung hydriert. Nach Aufarbeitung und Reinigung isoliert man 1,4 mg (2,7 µmol, 8%) der Titelverbindung als farbloses Öl.
¹H-NMR (CDCl₃): δ = 0,78 (3H), 0,90 (3H), 1,00 (3H), 1,25 (3H), 1,36 (3H), 1,51-1,72 (3H), 2,11 (2H), 2,23 (2H), 2,28 (1H), 2,66 (1H), 3,09 (1H), 3,51 (1H), 4,51 (1H), 5,02 (1H), 5,60 (1H), 6,99 (1H), 7,10-7,34 (6H), 7,49 (1H) ppm.

### BEISPIEL 40

### [1R-[1α,2β[2R*,3S*(2R*,3S*)],4α,4aβ,10aβ]]-3-[[3-(Ethoxycarbonyl)amino]-2-hydroxy-3-(3-thienyl)-1-oxopropyl]amino]-2-hydroxy-3-(3-thienyl)-propansäure-9-methylen-1,2,3,4,4a,9,10,10a-octahydro-1,12,12-trimethyl-4a,10a-epoxy-1,4-methanophenanthren-2-ylester

In Analogie zu Beispiel 1 werden 19 mg (19 µmol) bzw. 14 mg (16 µmol) der nach Beispiel 40a dargestellten Verbindungen umgesetzt. Nach Aufarbeitung und Reinigung isoliert man 12,2 mg (17,6 µmol, 50%) der Titelverbindung als farbloses Öl.
¹H-NMR (CDCl₃): δ = 0,83 (3H), 0,92 (3H), 1,05 (3H), 1,19 (3H), 1,61 (1H), 2,23 (1H), 2,71 (1H), 2,78 (1H), 3,12 (1H), 3,29 (1H), 3,50 (1H), 4,03 (2H), 4,32 (2H), 5,02 (1H), 5,14 (2H), 5,33 (1H), 5,61(1H), 5,70 (1H), 6,71 (1H), 6,74 (1H), 6,98 (1H), 7,06 (2H), 7,22 (1H), 7,38 (2H), 7,52 (2H), 7,93 (1H) ppm.

### BEISPIEL 40a

### [1R-[1α,2β[2R*,3S*(2R*,3S*)],4α,4aβ,10aβ]]-3-[[3-(Ethoxycarbonyl)amino]-2-triisopropylsilyloxy-3-(3-thienyl-1-oxopropyl]amino]-2-triisopropysilyloxy-3-(3-thienyl)-propansäure-9-methylen-1,2,3,4,4a,9,10,10a-octahydro-1,12,12-trimethyl-4a,10a-epoxy-1,4-methanophenanthren-2-ylester

Die Lösung von 25 mg (41 µmol) der nach Beispiel 40b dargestellten Verbindung in 0,5 ml wasserfreiem Dichlormethan versetzt man unter einer Atmosphäre aus trockenem Argon mit 51 mg der nach Beispiel 40d dargestellten Säure, 17 mg 1-Hydroxy-1H-benzotriazol, 56 µl N-Ethyldiisopropylamin, 39 mg O-(Benzotriazol-1-yl)-N,N,N',N'-tetramethyluroniumtetrafluoroborat und rührt 1 Stunde bei 23°C. Man gießt in eine gesättigte Natriumhydrogencarbonatlösung, extrahiert mit Dichlormethan und trocknet die vereinigten organischen Extrakte über Natriumsulfat. Den nach Filtration und Lösungsmittelabzug erhaltenen Rückstand reinigt man durch Chromatographie an zwei analytischen Kieselgelplatten. Als Laufmittel dient ein Gemisch aus n-Hexan und Ethylacetat, als Elutionsmittel ein Gemisch aus Dischlormethan und Methanol. Isoliert werden 19 mg (19 µmol, 46%) der Titelverbindung sowie 14 mg (16 µmol, 39%) einer mono-desilylierten Verbindung jeweils als farbloser Schaum.

### BEISPIEL 40b

### [1R-[1α,2β(2R*,3S*),4α,4aβ,10aβ]]-3-Amino-2-triisopropylsilyoxy-3-(3-thienyl)-propansäure-9-methylen-1,2,3,4,4a,9,10,10a-octahydro-1,12,12-trimethyl-4a,10a-epoxy-1,4-methanophenanthren-2-ylester

Die Lösung von 55 mg (79 µmol) der nach Beispiel 40c dargestellten Verbindung in 0,8 ml wasserfreiem Tetrahydrofuran versetzt man unter einer Atmosphäre aus trockenem Argon mit 16 µl n-Butylamin, 9,2 mg Tetrakis-triphenylphosphin-palladium (0), 4,2 mg Triphenylphosphin und rührt 2 Stunden bei 23°C. Man gießt in eine gesättigte Natriumhydrogencarbonatlösung, extrahiert mit Ethylacetat und trocknet die vereinigten organischen Extrakte über Natriumsulfat. Den nach Filtration und Lösungsmittelabzung erhaltenen Rückstand reinigt man durch Chromatographie an drei analytischen Kieselgelplatten. Als Laufmittel dient ein Gemisch aus n-Hexan und Ethylacetat, als Elutionsmittel ein Gemisch aus Dischlormethan und Methanol. Isoliert werden 30 mg (49 µmol, 62%) der Titelverbindung als farbloser Schaum.

### BEISPIEL 40c

### [1R-[1α,2β(2R*,3S*),4α,4aβ,10aβ]]-3-(Allyloxycarbonyl)amino-2-triisopropylsilyloxy-3-(3-thienyl)-propansäure-9-methylen-1,2,3,4,4a,9,10,10a-octahydro-1,12,12-trimethyl-4a,10a-epoxy-1,4-methanophenanthren-2-ylester

In Analogie zu Beispiel 1a werden 30 mg (106 µmol) [1R-(1α,2β,4α,4aβ,10aβ)]-9-methylen-1,2,3,4,4a,9,10,10a-octahydro-1,12,12-trimethyl-4a,10a-epoxy-1,4-methanophenanthren-2-ol unter Verwendung von (3R,4S)-1-Allyloxycarbonyl-3-triisopropylsilyloxy-4-(3-thienyl)-2-azetidinon, das man in Analogie zu dem in Tetrahedron 1992 auf Seite 6985ff beschriebenen Verfahren hergestellt hat, umgesetzt. Nach Aufarbeitung und Reinigung isoliert man 61 mg (88 µmol, 83%) der Titelverbindung als farbloses Öl.

### BEISPIEL 40d

### (2R,3S)-2-Triisopropylsilyloxy-3-(ethoxycarbonyl)amino-3-(3-thienyl)-propansäure

Die Losung von 160 mg (372 µmol) der nach Beispiel 40e dargestellten Verbindung in 6 ml Methanol versetzt man mit 2 ml einer 5%igen wässrigen Lithiumhydroxidlösung und rührt 20 Stunden bei 23°C. Man verdünnt mit Wasser, stellt durch Zugabe einer 4N Salzsäure den pH-Wert auf 6-7 ein und extrahiert mit Dichlormethan. Die vereinigten organischen Extrakte trocknet man über Natriumsulfat und reinigt den nach Filtration und Lösungsmittelabzug erhaltenen Rückstand durch Chromatographie an feinem Kieselgel mit einem Gradientensystem aus n-Hexan, Ethylacetat und Methanol, Isoliert werden 66 mg (159 µmol, 43%) der Titelverbihdung als farbloser Schaum.

### BEISPIEL 40e

### (2R,3S)-2-Triisopropylsilyloxy-3-(ethoxycarbonyl)amino-3-(3-thienyl)-propansäuremethylester

Die Lösung von 160 mg (402 µmol) (3R,4S)-1-Ethoxycarbonyl-3-triisopropylsilyloxy-4-(3-thienyl)-2-azetidinon, das man in Analogie zu dem in Tetrahedron 1992 auf Seite 6985ff beschriebenen Verfahren hergestellt hat, in 4 ml Methanol versetzt man mit 26 mg Natriummethanolat und rührt 3 Stunden unter einer Atmosphäre aus trockenem Argon bei 23°C. Man gießt in eine gesättigte Ammoniumchloridlösung, extrahiert mit Dichlormethan und trocknet die vereinigten organischen Extrakte über Natriumsulfat. Nach Filtration und Lösungsmittelabzug isoliert man 170 mg (396 µmol, 98%) der Titelverbindung als farblosen Schaum den man ohne Reinigung weiter umsetzt.

### BEISPIEL 41

### [1R-[1α,2β[2R*,3S*(2R*,3S*)],4α,4aβ,10aβ]]-3-[[3-(Ethoxycarbonyl)amino]-2-hydroxy-3-phenyl-1-oxopropyl]amino]-2-hydroxy-3-(3-thienyl)-propansäure-9-methylen-1,2,3,4,4a,9,10,10a-octahydro-1,12,12-trimethyl-4a,10a-epoxy-1,4-methanophenanthren-2-ylester

In Analogie zu Beispiel 1 werden 6,0 mg (6,0 µmol) der nach Beispiel 41a dargestellten Verbindung umgesetzt. Nach Aufarbeitung und Reinigung isoliert man 4,0 mg (5,8 µmol, 97%) der Titelverbindung als farbloses Öl.
¹H-NMR (CDCl₃): δ = 0,83 (3H), 0,91 (3H), 1,03 (3H), 1,21 (3H), 1,51 (1H), 2,20 (1H), 2,72 (1H), 2,79 (1H), 3,15 (1H), 3,19 (1H), 4,05 (2H), 4,31 (2H), 5,02 (1H), 5,08 (1H), 5,10 (1H), 5,43 (1H), 5,58 (1H), 5,72 (1H), 6,74 (1H), 6,87-7,03 (5H), 7,12 (1H), 7,18 (1H), 7,40 (2H), 7,58 (2H), 7,82 (1H) ppm.

### BEISPIEL 41a

### [1R-[1α,2β[2R*,3S*(2R*,3S*)],4α,4aβ,10aβ]]-3-[[3-(Ethoxycarbonyl)amino]-2-triisopropylsilyloxy-3-phenyl-1-oxopropyl]amino]-2-triisopropylsilyloxy-3-(3-thienyl)-propansäure-9-methylen-1,2,3,4,4a,9,10,10a-octahydro-1,12,12-trimethyl-4a,10a-epoxy-1,4-methanophenanthren-2-ylester

In Analogie zu Beispiel 40a werden 10,0 mg (16,4 µmol) der nach Beispiel 40b dargestellten Verbindung unter Verwendung der nach Beispiel 41b dargestellten Säure umgesetzt. Nach Aufarbeitung und Reinigung isoliert man 4,0 mg (4,0 µmol, 24%) der Titelverbindung als farbloses Öl.

### BEISPIEL 41b

### (2R,3S)-2-Triisopropylsilyloxy-3-(ethoxycarbonyl)amino)-3-pnenyl-propansäure

In Analogie zu Beispiel 40d werden 560 mg (1,3 mmol) der nach Beispiel 41d dargestellten Verbindung umgesetzt. Nach Aufarbeitung und Reinigung isoliert man 470 mg (1,15 mmol, 88%) der Titelverbindung als farbloses Öl.

### BEISPIEL 41c

### (2R,3S)-2-Triisopropylsilyloxy)-3-(ethoxycarbonyl)amino-3-phenylpropansäuremethylester

In Analogie zu Beispiel 40e werden 1,1 g (2,8 mmol) (3R,4S)-1-Ethoxycarbonyl-3-triisopropylsilyloxy-4-phenyl-2-azetidinon, das man in Analogie zu dem in Tetrahedron 1992 auf Seite 6985ff beschriebenen Verfahren hergestellt hat, umgesetzt. Nach Aufarbeitung und Reinigung isoliert man 1,16 g (2,74 mmol. 98%) der Titelverbindung als farbloses Öl.

### BEISPIEL 42

### [1R-[1α,2β[2R*,3S*(2R*,3S*)],4α,4aβ,9β,10aβ]]-3-[[3-(Ethoxycarbonyl)amino]-2-hydroxy-3-(-4-pyridyl)-1-oxopropyl]amino]-2-hydroxy-3-(4-pyridyl)propansäure-9-methyl-1,2,3,4,4a,9,10,10a-octahydro-1,12,12-trimethyl-4a,10a-epoxy-1,4-methanophenanthren-2-ylester

In Analogie zu Beispiel 1 werden 3,4 mg (3,4 µmol) der nach Beispiel 42a dargestellten Verbindung umgesetzt. Nach Aufarbeitung und Reinigung isoliert man 0,8 mg (1,2 µmol, 35%) der Titelverbindung als farbloses Öl.
¹H-NMR (CDCl₃): δ = 0,79 (3H), 0,93 (3H), 1,00 (3H), 1,17 (3H), 1,31 (3H), 2,00-2,15 (3H), 2,40 (1H), 2,77 (1H), 3,11 (1H), 3,55 (breit, 2H), 3,94-4,18 (2H), 4,49 (1H), 4,58 (1H), 5,20 (1H), 5,28 (1H), 5,51 (1H), 5,60 (1H), 6,98 (2H), 7,15-7,40 (5H), 7,46 (1H), 8,00-8,13 (3H), 8,53 (2H) ppm.

### BEISPIEL 42a

### [1R-[1α,2β[2R*,3S*(2R*,3S*)],4α,4aβ,9β,10aβ]]-3-[[3-(Ethoxycarbonyl)amino]-2-triisopropylsilyloxy-3-(4-pyridyl)-1-oxopropyl]amino]-2-triisopropysilyloxy-3-(4-pyridyl)-propansäure-9-methyl-1,2,3,4,4a,9,10,10a-octahydro-1,12,12-trimethyl-4a,10a-epoxy-1,4-methanophenantren-2-ylester

In Analogie zu Beispiel 40a werden 8,0 mg (13,2 µmol) der nach Beispiel 42b dargestellten Verbindung unter Verwendung der nach Beispiel 42d dargestellten Säure umgesetzt. Nach Aufarbeitung und Reinigung isoliert man 3,4 mg (3,4 µmol, 26%) der Titelverbindung als farbloses Öl.

### BEISPIEL 42b

### [1R-[1α,2β[2R*,3S*(2R*,3S*)],4α,4aβ,9β,10aβ]]-2-Triisopropylsilyloxy-3-amino-3-(4-pyridyl)-propansäure-9-methyl-1,2,3,4,4a,9,10,10a-octahydro 1,12,12-trimethyl-4a,10a-epoxy-1,4-methanophenanthren-2-ylester

In Analogie zu Beispiel 40b werden 31 mg (45 µmol) der nach Beispiel 42c dargestellten Verbindung umgesetzt. Nach Aufarbeitung und Reinigung isoliert man 20 mg (33 µmol, 74%) der Titelverbindung als farbloses Öl.

### BEISPIEL 42c

### [1R-[1α,2β(2R*,3S*),4α,4aβ,9β,10aβ]]-3-(Allyloxycarbonyl)amino-2-triisopropylsilyloxy-3-(4-pyridyl)-propansäure-9-methyl-1,2,3,4,4a,9,10,10a-octahydro-1,12,12-trimethyl-4a,10a-epoxy-1,4-methanophenanthren-2-ylester

In Analogie zu Beispiel 1a werden 20 mg (70 µmol) der nach Beispiel 33a dargestellten Verbindung unter Verwendung von (3R,4S)-1-Allyloxycarbonyl-3-triisopropylsilyl-oxy-4-(4-pyridyl)-2-azetidinon, das man in Analogie zu dem in Tetrahedron 1992 auf Seite 6985ff beschriebenen Verfahren hergestellt hat, umgesetzt. Nach Aufarbeitung und Reinigung isoliert man 31 mg (45 µmol, 65%) der Titelverbindung als farbloses Öl.

### BEISPIEL 42d

### (2R,3S)-2-Triisopropylsilyloxy)-3-(ethoxycarbonyl)amino-3-(4-pyridyl)-propansäure

In Analogie zu Beispiel 40d werden 220 mg (520 µmol) der nach Beispiel 42e dargestellten Verbindung umgesetzt. Nach Aufarbeitung und Reinigung isoliert man 52 mg (127 µmol, 25%) der Titelverbindung als farbloses Öl.

### BEISPIEL 42e

### (2R,3S)-2-Triisopropylsilyloxy-3-(ethoxycarbonyl)amino-3-(4-pyridyl)-propansäuremethylester

In Analogie zu Beispiel 40e werden 340 mg (0,87 mmol) (3R,4S)-1-Ethoxycarbonyl-3-triisopropylsilyloxy-4-(4-pyridyl)-2-azetidinon, das man in Analogie zu dem in Tetrahedron 1992 auf Seite 6985ff beschriebenen Verfahren hergestellt hat, umgesetzt. Nach Aufarbeitung und Reinigung isoliert man 350 mg (0,82 mmol, 95%) der Titelverbindung als farbloses Öl.

### BEISPIEL 43

### [1R-[1α,2β(2R*,3S*),4α,4aβ,9α,10aβ]]-3-(Ethoxycarbonyl)amino-2-hydroxy-3-(4-pyridyl)-propansäure-9-methyl-1,2,3,4,4a,9,10,10a-octahydro-1,12,12-trimethyl-4a,10a-epoxy-1,4-methanophenanthren-2-ylester

In Analogie zu Beispiel 1 werden 21 mg (31 µmol) der nach Beispiel 43a dargestellten Verbindung umgesetzt. Nach Aufarbeitung und Reinigung isoliert man 9,6 mg (18 µmol, 60%) der Titelverbindung als farbloses Öl.
¹H-NMR (CDCl₃): δ = 0,76 (3H), 0,89 (3H), 1,06 (3H), 1,22 (3H), 1,43 (3H), 1,48-1,66 (2H), 2,27 (1H), 2,46 (1H), 2,75 (1H), 3,14 (1H), 4,12 (2H), 4,56 (1H), 4,85 (1H), 5,31 (1H), 6,91 (1H), 7,22-7,61 (6H), 8,51 (2H) ppm.

### BEISPIEL 43a

### [1R-[1α,2β(2R*3S*),4α,4aβ,9α,10aβ]]-3-(Ethoxycarbonyl)amino-2-triisopropylsilyloxy-3-(4-pyridyl)-propansäure-9-methyl-1,2,3,4,4a,9,10,10a-octahydro-1,12,12-trimethyl-4a,10a-epoxy-1,4-methanophenanthren-2-ylester

In Analogie zu Beispiel 1a werden 20 mg (70 µmol) der nach Beispiel 33b dargestellten Verbindung B unter Verwendung von (3R,4S)-1-(Ethoxycarbonyl)-3-triisopropylsilyloxy-4-(4-pyridyl)-2-azetidinon, das man in Analogie zu dem in Tetrahedron 1992 auf Seite 6985ff beschriebenen Verfahren hergestellt hat, umgesetzt. Nach Aufarbeitung und Reinigung isoliert man 34 mg (50 µmol, 72%) der Titelverbindung als farbloses Öl.

### BEISPIEL 44

### [1R-[1α,2β(2R*,3S*),4α,4aβ,9α,10aβ]]-3-(Ethoxycarbonyl)amino-2-hydroxy-3-(3-thienyl)-propansäure-9-methyl-1,2,3,4,4a,9,10,10a-octahydro-1,12,12-trimethyl-4a,10a-epoxy-1,4-methanophenanthren-2-ylester

In Analogie zu Beispiel 1 werden 8,2 mg (12 µmol) der nach Beispiel 44a dargestellten Verbindung umgesetzt. Nach Aufarbeitung und Reinigung isoliert man 2,8 mg (5,3 µmol, 44%) der Titelverbindung als farbloses Öl.
¹H-NMR (CDCl₃): δ = 0,75 (3H), 0,87 (3H), 1,05 (3H), 1,23 (3H), 1,42 (3H), 1,45 (1H), 1,55 (1H), 2,24 (1H), 2,46 (1H), 2,71 (1H), 3,07-3,24 (2H), 4,13 (2H), 4,48 (1H), 4,82 (1H), 5,37 (1H), 6,89 (1H), 7,15-7,42 (6H), 7,50 (1H) ppm.

### BEISPIEL 44a

### [1R-[1α,2β(2R*,3S*),4α,4aβ,9α,10αβ]]-3-(Ethoxycarbonyl)amino-2-triisopropylsilyloxy-3-(3-thienyl)-propansäure-9-methyl-1,2,3,4,4a,9,10,10a-octahydro-1,12,12-trimethyl-4a,10a-epoxy-1,4-methanophenanthren-2-ylester

In Analogie zu Beispiel 1a werden 6,4 mg (22 µmol) der nah Beispiel 43b dargestellten Verbindung A unter Verwendung von (3R,4S)-1-(Ethoxycarbonyl)-3-triisopropylsilyloxy-4-(3-thienyl)-2-azetidinon, das man in Analogie zu dem in Tetrahedron 1992 auf Seite 6985ff beschriebenen Verfahren hergestellt hat, umgesetzt. Nach Aufarbeitung und Reinigung isoliert man 8,2 mg (12 µmol, 54%) der Titelverbindung als farblose, Öl.

### BEISPIEL 45

### [1R-[1α,2β(2R*,3S*),4α,4aβ,9β,10aβ]]-3-(Ethoxycarbonyl)amino-2-hydroxy-3-(1-methyl-4-pyridino)-propansäure-9-methyl-1,2,3,4,4a,9,10,10a-octahydro-1,12,12-trimethyl-4a,10a-epoxy-1,4-methanophenanthren-2-ylester-iodid

Die Lösung von 3,7 mg (7,1 µmol) der nach Beispiel 33 dargestellten Verbindung in 0,1 ml Dichlormethan versetzt man unter einer Atmosphäre aus trockenem Argon mit 4,4 µl Jodmethan und rührt 5 Stunden bei 23°C. Nach Abzug des Lösungsmittels erhält man 4,1 mg (6,2 µmol, 87%) der Titelverbindung als farblosen Schaum.
¹H-NMR (CDCl₃): δ = 0,77 (3H), 0,90 (3H), 1,05 (3H), 1,12 (3H), 1,36 (3H), 1,56 (1H), 2,09 (1H), 2,22 (1H), 2,39 (1H), 2,72 (1H), 3,08 (1H), 4,02 (2H), 4,53 (4H), 4,79 (1H), 4,93 (1H), 5,48 (1H), 6,61 (1H), 7,10-7,35 (3H), 7,52 (1H), 8,11 (2H), 8,99 (2H) ppm.

### BEISPIEL 46

### [1R-[1α,2β(2R*,3S*),4α,4aβ,9β,10aβ]]-3-(Ethoxycarbonyl)amino-2-(1-oxo-2-(dimethylamino)ethoxy)-3-(4-pyridyl)-propansäure-9-methyl-1,2,3,4,4a,9,10,10a-octahydro-1,12,12-trimethyl-4a,10a-epoxy-1,4-methanophenanthren-2-ylester

Die Lösung von 7,9 mg (15 µmol) der nach Beispiel 33 dargestellten Verbindung in 0,8 ml Dichlormethan versetzt man unter einer Atmosphäre aus trockenem Argon mit 4,7 mg N,N-Dimethylglycin, 6,3 mg 1,3-Dicyclohexylcarbodiimid 1,5 mg 4-Dimethylaminopyridin und rührt 16 Stunden bei 23°C. Man gießt in eine gesättigte Natriumchloridlösung, extrahiert mehrfach mit Dichlormethan, wäscht die vereinigten organischen Extrakte mit gesättigter Natriumchloridlösung und trocknet über Natriumsulfat. Den nach Filtration und Lösungsmittelabzug erhaltenen Rückstand reinigt man durch Chromatographie an ca. 10 ml feinem Kieselgel. Als Laufmittel dient ein Gemisch aus n-Hexan, Ethylacetat und Triethylamin. Isoliert werden 2,9 mg (4,8 µmol, 32%) der Titelverbindung als farbloses Öl.
¹H-NMR (CDCl₃): δ = 0,75 (3H), 0,86 (3H), 1,00 (3H), 1,23 (3H), 1,33 (1H), 1,40 (3H), 2,07 (1H), 2,15 (2H), 2,36 (6H), 2,59 (1H), 2,98-3,47 (3H), 4,13 (2H), 4,95 (1H), 5,43 (1H), 5,48 (1H), 6,50 (1H), 7,12-7,50 (6H), 8,54 (2H) ppm.

### BEISPIEL 47

### [1R-[1α,2β(2R*,3S*),4α,4aβ,9β,10aβ]]-3-(Ethoxycarbonyl)amino-2-(1-oxo-2-(dimethylamino)ethoxy)-3-(4-pyridyl)-propansäure-9-methyl-1,2,3,4,4a,9,10,10a-octahydro-1,12,12-trimethyl-4a,10a-epoxy-1,4-methanophenthren-2-ylester bis-hydrogen-methansolfonat

Die Losung von 3,2 mg (5,3 µmol) der nach Beispiel 46 dargestellten Verbindung in 50 µl Dichlormethan versetzt man unter einer Atmosphäre aus trockenem Argon mit 0,69 µl Methansulfonsäure und rührt 3 Stunden bei 23°C. Nach Abzug des Lösungsmittels erhält man 3,9 mg (4,9 µmol, 92%) der Titelverbindung als farblosen Schaum.

### BEISPIEL 48

### [1R-[1α,2β(2R*,3S*),4α,4aβ,9β,10aβ]]-3-(Ethoxycarbonyl)amino-2-(1-oxo-2-(dimethylamino)ethoxy)-3-(2-thienyl)-propansäure-9-methyl-1,2,3,4,4a,9,10,10a-octahydro-1,12,12-trimethyl-4a,10a-epoxy-1,4-methanophenanthren-2-ylester

In Analogie zu Beispiel 46 werden 15 mg (29 µmol) der nach Beispiel 29 dargestellten Verbindung umgesetzt. Nach Aufarbeitung und Reinigung isoliert man 11,6 mg (19 µmol, 65%) der Titelverbindung als farbloses Öl.
¹H-NMR (CDCl₃): δ = 0,73 (3H), 0,86 (3H), 0,99 (3H), 1,23 (4H), 1,39 (3H), 1,42 (1H), 2,04 (1H), 2,18 (1H), 2,39 (6H), 2,58 (1H), 3,05 (1H), 3,41(2H), 4,13(2H), 4,98 (1H), 5,48 (1H), 5,62 (1H), 6,49 (1H), 6,92 (1H), 7,09-7,31 (5H), 7,43 (1H) ppm.

### BEISPIEL 49

### [1R-[1α,2β(2R*,3S*),4α,4aβ,9β,10aβ]]-3-(Ethoxycarbonyl)amino-2-(1-oxo-2-(dimethylamino)ethoxy)-3-(2-thienyl)-propansäure-9-methyl-1,2,3,4,4a,9,10,10a-octahydro-1,12,12-trimethyl-4a,10a-epoxy-1,4-methanophenanthren-2-ylester hydrogen methansulfonat

In Analogie zu Beispiel 47 werden 9,9 mg (16 µmol) der nach Beispiel 48 dargestellten Verbindung umgesetzt. Nach Aufarbeitung und Reinigung isoliert man 11,4 mg (16 µmol, 99%) der Titelverbindung als farblosen Schaum

### BEISPIEL 50

### [1R-[1α,-2β(2R*,3S*),4α,4aβ,9β,10aβ]]-3-(Ethoxycarbonyl)amino-2-hydroxy-3-(4-hydroxyphenyl)-propansäure-9-methyl-1,2,3,4,4a,9,10,10a-octahydro-1,12,12-trimethyl-4a,10a-epoxy-1,4-methanophenanthren-2-ylester -β-Cyclodextrin-Addukt

Die Lösung von 5,4 mg (10 µmol) der nach Beispiel 9 dargestellten Verbindung in 57 µl Ethanol versetzt man mit der Lösung von 114 mg β-Cyclodextrin in 0,8 ml Wasser und erhitzt für 2 Stunden auf 50-70°C. Man läßt auf 23°C erkalten und rührt weitere 5 Tage. Man saugt den Niederschlag ab, wäscht mit 0,1 ml eines Wasser/Ethanol-Gemisches nach und trocknet bei 50°C. Isoliert werden 78 mg Addukt mit einem Wirkstoffgehalt von ca 4-6%.

### BEISPIEL 51

### [1R-[1α,2β(2R*,3S*),4α,4aβ,10aβ]]-3-(Ethoxycarbonyl)amino-2-hydroxy-3-(1-oxido-4-pyridyl)-propansäure-9-methylen-1,2,3,4,4a,9,10,10a-octahydro-1,12,12-trimethyl-4a,10a-epoxy-1,4-methanophenanthren-2-ylester

Die Lösung von 3 mg (5,8 µmol) der nach Beispiel 32 dargestellten Verbindung in einem 1:1-Gemisch aus Tetrahydrofuran und Toluol versetzt man bei 0°C nach jeweils 30 Minuten mit dreimal 1 mg einer 50%igen meta-Chlorperbenzoesäure und rührt anschließend noch 1 Stunde bei 0°C. Man gießt in eine gesättigte Natriumhydrogencarbonatlösung, extrahiert mehrfach mit Dichlormethan, wäscht die vereinigten organischen Extrakte mit gesättigter Natriumchloridlösung und trocknet über Natriumsulfat. Den nach Filtration und Lösungsmittelabzug erhaltenen Rückstand reinigt man durch Chromatographie an einer analytischen Dunnschichtplatte. Als Laufmittel dient ein Gemisch aus Ethylacetat und Ethanol, als Elutionsmittel ein Gemisch aus Dichlormethan und Methanol. Isoliert werden 1.4 mg (2,6 µmol, 44%, der Titelverbindung als farbloses Öl.
¹H-NMR (CDCl₃): δ = 0,85 (3H), 0,92 (3H), 1,09 (3H), 1,23 (3H), 1,39 (1H), 2,29 (1H), 2,71 (1H), 2,81 (1H), 3,14 (1H), 4,13 (2H), 4,53 (1H), 4,90 (1H), 5,05 (1H), 5,30 (1H), 5,54 (1H), 6,44 (1H), 7,20-7,62 (6H), 8,15 (2H) ppm.

### BEISPIEL 52

### [1R-[1α,2β(2R*,3S(2S)*),4α,4aβ,9β,10β]]-3-(Ethoxycarbonyl)amino-2-hydroxy-3-[4-[2-[[(1,1-dimethylethoxy)carbonyl]amino]-3-benzyloxy-1-oxopropoxy]pnenyl]-propansäure-9-methyl-1,2,3,4,4a,9,10,10a-octahydro-1,12,12-trimethyl-4a,10a-epoxy-1,4-methanophenanthren-2-ylester

In Analogie zu Beispiel 1 werden 18 mg (19 µmol) der nach Beispiel 52a dargestellten Verbindung umgesetzt. Nach Aufarbeitung und Reinigung isoliert man 5 mg (6 µmol, 32%) der Titelverbindung als farbloses Öl.
¹H-NMR (CDCl₃): δ = 0,78 (3H), 0,89 (3H), 1,00 (3H), 1,20 (3H), 1,40 (3H), 1,44 (9H), 1,68 (1H), 2,07 (1H), 2,20 (1H), 2,29 (1H), 2,67 (1H), 3,08 (1H), 3,21 (1H), 3,78 (1H), 3,95-4,18 (3H), 4,44-4,73 (4H), 4,92 (1H), 5,29 (1H), 5,49 (1H), 6,27 (1H), 6,98 (2H), 7,13-7,38 (8H), 7,43-7,57 (3H) ppm.

### BEISPIEL 52a

### [1R-[1α,2β(2R*,3S(2S)*),4α,4aβ,9β,10aβ]]-3-(Ethoxycarbonyl)amino-2-triisopropylsiloxy-3-[4-[2-[[(1,1-dimethylethoxy)carbonyl]amino]-3-benzyloxy-1-oxopropoxy]phenyl]-propansäure-9-methyl-1,2,3,4,4a,9,10,10a-octahydro-1,12,12-trimethyl-4a,10a-epoxy-1,4-methanophenanthren-2-ylester

In Analogie zu Beispiel 46 werden 15 mg (22 µmol) der nach Beispiel 20b dargestellten Verbindung unter Verwendung von N-Boc-O-Benzyl-L-Serin umgesetzt. Nach Aufarbeitung und Reinigung isoliert man 18 mg (19 µmol, 86%) der Titelverbindung als farbloses Öl.

### BEISPIEL 53

### [1R-[1α,2β(2R*,3S(2S)*),4α,4aβ,9β,10aβ]]-3-(Ethoxycarbonyl)amino-2-hydroxy-3-[4-[2-[[(1,1-dimethylethoxy)carbonyl]amino]-3-hydroxy-1-oxopropoxy]phenyl]-propansäure-9-methyl-1,2,3,4,4a,9,10,10a-octahydro-1,12,12-trimethyl-4a,10a-epoxy-1,4-methanophenanthren-2-ylester

In Analogie zu Beispiel 9a werden 5,1 mg (6,3 µmol) der nach Beispiel 52 dargestellten Verbindung hydriert Nach Aufarbeitung und Reinigung isoliert man 0,8 mg (1,1 µmol, 18%) der Titelverbindung als farbloses Öl.
¹H-NMR (CDCl₃): δ = 0,79 (3H), 0,89 (3H), 1,02 (3H), 1,20 (3H), 1,40 (3H), 1,48 (9H), 1,68 (1H), 2,08 (1H), 2,21 (1H), 2,29 (1H), 2,68 (1H), 3,07 (1H), 3,20 (1H), 3,98-4,21 (4H), 4,52 (1H), 4,60 (1H), 4,91 (1H), 5,28 (1H), 5,48 (1H), 6,28 (1H), 7,05 (2H), 7,14-7,36 (4H), 7,45-7,60 (3H) ppm.

### BEISPIEL 54

### [1R-[1α,2β(2R*,3S(E,E,2RS)*),4α,4aβ,9β,10aβ]]-3-(Ethoxycarbonyl)amino-2-hydroxy-3-[4-[[2-[[methoxy(3,7,11-trimethyl-2,6,10-dodecatrienyl)phosphinyl]oxy] acetyl]oxy]phenyl]-propansäure-9-methyl-1,2,3,4,4a,9,10,10a-octahydro-1,12,12-trimethyl-4a,10a-epoxy-1,4-methanophenanthren-2-ylester

In Analogie zu Beispiel 1 werden 6,0 mg (5,8 µmol) der nach Beispiel 54a dargestellten Verbindung umgesetzt. Nach Aufarbeitung und Reinigung isoliert man 1,4 mg (1,6 µmol, 28%) der Titelverbindung als farbloses Öl.
¹H-NMR (CDCl₃): δ = 0,79 (3H), 0,89 (3H), 1,01 (3H), 1,19 (3H), 1,39 (3H), 1,46-1,73 (13H), 1,90-2,20 (9H), 2,28 (1H), 2,66 (2H), 2,74 (1H), 3,07 (1H), 3,21 (1H), 3,78 (3H), 4,09 (2H), 4,52 (1H), 4,80 (2H), 4,91 (1H), 5,09 (2H), 5,15-5,32 (2H), 6,26 (1H), 7,07 (2H), 7,13-7,32 (4H), 7,44-7,60 (3H) ppm.

### BEISPIEL 54a

### [1R-[1α,2β(2R*,3S(E,E,2RS)*),4α,4aβ,9β,10aβ]]-3-(Ethoxycarbonyl)amino-2-triisopropylsilyloxy-3-[4-[[2-[[methoxy(3,7,11-trimethyl-2,6,10-dodecatrienyl) phosphinyl]oxy]acetyl]oxy]phenyl-propansäure-9-methyl-1,2,3,4,4a,9,10,10a-octahydro-1,12,12-trimethyl-4a,10a-epoxy-1,4-methanophenanthren-2-ylester

In Analogie zu Beispiel 46 werden 10 mg (15 µmol) der nach Beispiel 20b dargestellten Verbindung unter Verwendung von 2-[[methoxy(3,7,11-trimethyl-2,6,10-dodecatrienyl)phosphinyl]oxy]essigsäure, die man nach dem in J. Med. Chem 1995, 38, 439 beschriebenen Verfahren hergestellt hat, umgesetzt. Nach Aufarbeitung und Reinigung isoliert man 6 mg (5,8 µmol, 40%) der Titelverbindung als farbloses Öl.

### BEISPIEL 55

### [1R-[1α,2β(2R*3S(4R)*),4α,4aβ,9β,10aβ]]-3-(Ethoxycarbonyl)amino-2-hydroxy-3-[4-[4-[[4-(1-methylethylen)cyclohex-1-en]carbonyloxy]-1-oxobutoxy]phenyl]-propansäure-9-methyl-1,2,3,4,4a,9,10,10a-octahydro-1,12,12-trimethyl-4a,10a-epoxy-1,4-methanophenanthren-2-ylester

In Analogie zu Beispiel 1 werden 10 mg (11 µmol) der nach Beispiel 55a dargestellten Verbindung umgesetzt. Nach Aufarbeitung und Reinigung isoliert man 4,2 mg (5,5 µmol, 51%) der Titelverbindung als farbloses Öl.
¹H-NMR (CDCl₃): δ = 0,78 (3H), 0,89 (3H), 1,01 (3H), 1,19 (3H), 1,40 (3H), 1,48 (1H), 1,69 (1H), 1,74 (3H), 1,89 (1H), 2,00-2,39 (8H), 2,48 (1H), 2,58-2,72 (3H), 3,07 (1H), 3,20 (1H), 2,09 (2H), 2,23 (2H), 4,51 (1H), 4,72 (1H), 4,76 (1H), 4,91 (1H), 5,28 (1H), 6,27 (1H), 6,93-7,07 (3H), 7,12-7,32 (3H), 7,41-7,58 (3H) ppm.

### BEISPIEL 55a

### [1R-[1α,2β(2R*,3S(4R)*),4α,4aβ,9β,10aβ]]-3-(Ethoxycarbonyl)amino-2-triisopropylsilyloxy-3 [4-[4-[[4-(1-methylethylen)cyclohex-1-en]carbonyloxy]-1-oxobutoxy]phenyl]-propansäure-9-methyl-1,2,3,4,4a,9,10,10a-octahydro-1,12,12-trimethyl-4a,10a-epoxy-1,4-methanophenanthren-2-ylester

In Analogie zu Beispiel 14 werden 15 mg (22 µmol) der nach Beispiel 20b dargestellten Verbindung unter Verwendung der nach Beispiel 55b dargestellten Säurecchloridlösung umgesetzt. Nach Aufarbeitung und Reinigung isoliert man 10 mg (11 µmol, 50%) der Titelverbindung als farbloses Öl.

### BEISPIEL 55b

### 4-[[4-(1-methylethylen)cyclohex-1-en]carbonyloxy]-butansäurechlorid

Die Lösung von 35 mg (139 µmol) der nach Beispiel 55c dargestellten Säure in 1 ml wasserfreiem Toluol versetzt man bei 10°C unter einer Atmosphäre aus trockenem Argon mit 55 µl Thionylchlorid und läßt vor der weiteren Umsetzung 30 Minuten rühren.

### BEISPIEL 55c

### 4-[[4-(1-methylethylen)cyclohex-1-en]carbonyloxy]-butansäure

Die Lösung von 35 mg (147 µmol) des nach Beispiel 55d dargestellten Alkohols in 2 ml Aceton kühlt man auf -30°C, versetzt mit 112 µl einer standardisierten 8N Chromschwefelsäurelösung und rührt 1,5 Stunden. Man versetzt mit 5 ml Wasser, extrahiert mehrfach mit Diethylether, wäscht die vereinigten organischen Extrakte mit einer gesättigten Natriumchloridlösung und trocknet über Magnesiumsulfat. Den nach Filtration und Lösungsmittelabzug erhaltenen Rückstand setzt man ohne Reinigung weiter um. Isoliert werden 35 mg (138 µmol, 94%) der Titelverbindung als blass gelbes Öl.

### BEISPIEL 55d

### 4-[[4-(1-methylethylen)cyclohex-1-en]carbonyloxy]-butan-1-ol

Die Lösung von 747 mg (2,3 mmol) des nach Beispiel 55e dargestellten Ethers in einem Gemisch aus 20 ml Tetrahydrofuran und 3 ml Wasser versetzt man mit 666 mg p-Toluolsulfonsäure und erwärmt 16 Stunden auf 50°C. Man gießt in eine gesättigte Natriumhydrogencarbonatlösung, extrahiert mehrfach mit Ethylacetat, wäscht die vereinigten organischen Extrakte mit einer gesättigten Natriumchloridlösung und trocknet über Magnesiumsulfat. Den nach Filtration und Lösungsmittelabzug erhaltenen Rückstand reinigt man durch Chromatographie an ca. 150 ml feinem Kieselgel mit einem Gemisch aus n-Hexan und Ethylacetat. Isoliert werden 463 mg (1,94 mmol, 84%) der Titelverbindung als farbloses Öl.

### BEISPIEL 55e

### 4-[[4-(1-methylethylen)cyclohex-1-en]carbonyloxy]-butan-1-tetrahydropyran-2-yloxy

Die Lösung von 670 mg (4,0 mmol) (S)-Perillsäure in 40 ml wasserfreiem Toluol versetzt man unter eher Atmosphäre aus trockenem Argon bei 0°C mit 1,5 ml Thionylchlorid, läßt auf 23°C erwärmen, versetzt mit der Lösung von 697 mg 4-Hydroxy-butan 1-tetrahydropyran-2-yloxy in 20 ml wasserfreiem Dichlormethan, mit 1,44 ml Triethylamin und rührt 1 Stunde bei 23°C. Man gießt in eine gesättigte Natriumhydrogencarbonatlösung, extrahiert mehrfach mit Ethylacetat, wäscht die vereinigten organischen Extrakte mit einer gesättigten Natriumchloridlösung und trocknet über Magnesiumsulfat. Den nach Filtration und Lösungsmittelabzug erhaltenen Rückstand reinigt man durch Chromatographie an ca. 150 ml feinem Kieselgel mit einem Gemisch aus n-Hexan und Ethylacetat. Isoliert werden 747 mg (2,3 mmol, 58%) der Titelverbindung als farbloses Öl.

### BEISPIEL 56

### [1R-[1α,2β(2R*,3S(E,E,2R oder 2S)*),4α,4aβ,10aβ]]-3-[2-[[methoxy(3,7,11-trimethyl-2,6,10-dodecatrienyl)phosphinyl]oxy]acetyl]amino-2-hydroxy-3-(3-thienyl)-propansäure-9-methylen-1,2,3,4,4a,9,10,10a-octahydro-1,12,12-trimethyl-4a,10a-epoxy-1,4-methanophenanthren-2-ylester

In Analogie zu Beispiel 1 werden 2,3 mg (2,4 µmol) der nach Beispiel 56a dargestellten Verbindung umgesetzt. Nach Aufarbeitung und Reinigung isoliert man 1,3 mg (1,6 µmol, 68%) der Titelverbindung als farbloses Öl.
¹H-NMR (CDCl₃): δ = 0,83 (3H), 0,92 (3H), 1,04 (3H), 1,48-1,74 (13H), 1,91-2,13 (8H), 2,29 (1H), 2,61 (1H), 2,69 (2H), 2,78 (1H), 3,10 (1H), 3,32 (1H), 3,68 (3H), 4,38-4,59 (3H), 4,98-5,21 (5H), 5,53 (1H), 5,64 (1H), 7,13 (1H), 7,21-7,36 (4H), 7,47-7,62 (3H) ppm.

### BEISPIEL 56a

### [1R-[1α,2β(2R*,3S(E,E,2R oder 2S)*),4α,4aβ,10aβ]]-3-[2-[[methoxy(3,7,11-trimethyl-2,6,10-dodecatrienyl)phosphinyl]oxy]acetyl]amino-2-triisopropylsilyloxy-3-(3-thienyl)-propansäure-9-methylen-1,2,3,4,4a,9,10,10a-octahydro-1,12,12-trimethyl-4a,10a-epoxy-1,4-methanophenanthren-2-ylester (A) und [1R-[1α,2β (2R*,3S(E,E,2S oder 2R)*),4α,4aβ,10aβ]]-3-[2-[[methoxy(3,7,11-trimethyl-2,6,10-dodecatrienyl)phosphinyl]oxy]acetyl]amino-2-triisopropylsilyloxy-3-(3-thienyl)-propansäure-9-methylen-1,2,3,4,4a,9,10,10a-octahydro-1,12,12-trimethyl-4a,10a-epoxy-1,4-methanophenanthren-2-ylester (B)

In Analogie zu Beispiel 46 werden 10 mg (16 µmol) der nach Beispiel 56b dargestellten Verbindung unter Verwendung von 2-[[methoxy(3,7,11-trimethyl-2,6,10-dodecatrienyl)phosphinyl]oxy]essigsäure, die man nach dem in J. Med. Chem. 1995, 38, 439 beschriebenen Verfahren hergestellt hat, umgesetzt. Nach Aufarbeitung und Reinigung isoliert man 2,3 mg (2,4 µmol, 15%) der Titelverbindung A sowie 1,9 mg (2,0 µmol, 13%) der Titelverbindung B jeweils als farbloses Öl.

### BEISPIEL 56b

### [1R-[1α,2β(2R*,3S*),4α,4aβ,10aβ]]-3-amino-2-triisopropylsilyloxy-3-(3-thienyl)-propansäure-9-methylen-1,2,3,4,4a,9,10,10a-octahydro-1,12,12-trimethyl-4a,10a-epoxy-1,4-methanophenanthren-2-ylester

In Analogie zu Beispiel 40b werden 119 mg (172 µmol) der nach Beispiel 56c dargestellten Verbindung umgesetzt. Nach Aufarbeitung und Reinigung isoliert man 49 mg (81 µmol, 47%) der Titelverbindung als farbloses Öl.

### BEISPIEL 56c

### [1R-[1α,2β(2R*,3S*),4α,4aβ,10aβ]]-3-(allyloxycarbonyl)amino-2-triisopropylsiloxy-3-(3-thienyl)-propansäure-9-methylen-1,2,3,4,4a,9,10,10a-octahydro-1,12,12-trimethyl-4a,10a-epoxy-1,4-methanophenanthren-2-ylester

In Analogie zu Beispiel 1a werden 65 mg (0,23 mmol) [1R-(1α,2β,4α,4aβ,10aβ)]-9-methylen-1,2,3,4,4a,9,10,10a-octahydro-1,12,12-trimethyl-4a,10a-epoxy-1,4-methano phenanthren-2-ol unter Verwendung von (3R,4S)-1-(Allyloxycarbonyl)-3-triisopropylsilyloxy-4-(3-thienyl)-2-azetidinon, das man in Analogie zu dem in Tetrahedron 1992 auf Seite 6985ff beschriebenen Verfahren hergestellt hat, umgesetzt. Nach Aufarbeitung und Reinigung isoliert man 119 mg (172 µmol, 75%) der Titelverbindung als farbloses Öl.

### BEISPIEL 57

### [1R-[1α,2β(2R*,3S(E,E,2S oder 2R)*),4α,4aβ,10aβ]]-3-[2-[[methoxy(3,7,11-trimethyl-2,6,10-dodecatrienyl)phosphinyl]oxy]acetyl]amino-2-hydroxy-3-(3-thienyl)-propansäure-9-methylen-1,2,3,4,4a,9,10,10a-octahydro-1,12,12-trimethyl-4a,10a-epoxy,-1,4-methanophenanthren-2-ylester

In Analogie zu Beispiel 1 werden 1,9 mg (2,1 µmol) der nach Beispiel 56a dargestellten Verbindung umgesetzt. Nach Aufarbeitung und Reinigung isoliert man 1,4 mg (1,8 µmol, 88%) der Titelverbindung als farbloses Öl.
¹H-NMR (CDCl₃): δ = 0,83 (3H), 0,91 (3H), 1,03 (3H), 1,46-1,74 (13H), 1,90-2,13 (8H), 2,29 (1H), 2,55 (1H), 2,62 (1H), 2,70 (1H), 2,77 (1H), 3,09 (1H), 3,41 (1H), 3,70 (3H), 4,38-4,58 (3H), 5,00-5,19 (5H), 5,52 (1H), 5,14 (1H), 7,14 (1H), 7,20-7,36 (4H), 7,41-7,59 (3H) ppm.

### BEISPIEL 58

### [1R-[1α,2β(2R*,3S*),4α,4aβ,9β,10aβ]]-3-(Ethoxycarbonyl)amino-2-hydroxy-3-[4-(1-oxohexadecyloxy)phenyl]-propansäure-9-methyl-1,2,3,4,4a,9,10,10a-octahydro-1,12,12-trimethyl-4a,10a-epoxy-1,4-methanophenanthren-2-ylester

In Analogie zu Beispiel 1 werden 9,9 mg (11 µmol) der nach Beispiel 58a dargestellten Verbindung umgesetzt. Nach Aufarbeitung und Reinigung isoliert man 2,2 mg (2,8 µmol, 26%) der Titelverbindung als farbloses Öl.
¹H-NMR (CDCl₃): δ = 0,79 (3H), 0,88 (3H), 0,90 (3H), 1,02 (3H), 1,13-1,46 (24H), 1,19 (3H), 1,40 (3H), 1,60-1,80 (3H), 2,07 (1H), 2,20 (1H), 2,29 (1H), 2,51 (2H), 2,67 (1H), 3,07 (1H), 3,21 (1H), 4,09 (2H), 4,52 (1H), 4,92 (1H), 5,27 (1H), 6,25 (1H), 7,01 (2H), 7,12-7,32 (3H), 7,43-7,57 (3H) ppm.

### BEISPIEL 58a

### [1R-[1α,2β(2R*,3S*),4α,4aβ,9β,10aβ]]-3-(Ethoxycarbonyl)amino-2-triisopropylsilyloxy-3-[4-(1-oxohexadecyloxy)phenyl]-propansäure-9-methyl-1,2,3,4,4a,9,10,10a-octahydro-1,12,12-trimethyl-4a,10a-epoxy-1,4-methanophenanthren-2-ylester

Die Losung von 10 mg (14,5 µmol) der nach Beispiel 20b dargestellten Verbindung in 0,3 ml wasserfreiem Dichlormethan versetzt man unter einer Atmosphäre aus trockenem Argon bei 0°C mit 5,7 µl Palmitinsäurechlorid, 3,0 µl Triethylamin und läßt 30 Minuten bei 0°C reagieren. Man gießt in eine gesättigte Natriumhydrogencarbonatlösung, extrahiert mehrfach mit Ethylacetat, wäscht die vereinigten organischen Extrakte mit einer gesättigten Natriumchloridlösung und trocknet über Magnesiumsulfat. Den nach Filtration und Lösungsmittelabzug erhaltenen Rückstand reinigt man durch Chromatographie an einer analytischen Dünnschichtplatte. Als Laufmittel dient ein Gemisch aus n-Hexan und Ethylacetat, alös Elutionsmittel ein Gemisch aus Dichlormethan und Methanol. Isoliert werden 9,9 mg (10,6 µmol, 73%) der Titelverbindung als farbloses Öl.

### BEISPIEL 59

### [1R-[1α,2β(2R*,3S(2S)*),4α,4aβ,9β,10aβ]]-3-(Ethoxycarbonyl)amino-2-hydroxy-3-[4-[4-[3-[4-[[bis(phenylmethoxy)phosphinyl]oxy]phenyl]-2-[[(1,1-dimethylethoxy) carbonyl]amino]-1-oxopropoxy]phenyl]-propansäure-9-methyl-1,2,3,4,4a,9,10,10a-octahydro-1,12,12-trimethyl-4a,10a-epoxy-1,4-methanophenanthren-2-ylester

In Analogie zu Beispiel 1 werden 33 mg (29 µmol) der nach Beispiel 59a dargestellten Verbindung umgesetzt. Nach Aufarbeitung und Reinigung isoliert man 18 mg (17 µmol, 59%) der Titelverbindung als farbloses Öl.
¹H-NMR (CDCl₃): δ = 0,78 (3H), 0,89 (3H), 1,02 (3H), 1,20 (3H), 1,39 (3H), 1,44 (9H), 2,09 (1H), 2,16 (1H), 2,28 (1H), 2,68 (1H), 2,99-3,31 (4H), 4,09 (2H), 4,52 (1H), 4,73 (1H), 4,92 (1H), 4,99-5,18 (4H), 5,28 (1H), 6,27 (1H), 6,75 (2H), 6,89-7,56 (22H) ppm.

### BEISPIEL 59a

### [1R-[1α,2β(2R*,3S(2S)*),4α,4aβ,9β,10aβ]]-3-(Ethoxycarbonyl)amino-2-triisopropylsilyloxy-3-[4-[4-[3-[4-[[bis(phenylmethoxy)phosphinyl]oxy]phenyl]-2-[[(1,1-dimethylethoxy)carbonyl]amino]-1-oxopropoxy]phenyl]-propansäure-9-methyl-1,2,3,4,4a,9,10,10a-octahydro-1,12,12-trimethyl-4a,10a-epoxy-1,4-methanophenanthren-2-ylester

In Analogie zu Beispiel 46 werden 20 mg (29 µmol) der nach Beispiel 20b dargestellten Verbindung unter Verwendung von N-Boc-L-Serin-O-dibenzylphosphat umgesetzt. Nach Aufarbeitung und Reinigung isoliert man 33 mg (27 µmol, 94%) der Titelverbindung als farbloses Öl.

### BEISPIEL 60

### [1R-[1α,2β(2R*,3S*),4α,4aβ,9β,10aβ]]-3-(Methoxycarbonyl)amino-2-hydroxy-3-(4-pyridyl)-propansäure-9-methyl-1,2,3,4,4a,9,10,10a-octahydro-1,12,12-trimethyl-4a,10a-epoxy-1,4-methanophenanthren-2-ylester

In Analogie zu Beispiel 1 werden 11 mg (16,6 µmol) der nach Beispiel 60a dargestellten Verbindung umgesetzt. Nach Aufarbeitung und Reinigung isoliert man 7,5 mg (14,8 µmol, 89%) der Titelverbindung als farbloses Öl.
¹H-NMR (CDCl₃): δ = 0,78 (3H), 0,89 (3H), 1,02 (3H), 1,40 (3H), 1,56 (1H), 2,10 (1H), 2,18 (1H), 2,24 (1H), 2,68 (1H), 3,09 (1H), 3,63 (3H), 4,56 (1H), 4,92 (1H), 5,30 (1H), 6,49 (1H), 7,13-7,56 (6H), 8,52 (2H) ppm.

### BEISPIEL 60a

### [1R-[1α,2β(2R*,3S*),4α,4aβ,9β,10aβ]]-3-(Methoxycarbonyl)amino-2-triisopropylsilyloxy-3-(4-pyridyl)-propansäure-9-methyl-1,2,3,4,4a,9,10,10a-octahydro-1,12,12-trimethyl-4a,10a-epoxy-1,4-methanophenanthren-2-ylester

In Analogie zu Beispiel 1a werden 11,4 mg (40 µmol) der nach Beispiel 33b dargestellten Verbindung A unter Verwendung von (3R,4S)-1-(Methoxycarbonyl)-3-triisopropylsilyloxy-4-(4-pyridyl)-2-azetidinon, das man in Analogie zu dem in Tetrahedron 1992 auf Seite 6985ff beschriebenen Verfahren hergestellt hat, umgesetzt. Nach Aufarbeitung und Reinigung isoliert man 11 mg (16,6 µmol, 42%) der Titelverbindung als farbloses Öl.

### BEISPIEL 61

### [1R-[1α,2β(1R*,2R(2R,3S)* oder 1S*,2S(2R,3S)*),4α,4aβ,9β,10aβ]]-2-[[3-(Ethoxycarbonyl)amino-2-hydroxy-3-(4-pyridyl)-1-oxopropyl]amino]cyclohexancarbonsäure-9-methyl-1,2,3,4,4a,9,10,10a-octahydro-1,12,12-trimethyl-4a,10a-epoxy-1,4-methanophenanthren-2-ylester

In Analogie zu Beispiel 1 werden 4,4 mg (5,5 µmol) der nach Beispiel 61a dargestellten Verbindung umgesetzt. Nach Aufarbeitung und Reinigung isoliert man 1,6 mg (2,5 µmol, 46%) der Titelverbindung als farbloses Öl.
¹H-NMR (CDCl₃): δ = 0,74 (3H), 0,89 (3H), 1,02 (3H), 1,21 (3H), 1,28 (3H), 1,37 (2H), 1,50-1,81 (6H), 1,88 (1H), 2,14 (2H), 2,24 (1H), 2,43 (1H), 2,61 (1H), 3,10 (1H), 3,86-4,13 (3H), 4,20 (1H), 4,69 (1H), 4,79 (1H), 4,98 (1H), 6,33 (1H), 6,61 (1H), 6,99-7,42 (6H), 8,38 (2H) ppm.

### BEISPIEL 61a

### [1R-[1α,2β(1R*,2R(2R,3S)* oder 1S*,2S(2R,3S)*),4α,4aβ,9β,10aβ]]-2-[[3-(Ethoxycarbonyl)amino-2-triisopropylsilyloxy-3-(4-pyridyl)-1-oxopropyl]amino] cyclohexancarbonsäure-9-methyl-1,2,3,4,4a,9,10,10-octahydro-1,12,12-trimethyl-4a,10a-epoxy-1,4-methanophenanthren-2-ylester

In Analogie zu Beispiel 40a werden 13 mg (32 µmol) der nach Beispiel 61b dargestellten Verbindung unter Verwendung der nach Beispiel 42d dargestellten Säure umgesetzt. Nach Aufarbeitung und Reinigung isoliert man 4,4 mg (5,5 µmol, 17%) der Titelverbindung als farbloses Öl.

### BEISPIEL 61b

### [1R-[1α,2β(1R*,2R(2R,3S)* oder 1S*,2S(2R,3S)*),4α,4aβ,9β,10aβ]]-2-aminocyclohexancarbonsäure-9-methyl-1,2,3,4,4a,9,10,10a-octahydro-1,12,12-trimethyl-4a,10a-epoxy-1,4-methanophenanthren-2-ylester

In Analogie zu Beispiel 40b werden 18,5 mg (38 µmol) der nach Beispiel 61c dargestellten Verbindung B umgesetzt. Nach Aufarbeitung und Reinigung isoliert man 13 mg (32 µmol, 85%) der Titelverhindung als farbloses Öl.

### BEISPIEL 61c

### [1R-[1α,2β(1R*,2R(2R,3S)*),4α,4aβ,9β,10aβ]]-2-(Allyloxycarbonyl)aminocyclo hexancarbonsäure-9-methyl-1,2,3,4,4a,9,10,10a-otahydro-1,12,12-trimethyl-4a,10a-epoxy-1,4-methanophenanthren-2-ylester (A) und [1R-[1α,2β(1S*,2S(2R,3S)*),4α,4aβ,9β,10aβ]]-2-(Allyloxycarbonyl)aminocyclo hexancarbonsäure-9-methyl-1,2,3,4,4a,9,10,10a-octahydro-1,12,12-trimethyl-4a,10a-epoxy-1,4-methanophenanthren-2-ylester (B)

In Analogie zu Beispiel 46 werden 39,6 mg (139 µmol) der nach Beispiel 33b dargestellten Verbindung A unter Verwendung der nach Beispiel 61d dargestellten Säure umgesetzt. Nach Aufarbeitung und Reinigung isoliert man 23 mg (47 µmol, 34%) der Titelverbindung B sowie 33 mg (67 µmol, 48%) der Titelverbindung A jeweils als farbloses Öl.

### BEISPIEL 61d

### (1RS,2RS)-2-(Allyloxycarbonyl)aminocyclohexancarbonsäure

In Analogie zu Beispiel 40d werden 180 mg (0,71 mmol) der nach Beispiel 61e dargestellten Verbindung umgesetzt. Nach Aufarbeitung und Reinigung isoliert man 95 mg (418 µmol, 59%) der Titelverbindung als farbloses Öl.

### BEISPIEL 61e

### (1RS,2RS)-2-(Allyloxycarbonyl)aminocyclohexancarbonsäureethylester

Die Lösung von 166 mg (0,8 mmol) (±)-trans-2-amino-1-carbonsäureethylester Hydrochlorid in 8 ml wasserfreiem Dichlormethan versetzt man bei 0°C unter einer Atmosphäre aus trockenem Argon mit 586 mg 4-Dimethylaminopyridin, 0,26 ml Chlorameisensäureallylester und rührt 18 Stunden bei 23°C. Man gießt in eine gesättigte Natriumhydrogencarbonatlösung, extrahiert mehrfach mit Ethylacetat, wäscht die vereinigten organischen Extrakte mit einer gesättigten Natriumchloridlösung und trocknet über Magnesiumsulfat. Den nach Filtration und Lösungsmittelabzug erhaltenen Rückstand setzt man ohne Reinigung weiter um. Isoliert werden 190 mg (744 µmol, 93%) der Titelverbindung als farbloses Öl.

### BEISPIEL 62

### [1R-[1α,2β(1S*,2S(2R,3S)* oder 1R*,2R(2R,3S)*),4α,4aβ,9β,10aβ]]-2-[[3-(Ethoxycarbonyl)amino-2-hydroxy-3-(4-pyridyl)-1-oxopropyl]amino]cyclohexancarbonsäure-9-methyl-1,2,3,4,4a,9,10,10a-octahydro-1,12,12-trimethyl-4a,10a-epoxy-1,4-methanophenanthren-2-ylester

In Analogie zu Beispiel 1 werden 11 mg (13,7 µmol) der nach Beispiel 62a dargestellten Verbindung umgesetzt. Nach Aufarbeitung und Reinigung isoliert man 4,9 mg (7,6 µmol, 55%) der Titelverbindung als farbloses Öl.
¹H-NMR (CDCl₃): δ = 0,77 (3H), 0,91 (3H), 1,03 (3H), 1,15-2,17 (12H), 1,23 (3H), 1,32 (3H), 2,32 (1H), 2,60 (1H), 2,68 (1H), 3,03 (1H), 3,82 (1H), 4,08 (2H), 4,30 (1H), 4,80 (1H), 5,11 (1H), 5,94 (1H), 7,07-7,34 (6H), 7,39 (1H), 8,23 (2H) ppm.

### BEISPIEL 62a

### [1R-[1α,2β(1S*,2S(2R,3S)* oder 1R*,2R(2R,3S)*),4α,4aβ,9β,10aβ]]-2-[[3-(Ethoxycarbonyl)amino-2-triisopropylsilyloxy-3-(4-pyridyl)-1-oxopropyl]amino] cyclohexancarbonsäure-9-methyl-1,2,3,4,4a,9,10,10a-octahydro-1,12,12-trimethyl-4a,10a-epoxy-1,4-methanophenanthren-2-ylester

In Analogie zu Beispiel 40a werden 18 mg (44 µmol der nach Beispiel 62b dargestellten Verbindung unter Verwendung der nach Beispiel 42d dargestellten Säure umgesetzt. Nach Aufarbeitung und Reinigung isoliert man 11 mg (13,7 µmol, 31%) der Titelverbindung als farbloses Öl.

### BEISPIEL 62b

### [1R-[1α,2β(1S*,2S(2R,3S)* oder 1R*,2R(2R,3S)*),4α,4aβ,9β,10aβ]]-2-aminocyclohexancarbonsäure-9-methyl-1,2,3,4,4a,9,10,10a-octahydro-1,12,12-trimethyl-4a,10a-epoxy-1,4-methanophenanthren-2-ylester

In Analogie zu Beispiel 40b werden 28 mg (57 µmol) der nach Beispiel 61c dargestellten Verbindung A umgesetzt. Nach Aufarbeitung und Reinigung isoliert man 18 mg (44 µmol, 77%) der Titelverbindung als farbloses Öl.

### BEISPIEL 63

### [1R-[1α,2β(2R*,3S*),4α,4aβ,9β,10aβ]]-3-(Ethoxycarbonyl)amino-2-hydroxy-3-(4-hydroxyphenyl)-propansäure-9-methyl-1,2,3,4,4a,9,10,10a-octahydro-1,12,12-trimethyl-4a,10a-epoxy-1,4-methanophenanthren-2-ylester

In Analogie zu Beispiel 1 werden 24 mg (35 µmol) der nach Beispiel 63a dargestellten Verbindung umgesetzt. Nach Aufarbeitung und Reinigung isoliert man 11,7 mg (22 µmol, 62%) der Titelverbindung als farbloses Öl.
¹H-NMR (CDCl₃): δ = 0,77 (3H), 0,90 (3H), 1,06 (3H), 1,21 (3H), 1,43 (3H), 1,58 (1H), 1,71 (1H), 2,30 (1H), 2,46 (1H), 2,74 (1H), 3,03 (1H), 3,13 (1H), 4,09 (2H), 4,48 (1H), 4,83 (1H), 5,19 (1H), 5,26 (1H), 6,67 (2H), 6,85 (1H), 7,13-7,40 (5H), 7,51 (1H) ppm.

### BEISPIEL 63a

### [1R-[1α,2β(2R*,3S*),4α,4aβ,9β,10aβ]]-3-(Ethoxycarbonyl)amino-2-triisopropylsilyloxy-3-(4-hydroxyphenyl)-propansäure-9-methyl-1,2,3,4,4a,9,10,10a-octahydro-1,12,12-trimethyl-4a,10a-epoxy-1,4-methanophenanthren-2-ylester

In Analogie zu Beispiel 1a werden 14,9 mg (52 µmol) der nach Beispiel 43b dargestellten Verbindung A unter Verwendung von (3R,4S)-1-Ethoxycarbonyl-3-triisopropylsilyloxy-4-(4-hydroxyphenyl)-2-azetidinon, das man in Analogie zu dem in Tetrahedron 1992 auf Seite 6985ff beschriebenen Verfahren hergestellt hat, umgesetzt. Nach Aufarbeitung und Reinigung isoliert man 24 mg (35 µmol, 67%) der Titelverbindung als farbloses Öl.

### BEISPIEL 64

### [1R-[1α,2β(2R*,3S*),4α,4aβ,9β,10aβ]]-3-[[[(1-Methyl)ethylamino]carbonyl] amino]-2-hydroxy-3-(4-pyridyl)-propansäure-9-methyl-1,2,3,4,4a,9,10,10a-octahydro-1,12,12-trimethyl-4a,10a-epoxy-1,4-methanophenanthren-2-ylester

In Analogie zu Beispiel 1 werden 13,4 mg (19 µmol) der nach Beispiel 64a dargestellten Verbindung umgesetzt. Nach Aufarbeitung und Reinigung isoliert man 8,9 mg (16,7 µmol, 88%) der Titelverbindung als farbloses Öl.
¹H-NMR (CDCl₃): δ = 0,63 (3H), 0,74 (3H), 0,84 (3H), 0,94 (3H), 1,05 (3H), 1,39 (3H), 1,76 (1H), 2,18 (2H), 2,45 (1H), 2,77 (1H), 3,14 (1H), 3,66 (1H), 4,62 (1H), 4,80 (1H), 5,11 (2H), 6,43 (1H), 7,15-7,37 (5H), 7,52 (1H), 8,53 (2H) ppm.

### BEISPIEL 64a

### [1R-[1α,2β(2R*,3S*),4α,4aβ,9β,10aβ]]-3-[[[(1-Methyl)ethylamino]carbonyl] amino]-2-triisopropylsilyloxy-3-(4-pyridyl)-propansäure-9-methyl-1,2,3,4,4a,9,10, 10a-octahydro-1,12,12-trimethyl-4a,10a-epoxy-1,4-methanophenanthren-2-ylester

In Analogie Zu Beispiel 1a werden 14,2 mg (23 µmol) der nach Beispiel 42b dargestellten Verbindung unter Verwendung von (3R,4S)-1-[[(1-Methyl)ethylamino] carbonyl]-3-triisopropylsilyloxy-4-(4-pyridyl)-2-azetidinon, das man in Analogie zu dem in Tetrahedron 1992 auf Seite 6985ff beschriebenen Verfahren hergestellt hat, umgesetzt. Nach Aufarbeitung und Reinigung isoliert man 13,4 mg (19 µmol, 84%) der Titelverbindung als farbloses Öl.

### BEISPIEL 65

### [1R-[1α,2β(2R*,3S*),4α,4aβ,9β,10aβ]]-3-(Butoxycarbonyl)amino-2-hydroxy-3-(4-pyridyl)-propansäure-9-methyl-1,2,3,4,4a,9,10,10a-octahydro-1,12,12-trimethyl-4a,10a-epoxy-1,4-methanophenanthren-2-ylester

In Analogie zu Beispiel 1 werden 14,8 mg (21 µmol) der nach Beispiel 65a dargestellten Verbindung umgesetzt. Nach Aufarbeitung und Reinigung isoliert man 10,4 mg (19 µmol, 90% der Titelverbindung als farbloses Öl.
¹H-NMR (CDCl₃): δ = 0,54-0,95 (11H), 1,02 (3H), 1,20-1,47 (4H), 1,50-1,63 (2H), 2,09 (1H), 2,16 (1H), 2,27 (1H), 2,68 (1H), 3,08 (1H), 3,39 (breit, 1H), 3,82 (1H), 4,05 (1H), 4,56 (1H), 4,93 (1H), 5,28 (1H), 6,30 (1H), 7,12-7,33 (3H), 7,36-7,53 (3H), 8,52 (2H) ppm.

### BEISPIEL 65a

### [1R-[1α,2β(2R*,3S*),4α,4aβ,9β,10aβ]]-3-(Butoxycarbonyl)amino-2-triisopropylsilyloxy-3-(4-pyridyl)-propansäure-9-methyl-1,2,3,4,4a,9,10,10a-octahydro-1,12,12-trimethyl-4a,10a-epoxy-1,4-methanophenanthren-2-ylester

In Analogie zu Beispiel 1a werden 11,4 mg (40 µmol) der nach Beispiel 33b dargestellten Verbindung A unter Verwendung von (3R,4S)-1-Butoxycarbonyl-3 triisopropylsilyloxy-4-(4-pyridyl)-2-azetidinon, das man in Analogie zu dem in Tetrahedron 1992 auf Seite 6985ff beschriebenen Verfahren hergestellt hat, umgesetzt. Nach Aufarbeitung und Reinigung isoliert man 14,8 mg (21 µmol, 53%) der Titelverbindung als farbloses Öl.

### BEISPIEL 66

### [1R-[1α,2β(2R*,3S*),4α,4aβ,9β,10aβ]]-3-[(1-Oxopropyl)amino]-2-hydroxy-3-(4-pyridyl)-propansäure-9-methyl-1,2,3,4,4a,9,10,10a-octahydro-1,12,12-trimethyl-4a,10a-epoxy-1,4-methanophenanthren-2-ylester

In Analogie zu Beispiel 1 werden 10,9 mg (16 µmol) der nach Beispiel 66a dargestellten Verbindung umgesetzt. Nach Aufarbeitung und Reinigung isoliert man 6,7 mg (13 µmol, 81%) der Titelverbindung als farbloses Öl.
¹H-NMR (CDCl₃): δ = 0,76 (3H), 0,90 (3H), 0,98 (3H), 1,10 (3H), 1,33 (3H), 1,68 (1H), 2,09 (2H), 2,30 (3H), 2,69 (1H), 3,08 (1H), 3,72 (1H, breit), 4,57 (1H), 5,05 (1H), 5,49 (1H), 7,10 (1H), 7,16 (1H), 7,21-7,40 (4H), 7,48 (1H), 8,52 (2H) ppm.

### BEISPIEL 66a

### [1R-[1α,2β(2R*,3S*),4α,4aβ,9β,10aβ]]-3-[(1-Oxopropyl)amino]-2-triisopropylsilyloxy-3-(4-pyridyl)-propansäure-9-methyl-1,2,3,4,4a,9,10,10a-octahydro-1,12,12-trimethyl-4a,10a-epoxy-1,4-methanophenanthren-2-ylester

In Analogie zu Beispiel 1a werden 11,4 mg (40 µmol) der nach Beispiel 33b dargestellten Verbindung A unter Verwendung von (3R,4S)-1-(1-Oxopropyl)-3-triisopropylsilyloxy-4-(4-pyridyl)-2-azetidinon, das man in Analogie zu dem in Tetrahedron 1992 auf Seite 6985ff beschriebenen Verfahren hergestellt hat, umgesetzt. Nach Aufarbeitung und Reinigung isoliert man 10,9 mg (16 µmol, 41%) der Titelverbindung als farbloses Öl.

### BEISPIEL 67

### [1R-[1α,2β(2R*,3S*),4α,4aβ,9β,10aβ]]-3-[(1-Oxobutyl)amino]-2-hydroxy-3-(4-pyridyl)-propansäure-9-methyl-1,2,3,4,4a,9,10,10a-octahydro-1,12,12-trimethyl-4a,10a-epoxy-1,4-methanophenanthren-2-ylester

In Analogie zu Beispiel 1 werden 10,4 mg (15 µmol) der nach Beispiel 67a dargestellten Verbindung umgesetzt. Nach Aufarbeitung und Reinigung notiert man 6,3 mg (12 µmol, 81%) der Titelverbindung als farbloses Öl.
¹H-NMR (CDCl₃): δ = 0,78 (3H), 0,82 (3H) 0,90 (3H), 0,99 (3H), 1,35 (3H), 1,62 (2H), 1,70 (1H), 2,09 (2H), 2,25 (2H), 2,32 (1H), 2,70 (1H), 3,09 (1H), 3,60 (1H, breit), 4,57 (1H), 5,07 (1H), 5,48 (1H), 7,12 (1H), 7,18 (1H), 7,21-7,40 (4H), 7,48 (1H), 8,52 (2H) ppm.

### BEISPIEL 67a

### [1R-[1α,2β(2R*,3S*),4α,4aβ,9β,10aβ]]-3-[(1-Oxobutyl)amino]-2-triisopropylsilyloxy-3-(4-pyridyl)-propansäure-9-metyl-1,2,3,4,4a,9,10,10a-octahydro-1,12,12-trimethyl-4a,10a-epoxy-1,4-methanophenanthren-2-ylester

In Analogie zu Beispiel 1a werden 11,4 mg (40 µmol) der nach Beispiel 33b dargestellten Verbindung A unter Verwendung von (3R,4S)-1-(1-Oxobutyl)-3-triisopropylsilyloxy-4-(4-pyridyl)-2-azetidinon das man in Analogie zu dem in Tetrahedron 1992 auf Seite 6985ff beschriebenen Verfahren hergestellt hat, umgesetzt. Nach Aufarbeitung und Reinigung isoliert man 10,4 mg (15 µmol, 38%) der Titelverbindung als farbloses Öl.

### BEISPIEL 68

### [1R-[1α,2β(2R*,3S*),4α,4aβ,9β,10aβ]]-3-[(Methoxycarbonyl)amino]-2-hydroxy-3-(3-thienyl)-propansäure-9-methyl-1,2,3,4,4a,9,10,10a-octahydro-1,12,12-trimethyl-4a,10a-epoxy-1,4-methanophenanthren-2-ylester

In Analogie zu Beispiel 1 werden 32 mg (48 µmol) der nach Beispiel 68a dargestellten Verbindung umgesetzt. Nach Aufarbeitung und Reinigung isoliert man 15 mg (29 µmol, 61%) der Titelverbindung als farbloses Öl.
¹H-NMR (CDCl₃): δ = 0,77 (3H), 0,88 (3H), 1,03 (3H), 1,41 (3H), 1,51 (1H), 2,10 (1H), 2,21 (1H), 2,25 (1H), 2,65 (1H), 3,09 (1H), 3,28 (1H), 3,67 (3H), 4,51 (1H), 4,88 (1H), 5,36 (1H), 6,43 (1H), 7,13-7,33 (5H), 7,40 (1H), 7,50 (1H) ppm.

### BEISPIEL 68a

### [1R-[1α,2β(2R*,3S*),4α,4aβ,9β,10aβ]]-3-[(Methoxy-carbonyl)amino]-2-triisopropylsilyloxy-3-(3-thienyl)-propansäure-9-methyl-1,2,3,4,4a,9,10,10a-octahydro-1,12,12-trimethyl-4a,10a-epoxy-1,4-methanophenanthren-2-ylester

In Analogie zu Beispiel 1a werden 17 mg (60 µmol) der nach Beispiel 33b dargestellten Verbindung A unter Verwendung von (3R,4S)-1-(Methoxycarbonyl)-3-triisopropylsilyloxy-4-3-thienyl)-2-azetidinon, das man in Analogie zu dem in Tetrahedron 1992 auf Seite 6985ff beschriebenen Verfahren hergestellt hat, umgesetzt. Nach Aufarbeitung und Reinigung isoliert man 32 mg (48 µmol, 80%) der Titelverbindung als farbloses Öl.

### BEISPIEL 69

### [1R-[1α,2β(2R*,3S*),4α,4aβ,9β,10aβ]]-3-(Ethoxycarbonyl)amino-2-hydroxy-3-(4-pyridyl)-propansäure-9-methyl-1,2,3,4,4a,9,10,10a-octahydro-1,12,12-trimethyl-10a-hydroxy-1,4-methanophenanthren-2-ylester

In Analogie zu Beispiel 9a werden 7,0 mg (13 µmol) der nach Beispiel 33 dargestellten Verbindung umgesetzt. Nach Aufarbeitung und Reinigung isoliert man 1,0 mg (2 µmol, 14%) der Titelverbindung als farbloses Öl
¹H-NMR (CDCl₃): δ = 0,55 (3H), 0,93 (3H), 1,02 (3H), 1,25 (3H), 1,41 (3H), 1,47 (1H), 1,80 (1H), 2,18 (1H), 2,47 (1H), 2,75 (1H), 3,02 (1H), 3,10 (2H), 3,38 (1H, breit), 4,12 (2H), 4,66 (1H), 5,22 (1H), 5,33 (1H), 6,22 (1H), 7,10-7,25 (4H), 7,36 (2H), 8,58 (2H) ppm.

### BEISPIEL 70

### [1R-[1α,2β(2R*,3S*),4α,4aβ,9α,10aβ]]-3-(Ethoxycarbonyl)amino-2-hydroxy-3-(4-pyridyl)-propansäure-9-methyl-1,2,3,4,4a,9,10,10a-octahydro-1,12,12-trimethyl-10a-hydroxy-1,4-methanophenanthren-2-ylester

In Analogie zu Beispiel 1 werden 5,4 mg (8 µmol) der nach Beispiel 70a dargestellten Verbindung umgesetzt. Nach Aufarbeitung und Reinigung isoliert man 1,0 mg (1,9 µmol, 24%) der Titelverbindung als farbloses Öl.
¹H-NMR (CDCl₃): δ = 0,56 (3H), 0,96 (3H), 1,01 (3H), 1,20-1,33 (3H), 1,42 (3H), 1,53-1,84 (4H), 2,53 (1H), 2,82 (1H), 2,92 (1H), 3,11 (1H), 3,45 (1H), 4,10 (2H), 4,69 (1H), 5,22 (1H), 5,45 (1H), 5,95 (1H), 7,12-7,43 (6H), 8,59 (2H) ppm.

### BEISPIEL 70a

### [1R-[1α,2β(2R*,3S*),4α,4aβ,9α,10aβ]]-3-(Ethoxycarbonyl)amino-2-triisopropylsilyloxy-3-(4-pyridyl)-propansäure-9-methyl-1,2,3,4,4a,9,10,10a-octahydro-1,12,12-trimethyl-10a-hydroxy-1,4-methanophenanthren-2-ylester

In Analogie Zu Beispiel 1a werden 14,3 mg (50 µmol) der nach Beispiel 70b dargestellten Verbindung unter Verwendung von (3R,4S)-1-(Ethoxycarbonyl)-3-triisopropylsilyloxy-4-(4-pyridyl)-2-azetidinon, das man in Analogie zu dem in Tetrahedron 1992 auf Seite 6985ff beschriebenen Verfahren hergestellt hat, umgesetz. Nach Aufarbeitung und Reinigung isoliert man 15,7 mg (23 µmol, 46%) der Titelverbindung als farbloses Öl.

### BEISPIEL 70b

### [1R-[1α,2β,4α,4aβ,9α,10aβ)]-9-methyl-1,2,3,4,4a,9,10,10a-octahydro-1,12,12-trimethyl-10a-hydroxy-1,4-methanophenanthren-2-ol

Die Lösung von 100 mg (354 µmol) [1R-(1α,2β,4α,4aβ,10aβ)]-9-methylen-1,2,3,4,4a,9,10,10a-octahydro-1,12,12-trimethyl-4a,10a-epoxy-1,4-methanophenanthren-2-ol, das man in Analogie zu dem in J. Am. Chem Soc. 1992, auf Seite 5879ff beschriebenen Verfahren hergestellt hat, in 3,5 ml wasserfreiem Tetrahydrofuran versetzt man mit 40 mg Tris-triphenylphosphin-rhodium(I)-chlorid und hydriert unter einer Atmosphäre aus Wasserstoff unter kräftigem Schütteln. Nach erfolgter Wasserstoffaufnahme engt man ein und chromatographiert über ca. 70 ml feines Kieselgel mit einem Gemisch aus n-Hexan und Ethylacetat. Isoliert werden 14,3 mg (50 µmol, 14%) der Titelverbindung als farbloser Feststoff neben den, in Beispiel 33b unter Variante B erwähnten Produkten.

### BEISPIEL 71

### [1R-[1α,2β(2R*,3S*),4α,4aβ,9β,10aβ]]-3-(Methoxycarbonyl)amino-2-hydroxy-3-(4-pyridyl)-propansäure-9-methyl-1,2,3,4,4a,9,10,10a-octahydro-1,12,12-trimethyl-10a-hydroxy-1,4-methanophenanthren-2-ylester

In Analogie zu Beispiel 9a werden 7,7 mg (15 µmol) der nach Beispiel 60 dargestellten Verbindung umgesetzt. Nach Aufarbeitung und Reinigung isoliert man 1,3 mg (2,6 µmol, 17%) der Titelverbindung als farbloses Öl.
¹H-NMR (CDCl₃): δ = 0,54 (3H), 0,93 (3H), 1,01 (4H), 1,41 (3H), 1,46 (1H), 1,80 (1H), 2,18 (1H), 2,48 (1H), 2,75 (1H), 3,02 (1H), 3,09 (1H), 3,12 (1H), 3,67 (3H), 4,66 (1H), 5,20 (1H), 5,32 (1H), 6,42 (1H), 7,09-7,31 (4H), 7,36 (2H), 8,57 (2H) ppm.

### BEISPIEL 72

### [1R-[1α,2β(2R*,3S*),4α,4aβ,9β,10aβ]]-3-(Ethoxycarbonyl)amino-2-hydroxy-3-(3-pyridyl)-propansäure-9-methyl-1,2,3,4,4a,9,10,10a-octahydro-1,12,12-trimethyl-4a,10a-epoxy-1,4-methanophenanthren-2-ylester

In Analogie zu Beispiel 1 werden 60 mg (88 µmol) der nach Beispiel 72a dargestellten Verbindung umgesetzt. Nach Aufarbeitung und Reinigung isoliert man 32 mg (61 µmol, 70%) der Titelverbindung als farbloses Öl.
¹H-NMR (CDCl₃): δ = 0,78 (3H), 0,88 (3H), 1,02 (3H), 1,18 (3H), 1,39 (3H), 1,66 (1H), 2,09 (1H), 2,22 (1H), 2,29 (1H), 2,68 (1H), 3,07 (1H), 3,41 (breit, 1H), 4,10 (2H), 4,56 (1H), 4,91 (1H), 5,31 (1H), 6,38 (1H), 7,10-7,37 (4H), 7,49 (1H), 7,88 (1H), 8,50 (1H), 8,25 (1H) ppm.

### BEISPIEL 72a

### [1R-[1α,2β(2R*,3S*),4α,4aβ,9β,10aβ]]-3-(Ethoxycarbonyl)amino-2-triisopropylsilyloxy-3-(3-pyridyl)-propansäure-9-methyl-1,2,3,4,4a,9,10,10a-octahydro-1,12,12-trimethyl-4a,10a-epoxy-1,4-methanophenanthren-2-ylester

In Analogie zu Beispiel 1a werden 25 mg (88 µmol) der nach Beispiel 33b dargestellten Verbindung A unter Verwendung von (3R,4S)-1-(Ethoxycarbonyl)-3-triisopropylsilyloxy-4-(3-pyridyl)-2-azetidinon, das man in Analogie zu dem in Tetrahedron 1992 auf Seite 6985ff beschriebenen Verfahren hergestellt hat, umgesetzt. Nach Aufarbeitung isoliert man 60 mg (88 µmol, 100%) der Titelverbindung als farbloses Öl, das man ohne Reinigung weiter umsetzt.

### BEISPIEL 73

### [1R-[1α,2β(2R*,3S*),4α,4aβ,9β,10aβ]]-3-(Methoxycarbonyl)amino-2-hydroxy-3-(3-thienyl)-propansäure-9-methyl-1,2,3,4,4a,9,10,10a-octahydro-1,12,12-trimethyl-10a-hydroxy-1,4-methanophenanthren-2-ylester

In Analogie zu Beispiel 9a werden 8 mg (16 µmol) der nach Beispiel 68 dargestellten Verbindung umgesetzt. Nach Aufarbeitung und Reinigung isoliert man 1,5 mg (2,9 µmol, 18%) der Titelverbindung als farbloses Öl.
¹H-NMR (CDCl₃): δ = 0,55 (3H), 0,98 (3H), 1,00 (3H), 1,40 (3H), 1,94 (1H), 2,22 (2H), 2,43 (1H), 2,69 (1H), 3,04 (2H), 3,33 (1H), 3,68 (3H), 4,00 (1H), 4,62 (1H), 5,14 (1H), 5,38 (1H), 5,99 (1H), 7,12 (2H), 7,18-7,35 (4H), 7,42 (1H) ppm.

### BEISPIEL 74

### [1R-[1α,2β(2R*,3S*),4α,4aβ,9α,10aβ]]-3-(Ethoxycarbonyl)amino-2-hydroxy-3-(4-pyridyl)-propansäure-9-hydroxy-9-hydroxmethyl-1,2,3,4,4a,9,10,10a-octahydro-1,12,12-trimethyl-4a,10a-epoxy-1,4-methanophenanthren-2-ylester

In Analogie zu Beispiel 1 werden 28 mg (40 µmol) der nach Beispiel 74a dargestellten Verbindung umgesetzt. Nach Aufarbeitung und Reinigung isoliert man 5,5 mg (10 µmol, 25%) der Titelverbindung als farbloses Öl.
¹H-NMR (CDCl₃): δ = 0,77 (3H), 0,87 (3H), 1,14 (3H), 1,30 (3H), 1,55 (1H), 2,06 (1H), 2,39 (1H), 2,68 (1H), 3,06 (1H), 3,30 (breit, 2H), 3,97 (1H), 4,10-4,26 (3H), 4,40 (1H), 4,42 (1H), 4,72 (1H), 5,56 (1H), 5,68 (1H), 7,31-7,48 (4H), 7,53 (1H), 7,79 (1H), 8,56 (breit, 2H) ppm.

### BEISPIEL 74a

### [1R-[1α,2β(2R*,3S*),4α 4aβ,9α,10aβ]]-3-Ethoxycarbonyl)amino-2-triisopropylsilyloxy-3-(4-pyridyl)-propansäure-9-hydroxy-9-hydroxymethyl-1,2,3,4,4a,9,10,10a-octahydro-1,12,12-trimethyl-4a,10a-epoxy-1,4-methanophenanthren-2-ylester

In Analogie zu Beispiel 1a werden 12,7 mg (40 µmol) der nach Beispiel 74b dargestellten Verbindung unter Verwendung von (3R,4S)-1-(Ethoxycarbonyl)-3-trisopropylsilyloxy-4-(4-pyridyl)-2-azetidinon, das man in Analogie zu dem in Tetrahedron 1992 auf Seite 6985ff beschriebenen Verfahren hergestellt hat, umgesetzt. Nach Aufarbeitung isoliert man 28 mg (39 µmol, 99%) der Titelverbindung als farbloses Öl, das man ohne Reinigung weiter umsetzt.

### BEISPIEL 74b

### [1R-(1α,2β,4a,4aβ,9α,10aβ)]-9-hydroxy-9-hydroxymethyl-1,2,3,4,4a,9,10,10a-octahydro- 1,12,12-trimethyl-4a,10a-epoxy-1,4-methanophenanthren-2-ol

Die Lösung von 77 mg (273 µmol) [1R-(1α,2β,4α,4aβ,10aβ)]-9-methylen-1, 2,3,4,4a,9,10,10a-octahydro-1,12,1,-trimethyl-4a,10a-epoxy-1,4-methanophenanthren-2-ol, das man in Analogie zu dem in J. Am. Chem. Soc. 1992, auf Seite 5879ff beschriebenen Verfahren hergestellt hat, in 5,4 ml Aceton versetzt man mit 273 mg N-Methylmorpholin-N-oxid, 0,55 ml einer 2,5%igen Lösung von Osmiumtetroxid in tert.-Butanol, 10,8 ml Wasser und rührt 1 Stunde bei 40°C. Man kühlt auf 0°C, versetzt mit Natriumthiosulfatlösung und extrahiert mehrfach mit Dichlormethan. Die vereinigten organischen Extrakte wäscht man mit Wasser und gesättigter Natriumchloridlösung und trocknet über Natriumsulfat. Den nach Filtration und Lösungsmittelabzug erhaltenen Rückstand reinigt man durch Chromatographie an 2 analytischen Dünnschichtplatten. Als Laufmittel dient ein Gemisch aus n-Hexan und Ethylacetat, als Elutionsmittel ein Gemisch aus Dichlormethan und Methanol. Isoliert werden 37 mg (117 µmol, 43%) der Titelverbindung als farbloses Öl.

### BEISPIEL 75

### [1R-[1α,2β(2R*,3S*),4α,4aβ,9β,10aβ]]-3-(Methoxycarbonyl)amino-2-hydroxy-3-(4-pyridyl)-propansäure-9-methyl-1,2,3,4,4a,9,10,10a-octahydro-1,12,12-trimethyl-4a,10a-dihydroxy-1,4-methanophenanthren-2-ylester

Die Lösung von 7,6 mg (15 µmol) der nach Beispiel 60 dargestellten Verbindung in 0,4 ml Tetrahydrofuran versetzt man mit 0,4 ml Wasser, 40 µl einer 4N Salzsäure und rührt unter einer Atmosphäre aus Argon 18 Stunden bei 23°C. Man neutralisiert durch Zugabe einer gesättigten Natriumhydrogencarbonatlösung und extrahiert mehrfach mit Dichlormethan. Die vereinigten organischen Extrakte wäscht man mit gesättigter Natriumchloridlösung und trocknet über Natriumsulfat. Den nach Filtration und Lösungsmittelabzug erhaltenen Rückstand reinigt man durch Chromatographie an einer analytischen Dünnschichtplatte. Als Laufmittel dient Ethylacetat, als Elutionsmittel ein Gemisch aus Dichlormethan und Methanol. Isoliert werden 6,4 mg (12 µmol, 81%) der Titelverbindung als farbloses Öl.
¹H-NMR (CDCl₃): δ = 0,53 (3H), 0,98 (3H), 1,00 (3H), 1,38 (3H), 1,94 (1H), 2,19 (2H), 2,41 (1H), 2.69 (1H), 3,03 (1H), 3,38 (1H), 3.67 (3H), 4,16 (1H), 4,58 (1H), 5,15 (1H), 5,30 (1H), 6,39 (1H), 7,11 (1H), 7,22 (2H), 7,33 (2H), 7,39 (1H), 8,47 (2H) ppm.

### BEISPIEL 76

### [1R-[1α,2β(2R*,3S*),4α,4aβ,9α,10aβ]]-3-(Methoxycarbonyl)amino-2-hydroxy-3-(4-pyridyl)-propansäure-9-yliden-9-oxymethylen-1,2,3,4,4a,9,10,10a-octahydro-1,12,12-trimethyl-4a,10a-epoxy-1,4-methanophenanthren-2-ylester

In Analogie zu Beispiel 1 werden 61 mg (90 µmol) der nach Beispiel 76a dargestellten Verbindung umgesetzt. Nach Aufarbeitung und Reinigung isoliert man 41 mg (79 µmol, 88%) der Titelverbindung als farbloses Öl.
¹H-NMR (CDCl₃): δ = 0,85 (3H), 0,92 (3H), 1,04 (3H), 1,52 (1H), 2,23 (1H), 2,28 (1H), 2,52 (1H), 2,73 (2H), 3,02 (1H), 3,35 (1H), 3,68 (3H), 4,55 (1H), 4,92 (1H), 5,32 (1H), 6,53 (1H), 7,26-7,41 (3H), 7,45 (2H), 7,54 (1H) 8,51 (2H) ppm.

### BEISPIEL 76a

### [1R-[1α,2β(2R*,3S*),4α,4aβ,9α,10aβ]]-3-(Methoxycarbonyl)amino-2-trimethylsilyloxy-3-(4-pyridyl)-propansäure-9-yliden-9-oxymethylen-1,2,3,4,4a,9,10,10a-octahydro-1,12,12-trimethyl-4a,10a-epoxy-1,4-methanophenanthren-2-ylester

In Analogie zu Beispiel 1a werden 55 mg (180 µmol) der nach Beispiel 76b dargestellten Verbindung unter Verwendung von (3R,4S)-1-(Methoxycarbonyl)-3-triisopropylsilyloxy-4-(4-pyridyl)-2-azetidinon, das man in Analogie zu dem in Tetrahedron 1992 auf Seite 6985ff beschriebenen Verfahren hergestellt hat, umgesetzt. Nach Aufarbeitung und Reinigung isoliert man 61 mg (90 µmol, 50%) der Titelverbindung als farbloses Öl.

### BEISPIEL 76b

### [1R-(1α,2β,4α,4aβ,9α,10aβ)]-1,2,3,4,4a,9,10,10a-Octahydro-1,12,12-trimethyl-4a,10a-epoxy-9-yliden-9-oxymethylen-1,4-methanophenanthren-2-ol

Die Lösung von 134 mg (420µmol) des nach Beispiel 74b dargestellten Diols löst man in 4 ml wasserfreiem Toluol, kühlt unter einer Atmosphäre aus trockenem Argon auf 3°C, versetzt mit 190 µl 1,8-Diazabicyclo[5.4.0]undec-7-en, 83 µl Perfluorbutan-1-sulfonsäurefluorid und rührt 1 Stunde. Man gießt m eine gesättigte Natriumhydrogencarbonatlösung, extrahiert mehrfach mit Dichlormethan, wäscht die vereinigten organischen Extrakte mit gesättigter Natriumchloridlösung und trocknet über Natriumsulfat. Den nach Filtration und Lösungsmittelabzug erhaltenen Rückstand reinigt man durch Chromatographie an ca. 10 ml feinem Kieselgel mit einem Laufmittelgemisch aus n-Hexan und Ethylacetat. Isoliert werden 93 mg (311 µmol, 74%) der Titelverbindung als farbloses Öl.

### BEISPIEL 77

### [1R-[1α,2β(2R*,3S*),4α,4aβ,9α,10aβ]]-3-(Ethoxycarbonyl)amino-2-hydroxy-3-(3-thienyl)-propansäure-9-hydroxy-9-acetoxymethyl-1,2,3,4,4a,9,10,10a-octahydro-1,12,12-trimethyl-4a,10a-epoxy-1,4-methanophenanthren-2-ylester

In Analogie zu Beispiel 1 werden 2,8 mg (3,7 µmol) der nach Beispiel 77a dargestellten Verbindung umgesetzt. Nach Aufarbeitung und Reinigung isoliert man 1,2 mg (2,0 µmol, 54%) der Titelverbindung als farbloses Öl.
¹H-NMR (CDCl₃): δ = 0,79 (3H), 0,92 (3H), 1,03 (3H), 1,19 (3H), 1,65 (1H), 2,02 (1H), 2,15 (3H), 2,30 (1H), 2,70 (1H), 2,75 (1H), 3,43 (2H), 4,11 (2H), 4,20 (1H), 4,56 (1H), 4,60 (1H), 5,02 (1H), 5,35 (1H), 6,19 (1H), 7,19 (1H), 7,22-7,38 (3H), 7,42 (1H), 7,52 (1H), 7,69 (1H) ppm.

### BEISPIEL 77a

### [1R-[1α,2β(2R*,3S*),4α,4aβ,9α,10aβ]]-3-(Ethoxycarbonyl)amino-2-triisopropylsilyloxy-3-(3-thienyl)-propansäure-9-hydroxy-9-acetoxymethyl-1,2,3,4,4a,9,10,10a-octahydro-1,12,12-trimethyl-4a,10a-epoxy-1,4-methanophenanthren-2-ylester

In Analogie zu Beispiel 1a werden 10,7 mg (30 µmol) der nach Beispiel 77b dargestellten Verbindung unter Verwendung von (3R,4S)-1-(Ethoxycarbonyl)-3-triisopropylsilyloxy-4-(3-thienyl)-2-azetidinon, das man in Analogie zu dem in Tetrahedron 1992 auf Seite 6985ff beschriebenen Verfahren hergestellt hat, umgesetzt. Nach Aufarbeitung und Reinigung isoliert man 2,8 mg (3,7 µmol, 12%) der Titelverbindung als farbloses Öl.

### BEISPIEL 77b

### [1R-(1α,2β,4α.4aβ,9α,10aβ)]-1,2,3,4,4a,9,10,10a-Octahydro-1,12,12-trimethyl-4a,10a-epoxy-9-hydroxy-9-acetoxymethyl-1,4-methanophenanthren-2-ol

Die Lösung von 51 mg (161 µmol) des nach Beispiel 74b dargestellten Diols in 1,6 ml wasserfreiem Pyridin versetzt man bei 3°C unter einer Atmosphäre aus trockenem Argon mit 18 µl Acetanhydrid, einer Mikrospatelspitze 4-Dimethylaminopyridin und rührt 1 Stunde. Man gießt in Wasser, extrahiert mehrfach mit Dichlormethan, wäscht die vereinigten organischen Extrakte mit gesättigter Natriumchloridlösung und trocknet über Natriumsulfat. Den nach Filtration und Lösungsmittelabzug erhaltenen Rückstand reinigt man durch Chromatographie an 3 analytischen Dünnschichtplatten. Als Laufmittel dient ein Gemisch aus n-Hexan und Ethylacetat, als Elutionsmittel ein Gemisch aus Dichlormethan und Methanol. Isoliert werden 36 mg (100 µmol, 62%) der Titelverbindung als färbloses Öl.

### BEISPIEL 78

### [1R-[1α,2β(2R*,3S*),4α,4aβ,9β,10aβ]]-3-(Methoxycarbonyl)amino-2-hydroxy-3-(4-pyridyl)-propansäure-9-methyl-1,2,3,4,4a,9,10,10a-octahydro-1,12,12-trimethyl-4a-methoxy-10a-hydroxy-1,4-methanophenanthren-2-ylester

In Analogie zu Beispiel 1 werden 24 mg (35 µmol) der nach Beispiel 78a dargestellten Verbindung umgesetzt. Nach Aufarbeitung und Reinigung isoliert man 16,7 mg (31 µmol, 89%) der Titelverbindung als farbloses Öl.
¹H-NMR (CDCl₃): δ = 0,44 (3H), 0,94 (3H), 0,99 (3H), 1,40 (3H), 1,66 (1H), 1,96 (1H), 2,06 (1H), 2,37 (1H), 2,79 (1H), 3,00 (3H), 3,02 (1H), 3,63 (1H), 3,70 (3H), 4,09 (1H), 4,61 (1H), 5,07 (1H), 5,30 (1H), 6,92 (1H), 7,14-7,33 (4H), 7,49 (2H), 8,56 (2H) ppm.

### BEISPIEL 78a

### [1R-[1α,2β(2R*,3S*),4α,4aβ,9β,10aβ]]-3-(Methoxycarbonyl)amino-2-triisopropylsilyloxy-3-(4-pyridyl)-propansäure-9-methyl-1,2,3,4,4a,9,10,10a-oxtahydro-1,12,12-trimethyl-4a-methoxy-10a-hydroxy-1,4-methanophenanthren-2-ylester

In Analogie zu Beispiel 1a werden 22 mg (70 µmol) der nach Beispiel 78b dargestellten Verbindung unter Verwendung von (3R,4S)-1-(Methoxycarbonyl)-3-triisopropylsilyloxy-4-(4-pyridyl)-2-azetidinon, das man in Analogie zu dem in Tetrahedron 1992 auf Seite 6985ff beschriebenen Verfahren hergestellt hat, umgesetzt. Nach Aufarbeitung und Reinigung isoliert man 24 mg (35 µmol, 50%) der Titelverbindung als farbloses Öl.

### BEISPIEL 78b

### [1R-(1α,2β,4α,4aβ,9β,10aβ)]-1,2,3,4,4a,9,10,10a-Octahydro-1,12,12-trimethyl-4a-methoxy-10a-hydroxy-9-methyl-1,4-methanophenanthren-2-ol

Die Lösung von 20 mg (70 µmol) der nach Beispiel 33b dargestellten Verbindung A in 1 ml wasserfreiem Methanol versetzt man bei 23°C unter einer Atmosphäre aus trockenem Argon mit 200 mg Dovex® und rührt 1 Stunde. Man filtriert, engt ein und reinigt den Rückstand durch Chromatographie an 1 analytischen Dünnschichtplatte. Als Laufmittel dient ein Gemisch aus n-Hexan und Ethylacetat, als Elutionsmittel ein Gemisch aus Dichlormethan und Methanol. Isoliert werden 22 mg (70 µmol, 100%) der Titelverbindung als farbloses Öl.

### BEISPIEL 79

### [1R-[1α,2β(2R*,3S*)-4α,4aβ,9α,10aβ]]-3-(Methoxycarbonyl)amino-2-hydroxy-3-(4-pyridyl)-propansäure-9-hydroxy-9-acetoxymethyl-1,2,3,4,4a,9,10,10a-octahydro-1,12,12-trimethyl-4a,10a-epoxy-1,4-methanophenanthren-2-ylester

In Analogie zu Beispiel 1 werden 9,4 mg (13 µmol) der nach Beispiel 79a dargestellten Verbindung umgesetzt. Nach Aufarbeirung und Reinigung isoliert man 4,8 mg (8,3 µmol, 64%) der Titelverbindung als farbloses Öl.
¹H-NMR (CDCl₃): δ = 0,79 (3H), 0,96 (3H), 1,09 (3H), 1,72 (1H), 2,06 (1H), 2,13 (3H), 2,39 (1H), 2,68 (1H), 2,98 (1H), 3,56 (3H), 4,10 (1H), 4,30 (1H), 4,39 (1H), 4,69 (1H), 4,92 (1H), 5,52 (1H), 5,75 (1H), 7,23-7,58 (5H), 7,78 (1H), 8,65 (2H) ppm.

### BEISPIEL 79a

### [1R-[1α,2β(2R*,3S*),4α,4aβ,9α,10aβ]]-3-(Methoxycarbonyl)amino-2-triisopropylsilyloxy-3-(4-pyridyl)-propansäure-9-hydroxy-9-acetoxymethyl-1,2,3,4,4a,9,10,10a-octahydro-1,12,12-trimethyl-4a,10a-epoxy-1,4-methanophenanthren-2-ylester

In Analogie zu Beispiel 77b werden 13 mg (19 µmol) der nach Beispiel 79b dargestellten Verbindung umgesetzt. Nach Aufarbeitung und Reinigung isoliert man 9,4 mg (13 µmol, 67%) der Titelverbindung als farbloses Öl.

### BEISPIEL 79b

In Analogie zu Beispiel 1a werden 19 mg (60 µmol) der nach Beispiel 74b dargestellten Verbindung unter Verwendung von (3R,4S)-1-(Methoxycarbonyl)-3-triisopropylsilyloxy-4-(4-pyridyl)-2-azetidinon, das man in Analogie zu dem in Tetrahedron 1992 auf Seite 6985ff beschriebenen Verfahren hergestellt hat, umgesetzt. Nach Aufarbeitung und Reinigung isoliert man 13 mg (19 µmol, 31%) der Titelverbindung als farbloses Öl.

### BEISPIEL 80

### [1R-[1α,2β(2R*,3S*),4α,4aβ,9β,10aβ]]-3-[[[(1-Methyl)ethylamino]carbonyl] amino]-2-hydroxy-3-(3-thienyl)-propansäure-9-methyl-1,2,3,4,4a,9,10,10a-octahydro-1,12,12-trimethyl-4a,10a-epoxy-1,4-methanophenanthren-2-ylester

In Analogie zu Beispiel 1 werden 3 mg (4,3 µmol) der nach Beispiel 80a dargestellten Verbindung umgesetzt. Nach Aufarbeitung und Reinigung isoliert man 1,6 mg (3,0 µmol, 70%) der Titelverbindung als farbloses Öl.
¹H-NMR (CDCl₃): δ = 0,72 (3H), 0,79 (3H), 0,82 (3H) 0,92 (3H), 1,04 (3H), 1,39 (3H), 1,69 (1H), 2,17 (2H), 2,39 (1H), 2,72 (1H), 3,13 (1H), 3,21 (1H), 3,73 (1H), 4,56 (1H), 4,72 (1H), 5,08 (1H), 5,25 (1H), 6,15 (1H), 7,08 (1H), 7,14-7,35 (5H), 7,49 (1H) ppm.

### BEISPIEL 80a

### [1R-[1α,2β(2R*,3S*),4α,4aβ,9β,10aβ]]-3-[[[(1-Methyl)ethylamino]carbonyl] amino]-2-triisopropylsilyloxy-3-(3-thienyl)-propansäure-9-methyl-1,2,3,4,4a,9,10,10a-octahydro-1,12,12-trimethyl-4a,10a-epoxy-1,4-methanophenanthren-2-ylester

In Analogie zu Beispiel 9a werden 18 mg (26 µmol) der nach Beispiel 80b dargestellten Verbindung hydriert. Nach Aufarbeitung und Reinigung isoliert man 3 mg (4,3 µmol, 17%) der Titelverbindung als farbloses Öl.

### BEISPIEL 80b

### [1R-[1α,2β(2R*,3S*),4α,4aβ,10aβ]]-3-Amino-2-triisopropylsilyloxy-3-(3-thienyl)-propansäure-9-methylen-1,2,3,4,4a,9,10,10a-octahydro-1,12,12-trimethyl-4a,10a-epoxy-1,4-methanophenanthren-2-ylester

Die Lösung von 20 mg (33 µmol) des nach Beispiel 40b dargestellten Amins in 0,3 ml wasserfreiem Dichlormethan versetzt man unter einer Atmosphäre aus trockenem Argon bei 0°C mit 14 µl Triethylamin, 12 mg 4-Dimethylaminopyridin, 9,7 µl Isopropylisocyanat und rührt 1 Stunde. Man gießt in Wasser, extrahiert mehrfach mit Dichlormethan, wäscht die vereinigten organischen Extrakte mit gesättigter Natriumchloridlösung und trocknet über Natriumsulfat. Den nach Filtration und Lösungsmittelabzug erhaltenen Rückstand reinigt man durch Chromatographie an 2 analytischen Dünnschichtplatten. Als Laufmittel dient ein Gemisch aus n-Hexan und Ethylacetat, als Elutionsmittel ein Gemisch aus Dichlormethan und Methanol. Isoliert werden 18 mg (26 µmol, 79%) der Titelverbindung als farbloses Öl.

### BEISPIEL 81

### [1R-[1α,2β(2R*,3S*),4α,4aβ,9β,10aβ]]-3-(Butoxycarbonyl)amino-2-hydroxy-3-(4-hydroxyphenyl)-propansäure-9-methyl-1,2,3,4,4a,9,10,10a-octahydro-1,12,12-trimethyl-4a,10a-epoxy-1,4-methanophenanthren-2-ylester

In Analogie zu Beispiel 1 werden 7 mg (9,7 µmol) der nach Beispiel 81a dargestellten Verbindung umgesetzt. Nach Aufarbeitung und Reinigung isoliert man 3,2 mg (5,7 µmol, 59%) der Titelverbindung als farbloses Öl.
¹H-NMR (CDCl₃): δ = 0,66-1,02 (12H), 1,21-1,47 (5H), 1,48-1,88 (3H), 2,09 (1H), 2,20 (1H), 2,32 (1H), 2,67 (1H), 3,07 (1H), 3,20 (1H), 3,81 (1H), 4,03 (1H), 4,50 (1H), 4,93 (1H), 5,12 (1H), 5,18 (1H), 6,26 (1H), 6,71 (2H), 7,10-7,40 (5H), 7,48 (1H) ppm.

### BEISPIEL 81a

### [1R-[1α,2β(2R*,3S*),4α,4aβ,9β,10aβ]]-3-(Butoxycarbonyl)amino-2-triisopropylsilyloxy-3-(4-hydroxyphenyl)-propansäure-9-methyl-1,2,3,4,4a,9,10,10a-octahydro-1,12,12-trimethyl-4a,10a-epoxy-1,4-methanophenanthren-2-ylester

In Analogie zu Beispiel 9a werden 13 mg (16 µmol) der nach Beispiel 81b dargestellten Verbindung hydriert. Nach Aufarbeitung und Reinigung isoliert man 7 mg (9,7 µmol, 61%) der Titelverbindung als farbloses Öl.

### BEISPIEL 81b

### [1R-[1α,2β(2R*,3S*),4α,4aβ,9β,10aβ]]-3-(Butoxycarbonyl)amino-2-triisopropylsilyloxy-3-(4-benzyloxyphenyl)-propansäure-9-methyl-1,2,3,4,4a,9,10,10-octahydro-1,12,12-trimethyl-4a,10a-epoxy-1,4-methanophenanthren-2-ylester

In Analogie zu Beispiel 1a werden 10 mg (35 µmol) der nach Beispiel 33b dargestellten Verbindung A unter Verwendung von (3R,4S)-1-Butoxycarbonyl-3-triisopropylsilyloxy-4-(4-pyridyl)-2-azetidinon, das man in Analogie zu dem in Tetrahedron 1992 auf Seite 6985ff beschriebenen Verfahren hergestellt hat, umgesetzt. Nach Aufarbeitung und Reinigung isoliert man 13 mg (16 µmol, 46%) derTitelverbindung als farbloses Öl.

### BEISPIEL 82

### [1R-[1α,2β(2R*,3S*),4α,4aβ,9β,10aβ]]-3-(Methoxycarbonyl)amino-2-hydroxy-3-cyclohexyl-propansäure-9-methyl-1,2,3,4,4a,9,10,10a-octahydro-1,12,12-trimethyl-4a,10a-epoxy-1,4-methanophenanthren-2-ylester

In Analogie zu Beispiel 1 werden 10 mg (15 µmol) der nach Beispiel 82a dargestellten Verbindung umgesetzt. Nach Aufarbeitung und Reinigung isoliert man 4,9 mg (9,6 µmol, 64%) der Titelverbindung als farbloses Öl.
¹H-NMR (CDCl₃): δ = 0,78 (3H), 0,94 (3H), 1,02 (3H), 1,32 (3H), 0,85-1,38 (6H), 1,52-1,96 (6H), 2,04 (1H), 2,12 (1H), 2,37 (1H), 2,66 (1H), 3,05 (1H), 3,63 (4H), 3,90 (1H), 4,43 (1H), 4,93 (1H), 5,61 (1H), 7,12-7,33 (3H), 7,48 (1H) ppm.

### BEISPIEL 82a

### [1R-[1α,2β(2R*,3S*),4α,4aβ,9β,10aβ]]-3-(Methoxycarbonyl)amino-2-triisopropylsilyloxy-3-cyclohexyl-propansäure-9-methyl-1,2,3,4,4a,9,10,10a-octahydro-1,12,12-trimethyl-4a,10a-epoxy-1,4-methanophenanthren-2-ylester

In Analogie zu Beispiel 1a werden 8,5 mg (30 µmol) der nach Beispiel 33b dargestellten Verbindung A unter Verwendung des nach Beispiel 82b dargestellten β-Lactams umgesetzt. Nach Aufarbeitung und Reinigung isoliert man 10 mg (15 µmol, 50%) der Titelverbindung als farbloses Öl.

### BEISPIEL 82b

### (3R,4S)-1-Methoxycarbonyl-3-triisopropylsilyloxy-4-cyclohexyl-2-azetidinon

In Analogie zu Beispiel 61e werden 326 mg (1,0 mmol) der nach Beispiel 82c dargestellten Verbindung unter Verwendung von Chlorameisensäuremethylester umgesetzt. Nach Aufarbeitung und Reinigung isoliert man 75 mg (204 µmol, 20%) der Titelverbindung als farbloses Öl.

### BEISPIEL 82c

### (3R,4S)-3-Triisopropylsilyloxy-4-cyclohexyl-2-azetidinon

Die Lösung von 1,0g (3,13 mmol) (3R,4S)-3-Triisopropylsilyloxy-4-phenyl-2-azetidinon, das man in Analogie zu dem in Tetrahedron 1992 auf Seite 6985ff beschriebenen Verfahren hergestellt hat, in 114 ml Ethylacetat versetzt man mit 0,57g Platin auf Kohle (3%ig) und hydriert bei ca. 4 bar. Nach Filtration wird der erhaltene Rückstand an ca. 70 ml feinem Kieselgel mit einem Laufmittelgemisch aus n-Hexan und Ethylacetat chromatographisch gereinigt. Isoliert werden 735 mg (2,26 mmol, 72%) der Titelverbindung als farblosen Feststoff.

### BEISPIEL 83

### [1R-[1α,2β(2R*,3S*),4α,4aβ,9β,10aβ]]-3-(Methoxycarbonyl)amino-2-hydroxy-3-(4-hydroxyphenyl)-propansäure-9-methyl-1,2,3,4,4a,9,10,10a-octahydro-1,12,12-trimethyl-4a,10a-epoxy-1,4-methanophenanthren-2-ylester

In Analogie zu Beispiel 1 werden 11 mg (16 µmol) der nach Beispiel 83a dargestellten Verbindung umgesetzt. Nach Aufarbeitung und Reinigung isoliert man 6,2 mg (12 µmol, 75%) der Titelverbindung als farbloses Öl.
¹H-NMR (CDCl₃): δ = 0,79 (3H), 0,89 (3H), 1,02 (3H), 1,40 (3H), 1,72 (1H), 2,09 (1H), 2,20 (1H), 2,29 (1H), 2,68 (1H), 3,07 (1H), 3,14 (1H), 3,60 (3H), 4,50 (1H), 4,91 (1H), 5,18 (1H), 5,21 (1H), 6,43 (1H), 6,69 (2H), 7,10-7,40 (5H), 7,49 (1H) ppm.

### BEISPIEL 83a

### [1R-[1α,2β(2R*,3S*),4α,4aβ,9β,10aβ]]-3-(Methoxycarbonyl)amino-2-triisopropylsilyloxy-3-(4-hydroxyphenyl)-propansäure-9-methyl-1,2,3,4,4a,9,10,10a-octahydro-1,12,12-trimethyl-4a,10a-epoxy-1,4-methanophenanthren-2-ylester

In Analogie zu Beispiel 1a werden 15 mg (53 µmol) der nach Beispiel 33b dargestellten Verbindung A unter Verwendung von (3R,4S)-1-Methoxycarbonyl-3-triisopropylsilyloxy-4-(4-hydroxyphenyl)-2-azetidinon, das man in Analogie zu dem in Tetrahedron 1992 auf Seite 6985ff beschriebenen Verfahren hergestellt hat, umgesetzt. Nach Aufarbeitung und Reinigung isoliert man 20 mg (30 µmol, 56%) der Titelverbindung als farbloses Öl.

### BEISPIEL 84

### [1R-[1α,2β(2R*,3S*),4a,4aβ,9β,10aβ]]-3-(Methoxycarbonyl)amino-2-hydroxy-3-(4-hydroxyphenyl)-propansäure-9-methyl-1,2,3,4,4a,9,10,10a-octahydro-1,12,12-trimethyl-10a-hydroxy-1,4-methanophenanthren-2-ylester

In Analogie zu Beispiel 1 werden 25 mg (37 µmol) der nach Beispiel 84a dargestellten Verbindung umgesetzt. Nach Aufarbeitung und Reinigung isoliert man 8,5 mg (16,2 µmol, 44%) der Titelverbindung als farbloses Öl.
¹H-NMR (CDCl₃): δ = 0,55 (3H), 0,94 (3H), 1,08 (3H), 1,42 (3H), 1,52 (1H), 1,90 (1H), 2,11 (1H), 2,51 (1H), 2,83 (1H), 3,10 (2H), 3,20 (1H), 3,67 (3H), 4,56 (1H), 4,81 (1H), 5,13 (1H), 5,17 (1H), 5,56 (1H), 6,56 (2H), 6,87 (2H), 7,08-7,32 (4H), 8,39 (1H) ppm.

### BEISPIEL 84a

### [1R-[1α,2β(2R*,3S*),4α,4aβ,9β,10aβ]]-3-(Methoxycarbonyl)amino-2-triisopropyloxy-3-(4-hydroxyphenyl)-propansäure-9-methyl-1,2,3,4,4a,9,10,10a-octahydro-1,12,12-trimethyl-10a-hydroxy-1,4-methanophenanthren-2-ylester

In Analogie zu Beispiel 83a werden 15 mg (53 µmol) der nach Beispiel 84b dargestellten Verbindung umgesetzt. Nach Aufarbeitung und Reinigung isoliert man 25 mg (37 µmol, 69%) der Titelverbindung als farbloses Öl.

### BEISPIEL 84b

### [1R-(1α2β,4α4aβ,9β,10aβ)]-9-methyl-1,2,3,4,4a,9,10,10a-octahydro-1,12,12-trimethyl-10a-hydroxy-1,4-methanophenanthren-2-ol (A) und [1R-(1α,2β,4α,9β)]-9-methyl-1,2,3,4,9,10-hexahydro-1,12,12-trimethyl-1,4-methanophenanthren-2-ol (B)

In Analogie zu Beispiel 9a werden 20 mg (70 µmol) der nach Beispiel 33b/A dargestellten Verbindung umgesetzt. Nach Aufarbeitung und Reinigung isoliert man 5 mg (17 µmol, 25%) der Titelverbindung A sowie 7 mg (26µmol, 37%) der Titelverbinding B jeweils als farbloses Öl.

### BEISPIEL 85

### [1R-[1α,2β[2R*,3S*(2R*,3S*)],4α,4aβ,9β,10aβ]]-2-Hydroxy-3-amino-3-(4-pyridyl)-propansäure-9-methyl-1,2,3,4,4a,9,10,10a-octahydro-1,12,12-trimethyl-4a,10a-epoxy-1,4-methanophenanthren-2-ylester

In Analogie zu Beispiel 1 werden 11 mg (18 µmol) der nach Beispiel 42b dargestellten Verbindung umgesetzt. Nach Aufarbeitung und Reinigung isoliert man 6,3 mg (14 µmol, 78%) der Titelverbindung als farbloses Öl.
1H-NMR (CDCl₃): δ = 0,80 (3H), 0,92 (3H), 1,07 (3H), 1,33 (3H), 1,77 (1H), 1,89 (breit, 3H), 2,07 (1H), 2,16 (1H), 2,40 (1H), 2,69 (1H), 3,06 (1H), 4,45 (1H), 4,50 (1H), 4,85 (1H), 7,10-7,32 (3H), 7,41 (2H), 7,48 (1H), 8,54 (2H) ppm.

## Patentansprüche

1. Borneolester da allgemeinen Formel I 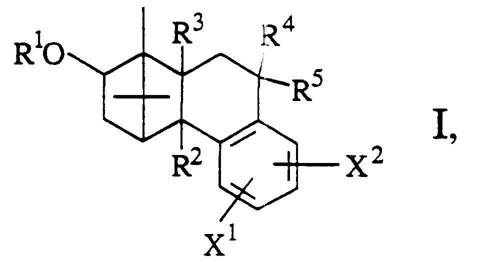 worin
R¹ T-C(O)-CH(OR⁶)-CH(NR^{7a}R^{7b})-R⁸, C(O)-CH(OR^{6a})-CH[NR⁷(C(O)-CH(OR^{6b})-CH(NR^{7a}R^{7b})-R^{8a})]-R^{8b},
R² Wasserstoff, -OH, C₁-C₁₀-Alkyl, C₁-C₁₀-Alkoxy, -OC(O)R^{9a}, OSO₂R^{9a}, NHR^{9a}, NR^{9a}R^{9b},
R³ Wasserstoff, -OH, C₁-C₁₀-Alkoxy, -OC(O)R^{9b}, -OSO₂R^{9b}, oder
R², R³ gemeinsam ein Sauerstoffatom,
R⁴ Wasserstoff, C₁-C₁₀-Alkyl, -(CH₂)ₙ-OR^{11a},
R⁵ Wasserstoff, C₁-C₁₀-Alkyl, -(CH₂)ₚ-OR^{11b}, oder
R⁴, R⁵ gemeinsam ein Sauerstoffatom, eine =CHR¹⁰-Gruppe, eine -CH₂-CH₂-Gruppe, eine CH₂-O-Gruppe,
R^{6a}, R^{6b} gleich oder verschieden sind und R⁶ bedeuten,
R⁶ Wasserstoff, C₁-C₁₀-Alkyl, Aryl, C₁-C₁₀-Acyl, C₇-C₂₀-Aralkyl, -SO₂R^{9c}, R¹⁵ bedeuten,
R^{7a}, R^{7b} gleich oder verschieden sind und R⁷ bedeuten,
R⁷ Wasserstoff, A, -C(O)R¹², -C(O)OR¹², -C(O)SR¹², C(O)NHR^{9d}, -C(O)NR^{9d}R^{9e}, -SO₂R¹², C₁-C₁₀-Alkyl,
R^{8a}, R^{8b} gleich oder verschieden sind und R⁸ bedeuten,
R⁸ 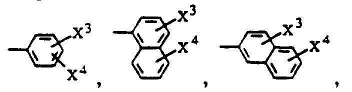 durch X³ substituiertes Heteroaryl, C₇-C₁₆-Aralkyl, C₁-C₁₀-Alkyl,
R^{9a-g}, R¹² gleich oder verschieden sind und C₁-C₂₀-Alkyl, C₄-C₈-Cycloalkyl, Aryl, C₇-C₂₀-Aralkyl bedeuten,
R¹⁰ Wasserstoff, C₁-C₁₀-Alkyl, -(CH₂)ₛ-OR¹³,
R^{11a,b}, R¹³ gleich oder verschieden sind und Wasserstoff, C₁-C₁₀-Alkyl, Aryl, C₁-C₂₀-Acyl, C₇-C₂₀-Aralkyl, -SO₂R^{9c} bedeuten,
R^{14a,b} gleich oder verschieden sind und Wasserstoff, A, C₁-C₁₀-Alkyl, Aryl, C₇-C₁₆-Aralkyl bedeuten,
R¹⁵ (Yⁱ₁-Yⁱ₂,Yⁱ₃-...-Yⁱᵣ)-H,
A B-[O-(CH₂)ₜ-C(O)]_{0 oder 1}-, Farnesyl-P(O)(OR^{9d})-O-(CH₂)ₜ-C(O)-,
B Hemmstoffe der Proteinkinasen oder Hemmstoffe der Farnesylproteintransferase wie z.B. Farnesyl, 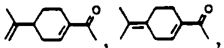
T eine Bindung, Zⁱ oder eine Gruppe 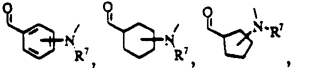
X¹...X⁴ gleich oder verschieden sind und X bedeuten, wobei X³ und X⁴ im Falle von R⁸ und R^{8b} aber nicht gleichzeitig in der Bedeutung von Wasserstoff verwendet werden können,
X Wasserstoff, Halogen, -NO₂, -N₃, -CN, NR^{14a}R^{14b}, -NHSO₂R^{9g}, C₁-C₁₀-Alkyl, C₁-C₂₀-Acyl, C₁-C₂₀-Acyloxy, OR⁶, -OP(O)R^{9g}(OR¹²) -CO₂R¹⁴, -O-A sein kann
Yⁱ_{1...r} gleich oder verschieden sind und -[C(O)-Wⁱ-NH]- = Zⁱ bedeuten, wobei Zⁱ als HO-Zⁱ-H eine, an ihrem terminalen Ende angeknüpfte α-, β- oder γ-Aminosäure "i" darstellt,
n 0 bis 8
p 1 bis 8
r 1 bis 5
s 1 bis 8
t 1 bis 6,
und freie Hydroxylgruppen in R¹, R², R³, R⁴, R⁵, R¹⁰, Z und X durch Veretherung oder Veresterung weiter funktionell abgewandelt sein können und freie Aminogruppen in R¹, R¹⁵ oder X bzw. freie Säuregruppen in X in deren Salze mit physiologisch verträglichen Säuren bzw. Basen überführt sein können, sowie deren α-, β- oder γ-Cyclodextrinclathrate, sowie die mit Liposomen verkapselten Verbindungen der allgemeinen Formel I bedeuten.

2. Arzneimittel, bestehend aus einer oder mehrerer Verbindungen des Anspruches 1 und üblicher Hilfs-, Träger- und Zusatzstoffe.

3. Verfahren zur Herstellung von Borneolderivaten der allgemeinen Formel I gemäß Patentanspruch 1, das dadurch gekennzeichnet, daß man einen Alkohol der allgemeinen Formel II 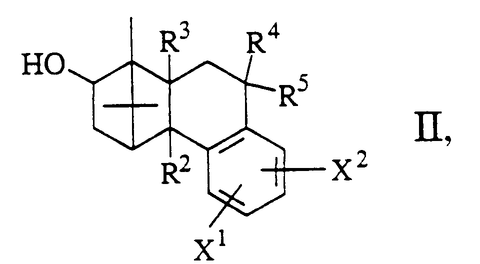 in dem R², R³, R⁴, R⁵, X¹ und X² die oben genannten Bedeutungen haben und in R², R³, R⁴, R⁵, X¹ oder X² enthaltene Hydroxylgruppen gegebenfalls geschützt sind, mit einer Verbindung der allgemeinen Formel IIIa, IIIb oder IIIc, 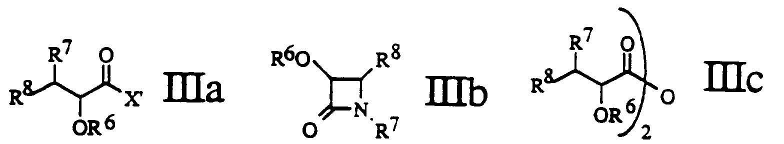 in denen R⁶, R⁷ und R⁸ jeweils die oben genannten Bedeutungen haben und X' Hydroxyl, OR⁹, Halogen und NR^{14a}R^{14b} sein kann, zu Verbindungen der allgemeinen Formel I, in denen freie Hydroxylgruppen durch Veretherung oder Veresterung weiter funktionell abgewandelt sein können, umsetzt.

## Claims

1. Borneol esters of the general formula I 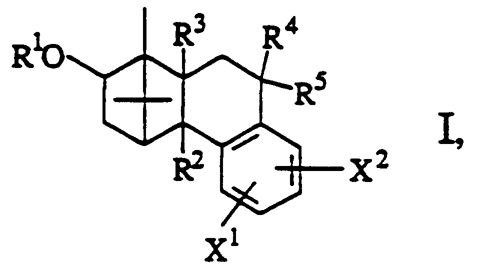 in which
R¹ denotes T-C(O)-CH(OR⁶)-CH(NR^{7a}R^{7b})-R⁸, C(O)-CH(OR^{6a})-CH[NR⁷(C(O)-CH(OR^{6b})-CH(NR^{7a}R^{7b})-R^{8a})]-R^{8b},
R² denotes hydrogen, -OH, C₁-C₁₀-alkyl, C₁-C₁₀-alkoxy, -OC(O)R^{9a}, -OSO₂R^{9a}, NHR^{9a}, NR^{9a}R^{9b},
R³ denotes hydrogen, -OH, C₁-C₁₀-alkoxy, -OC(OR)R^{9b},
R² and R³ together denote an oxygen atom,
R⁴ denotes hydrogen, C₁-C₄-alkyl, -(CH₂)ₙ-OR^{11a},
R⁵ denotes hydrogen, C₁-C₁₀-alkyl, -(CH₂)ₚ-OR^{11b}, or
R⁴ and R⁵ together denote an oxygen atom, a =CHR¹⁰ group, a -CH-₂CH₂- group, a -CH₂-O- group,
R^{6a} and R^{6b} are identical or different and denote R⁶,
R⁶ denotes hydrogen, C₁-C₁₀-alkyl, aryl, C₁-C₂₀-acyl, C₇-C₂₀-aralkyl, -SO₂R^{9c}, R¹⁵,
R^{7a} and R^{7b} are identical or different and denote R⁷,
R⁷ denotes hydrogen, A, -C(O)R¹², -C(O)OR¹², -C(O)SR¹², -C(O)NHR^{9d}, -C(O)NR^{9d},R^{9e}, -SO₂R¹², C₁-C₁₀-alkyl,
R^{8a} and R^{8b} are identical or different and denote R⁸,
R⁸ denotes 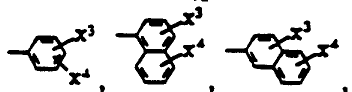 X³-substituted heteroaryl, C₇-C₁₆-aralkyl, C₁-C₁₀-alkyl,
R^{9a-g} and R¹² are identical or different and denote C₁-C₂₀-alkyl, C₄-C₈-cycloalkyl, aryl, C₇-C₂₀-aralkyl,
R¹⁰ denotes hydrogen, C₁-C₁₀-alkyl, -(CH₂)ₛ-OR¹³,
R^{11a,b} and R¹³ are identical or different and denote hydrogen, C₁-C₁₀-alkyl, aryl, C₁-C₂₀-acyl, C₇-C₂₀-aralkyl, -SO₂R^{9c},
R^{14a,b} are identical or different and denote hydrogen, A, C₁-C₁₀-alkyl, aryl, C₇-C₁₆-aralkyl,
R¹⁵ denotes (Yⁱ₁-Yⁱ₂-Yⁱ₃-...Yⁱᵣ)-H,
A denotes B-[O-(CH₂)ₜ-C(O)]_{0 or 1}-, farnesyl-P(O)(OR^{9d})-O-(CH₂)ₜ-C(O)-,
B denotes inhibitors of protein kinases or inhibitors of farnesyl protein transferase, such as, for example, farnesyl, 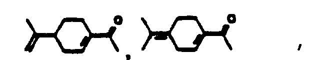
T denotes a bond, Zⁱ or a group 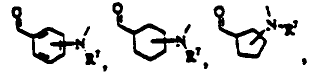
X¹...X⁴ are identical or different and denote X, where, however, X³ and X⁴ in the case of R⁸ and R^{8b} may not simultaneously have the meaning hydrogen,
X may be hydrogen, halogen, -NO₂, -N₃, -CN, -NR^{14a}R^{14b}, -NHSO₂R^{9g}, C₁-C₁₀-alkyl, C₁-C₂₀-acyl, C₁-C₂₀-acyloxy, OR⁶, -OP(O)R^{9g}(OR¹²)-CO₂R¹⁴, -O-A,
Yⁱ_{1...r} are identical or different and denote -[C(O)-WⁱNH]-= Zⁱ where Zⁱ as HO-Zⁱ-H denotes an α-, β- or γ-amino acid "i" which is attached at its terminal end,
n is from 0 to 8,
p is from 1 to 8,
r is from 1 to 5,
s is from 1 to 8,
t is from 1 to 6,
and free hydroxyl groups in R¹, R², R³, R⁴, R⁵, R¹⁰, Z and X may be functionally modified further by etherification or esterification and free amino acids in R¹, R¹⁵ or X or free acid groups in X may be converted into their salts with physiologically acceptable acids or bases, and their α-, β- or γ-cyclodextrin clathrates, and the liposome-encapsulated compounds of the general formula I.

2. Pharmaceuticals, comprising one or more compounds of Claim 1 and customary auxiliaries, carriers and additives.

3. Process for preparing borneol derivatives of the general formula I according to Patent Claim 1, characterized in that an alcohol of the general formula II 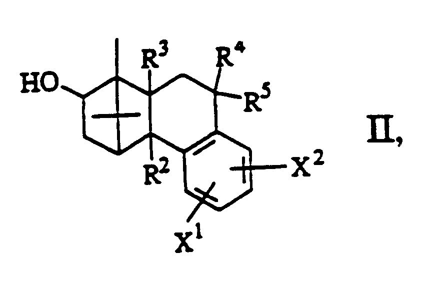 in which R², R³, R⁴, R⁵, X¹ and X² are as defined above and the hydroxyl groups in R², R³, R⁴, R⁵, X¹ or X² are, if appropriate, protected, is reacted with a compound of the general formula IIIa, IIIb or IIIc 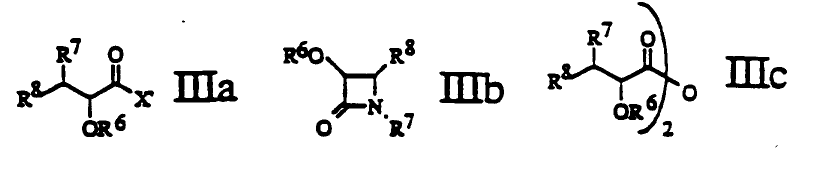 in which R⁶, R⁷ and R⁸ are each as defined above and X' may be hydroxyl, OR⁹, halogen and NR^{14a}R^{14b}, to give compounds of the general formula I in which free hydroxyl groups may be functionally modified further by etherification or esterification.

## Revendications

1. Ester de bornéol de formule générale I dans laquelle 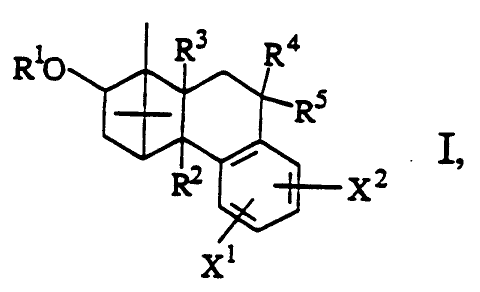
R¹ représente T-C(O)-CH(OR⁶)-CH(NR^{7a}R^{7b})-R⁸, C(O)-CH(OR^{6a})-CH[NR⁷(C(O)-CH(OR^{6b})-CH(NR^{7a}R^{7b})-R^{8a})]-R^{8b},
R² représente l'hydrogène, -OH, un alkyle en C₁-C₁₀, un alkoxy en C₁-C₁₀, -OC(O)R^{9a}, -OSO₂R^{9a}, NHR^{9a}, NR^{9a}R^{9b},
R³ représente l'hydrogène, -OH, un alkoxy en C₁-C₁₀, -OC(O)R^{9b}, -OSO₂R^{9b} ou
R², R³ représentent ensemble un atome d'oxygène,
R⁴ représente l'hydrogène, un alkyle en C₁-C₁₀, -(CH₂)ₙ-OR^{11a},
R⁵ représente l'hydrogène, un alkyle en c₁-c₁₀, -(CH₂)ₚ-OR^{11b}, ou
R⁴, R⁵ représentent ensemble un atome d'oxygène, un groupe =CHR¹⁰, un groupe CH₂-CH₂-, un groupe CH₂-O-
R^{6a}, R^{6b} sont identiques ou différents et représentent R⁶,
R⁶ représente l'hydrogène, un alkyle en C₁-C₁₀, un aryle, un acyle en C₁-C₂₀, un aralkyle en C₇-C₂₀, -SO₂R^{9c}, R¹⁵,
R^{7a}, R^{7b} sont identiques ou différents et représentent R⁷,
R⁷ représente l'hydrogène, A, -C(O)R¹², -C(O)OR¹², -C(O)SR¹², -C(O)NHR^{9d}, -C(O)NR^{9d}R^{9e}, -SO₂R¹², un alkyle en C₁-C₁₀,
R^{8a}, R^{8b} sont identiques ou différents et représentent R⁸,
R⁸ 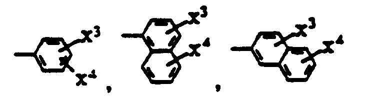 un hétéroaryle substitué par X³, un alkyle en C₇-C₁₆, un alkyle en C₁-C₁₀,
R^{9a-g}, R¹² sont identiques ou différents et représentent un alkyle en C₁-C₂₀, un cycloalkyle en C₄-C₈, un aryle, un aralkyle en C₇-C₂₀,
R¹⁰ représente l'hydrogène, un alkyle en C₁-C₁₀, -(CH₂)ₛ-OR¹³ʼ
R^{11a,b}, R¹³ sont identiques ou différents et représentent l'hydrogène, un alkyle en C₁-C₁₀, un aryle, un acyle en C₁-C₂₀, un aralkyle en C₇-C₂₀, -SO₂R^{9c},
R^{14a,b} sont identiques ou différents et représentent l'hydrogène, A, un alkyle en C₁-C₁₀, un aryle, un aralkyle en C₇-C₁₆,
R¹⁵ représente (Yⁱ₁-Yⁱ₂-Yⁱ₃-...-Yⁱᵣ)-H,
A représente B-(O-(CH₂)ₜ-C(O))₀ₒᵤ₁-, farnesyl-P(O)(OR^{9d})-O-(CH₂)ₜ-C(O)-,
B représente des inhibiteurs des protéine-kinases ou des inhibiteurs de la farnesyl-protéine-transférase, comme par exemple le farnésyle, 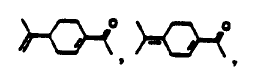
T représente une liaison, Zⁱ ou un groupe 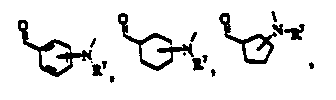
X¹...X⁴ sont identiques ou différents et représentent X, tandis que dans le cas de R⁸ et de R^{8b}, X³ et X⁴ peuvent être utilisés dans le sens de l'hydrogène mais non en même temps,
X peut représenter l'hydrogène, un halogène, -NO₂, N₃, -CN, -NR^{14a}R^{14b}, -NHSO₂R^{9g}, un alkyle en C₁-C₁₀, un acyle en C₁-C₂₀, un acyloxy en C₁-C₂₀, OR⁶, -OP(O)R^{9g}(OR¹²)-CO₂R¹⁴, -O-A,
Yⁱ_{1...r} sont identiques ou différents et représentent -[C(O)-Wⁱ-NH]-=Zⁱ, et Zⁱ représente 〈〈i〉〉 en tant que HO-Zⁱ-H un acide α, β- ou γ-aminé rattaché à son extrémité terminale,
n représente 0 à 8
p représente 1 à 8
r représente 1 à 5
s représente 1 à 8
t représente 1 à 6,
et des groupes hydroxyle libres dans R¹, R², R³, R⁴, R⁵, R¹⁰ Z et X peuvent être encore transformés fonctionnellement par éthérification ou estérification, et des groupes amino libres dans R¹, R¹⁵ ou X ou les groupes acides libres dans X peuvent être transformés en leurs sels avec des acides ou des bases physiologiquement acceptables, ainsi qu'en leurs α, β ou γ-cyclodextrineclathrates, ainsi que les composés de formule générale I encapsulés dans des liposomes.

2. Médicament constitué d'un ou de plusieurs composés selon la revendication 1 et des produits auxiliaires, supports et additifs habituels.

3. Procédé pour la préparation de dérivés de bornéol de formule générale (I) selon la revendication 1, caractérisé en ce que l'on fait réagir un alcool de formule générale (II) 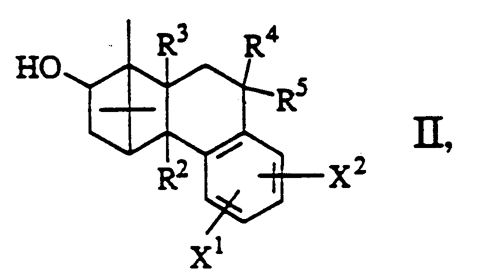 dans laquelle R², R³, R⁴, R⁵, X¹ et X² ont les significations données plus haut, et les groupes hydroxyles contenus dans R², R³, R⁴ R⁵, X¹ ou X² sont éventuellement protégés, avec un composé de formule générale IIIa, IIIb ou IIIc, 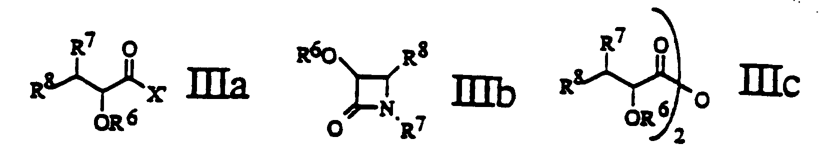 dans lesquelles R⁶, R⁷ et R⁸ ont chacun les significations données plus haut, et X' peut être un hydroxyle, OR⁹,un halogène et NR^{14a}R^{14b}, en composés de formule générale I dans lesquels les groupes hydroxyles libres peuvent être encore convertis fonctionnellement par éthérification ou estérification.
